# EUROPEAN PATENT SPECIFICATION

(11) **EP 0 944 587 B1**
(45) Date of publication and mention of the grant of the patent: **20.03.2002**
(21) Application number: 97947158.8
(22) Date of filing: 02.12.1997
(51) Int. Cl.: C07C 259/06, C07D 295/13, C07D 233/54, C07D 409/12, C07D 213/75, C07C 233/51, A61K 31/16, A61K 33/44, A61K 31/535, A61K 31/415

(54) **METALLOPROTEINASE INHIBITORS**
METALLOPROTEINASE-INHIBITOREN
INHIBITEURS DE METALLOPROTEASES

(30) Priority: 04.12.1996 GB 9625154; 27.06.1997 GB 9713472
(43) Date of publication of application: 29.09.1999
(73) Proprietor: BRITISH BIOTECH PHARMACEUTICALS LIMITED, Cowley Oxford, OX4 5LY (GB)
(72) Inventor: WHITTAKER, Mark, British Biotech Pharm. Ltd, Cowley, Oxford OX4 5LY (GB); BECKETT, Raymond Paul, British Biotech Pharm. Ltd, Cowley, Oxford OX4 5LY (GB); SPAVOLD, Zoe Marie, British Biotech Pharm. Ltd, Cowley, Oxford OX4 5LY (GB); MARTIN, Fionna M., British Biotech Pharm. Ltd, Cowley, Oxford OX4 5LY (GB)
(74) Representative: Walls, Alan James
(86) International application number: PCT/GB97/03316
(87) International publication number: WO 98/24759

(56) References cited:
- WO-A-91/02716
- WO-A-95/04033
- WO-A-95/19956
- WO-A-96/06074
- WO-A-96/16027
- WO-A-96/16931
- WO-A-96/33161
- WO-A-97/02239

## Description

The present invention relates to therapeutically active compounds, to processes for their preparation, to pharmaceutical compositions containing them, and to the use of such compounds in medicine. In particular, the compounds are inhibitors of metalloproteinases involved in tissue degradation.

### Background to the Invention

The matrix metalloproteinases (MMPs) are a family of enzymes including interstitial collagenase, neutrophil collagenase, collagenase-3, 72kDa gelatinase, 92kDa gelatinase, stromelysin-1, stromelysin-2, stromelysin-3, matrilysin, macrophage metalloelastase, membrane-type metalloproteinase-1 and membrane-type metalloproteinase-2. These enzymes share a common zinc-containing catalytic domain and a pro-sequence which maintains latency. A wide range of cells and tissues can express MMPs in response to activation by inflammatory stimuli such as interleukin-1 or tumour necrosis factor-α (TNF-α). Different stimuli can induce overlapping yet distinct repertoires of MMPs and different cell types can respond to the same stimuli by expression of distinct combinations of MMPs. MMPs can attack the protein components of extracellular matrix such as collagens, vitronectin and elastin, and have recently been shown to process membrane proteins such as pro-TNF-α to release soluble TNF-α. MMPs are thought to play a central role in the pathology of inflammatory diseases such as rheumatoid arthritis as well as in the growth and metastasis of tumours.

Compounds which have the property of inhibiting the action of MMPs are thought to be potentially useful for the treatment or prophylaxis of conditions involving such tissue breakdown, for example rheumatoid arthritis, osteoarthritis, osteopenias such as osteoporosis, periodontitis, gingivitis, corneal epidermal or gastric ulceration, and tumour metastasis, invasion and growth. MMP inhibitors are also of potential value in the treatment of neuroinflammatory disorders, including those involving myelin degradation, for example multiple sclerosis, as well as in the management of angiogenesis dependent diseases, which include arthritic conditions and solid tumour growth as well as psoriasis, proliferative retinopathies, neovascular glaucoma, ocular tumours, angiofibromas and hemangiomas.

Many known MMP inhibitors are peptide derivatives, based on naturally occurring amino acids, and are analogues of the cleavage site in the collagen molecule. Chapman et al (J. Med. Chem. 1993, 36, 4293-4301) report some general structure/activity findings in a series of N-carboxyalkyl peptides. Other known MMP inhibitors are less peptidic in structure, and may more properly be viewed as pseudopeptides or peptide mimetics. Such compounds usually have a functional group capable of binding to the active site zinc(II) ion in the MMP, and known classes include those in which the zinc binding group is a hydroxamic acid, carboxylic acid, mercapto, and oxygenated phosphorus (eg phosphinic acid and phosphonic acid) groups.

Two known classes of pseudopeptide or peptide mimetic MMP inhibitors have a hydroxamic acid group or a carboxylic group respectively as their zinc binding groups. Many such known MMPs may be represented by the structural formula (IA) 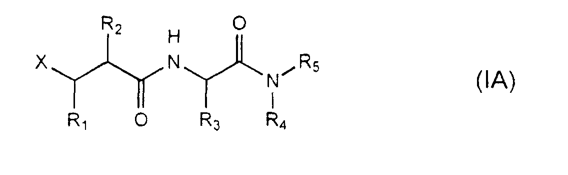 in which X is the zinc binding hydroxamic acid (-CONHOH) or carboxylic acid (-COOH) group and the groups R₁ to R₅ are variable in accordance with the specific prior art disclosures of such compounds.

WO 96/16027 (Syntex/Agouron) discloses a class of MMP inhibitor compounds which can be represented by formula (IA) above. The principal structural characterising feature of the compounds disclosed in WO 96/16027 is the group R₂ which is defined in the publication as being a group R²-X- wherein X is -(CH₂)ₘ-Y-(CH₂)ₙ, Y being O, S or a single bond, m and n being 0, 1, 2, 3 or 4 and m+n being 0, 1, 2, 3, or 4, and R² being (inter alia) aryl or heteroaryl, the latter terms including biaryl such as biphenyl and heteroaryl-aryl such as 4-pyridylphenyl.

Another known class of collagenase inhibitors is represented by those disclosed in EP-A-0574758 (Roche), EP-A-0684240 (Roche), and WO 95/33731 (Roche). In general, the compounds disclosed in those publications may be represented by the structural formula (IB): 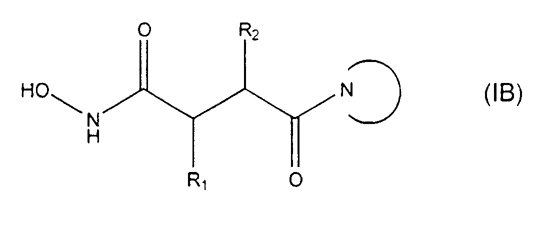 in which R₁, R₂ and the N-containing ring are variable in accordance with the specific disclosures of the publications.

M. A. Abreo *et al*. presented a poster entitled "Truncated Succinamide Hydroxamates With Nanomolar Potency against various MMPs" at the 213th ACS Meeting in San Francisco, 13th-17th April 1997. In that poster compounds of formula (IC) were disclosed: 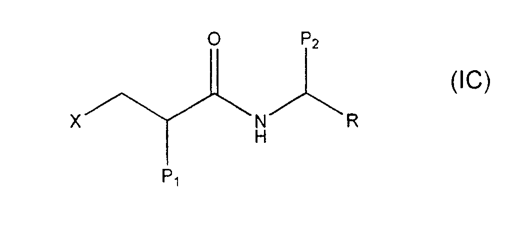 wherein X is -COOH or -CONHOH, P₁ is biphenylpropyl, R is hydroxymethyl and P₂ is the side chain found in one of the following amino acids, namely serine, tert-butylglycine, histidine, O-benzylthreonine, phenylalanine, tyrosine, methionine, threonine, and 3-(3-pyridyl)alanine. Also disclosed were compounds of formula (IC) wherein X and P₁ are as just defined, and P₂ and R together with the carbon atom to which they are attached form a trans-cyclohexan-2-ol or glucosyl ring. The authors stated that the compound (IC), P₂ = the histidine side chain and R = hydroxymethyl, showed good plasma levels after iv and oral dosing to mice. They also stated that the X-ray crystal structure of compound (IC), P₂ = tert-butyl and R = hydroxymethyl, was obtained with stromelysin-1, and that the hydroxyl moiety in R makes an H-bond in the P₃' area of the enzyme, while the tert-butyl group makes good hydrophobic contact in the P₂' area.

International patent application no. WO 97/02239, published 23.1.97, discloses compounds of formula (IA) above wherein R₁ is methoxy and R₂ may be, inter alia, a group which includes one or two triple bonds.

### Brief Description of the Invention

The present invention makes available a new class of MMP inhibitors which conform to the general formula (IA) or (IC) above. The compounds of the invention are principally distinguished from the compounds disclosed in WO 96/16027 and by Abreo et. al. (*loc*. *cit*.) by the identity of the group R₂ (formulae (IA) and (IC)), which in the present compounds contains a conformationally rigid triple bond.

### Detailed Description of the Invention

The present invention provides compounds of formula (I) 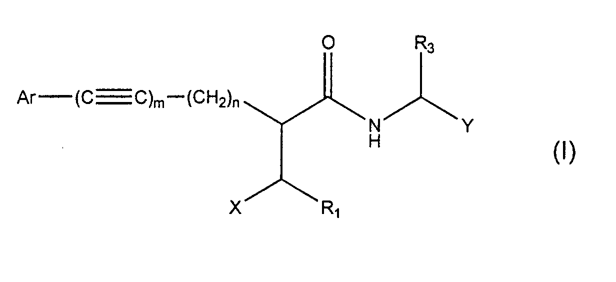 wherein
- Ar: represents an optionally substituted phenyl or heteroaryl group;
- m: is 1;
- n: is 0, 1, 2, 3 or 4;
- X: represents -COOH or -CONHOH;
- R₁: represents hydrogen, (C₁-C₆)alkyl; (C₃-C₈)cycloalkyl; (C₂-C₆)alkenyl; phenyl; substituted phenyl; phenyl (C₁-C₆)alkyl; substituted phenyl(C₁-C₆)alkyl; heterocyclyl; substituted heterocyclyl; heterocyclyl(C₁-C₆)alkyl; substituted heterocyclyl(C₁-C₆)alkyl; amino; protected amino; acylamino; OH; SH; (C₁-C₆)alkoxy; (C₁-C₆)alkylamino; di-(C₁-C₆)alkylamino; (C₁-C₆)alkylthio; amino(C₁-C₆)alkyl; hydroxy(C₁-C₆)alkyl, mercapto(C₁-C₆)alkyl or carboxy(C₁-C₆)alkyl wherein the amino-, hydroxy-, mercapto- or carboxyl-group are optionally protected or the carboxyl- group amidated; or a group B'SOₚA'- wherein p is 0, 1 or 2 and B' is hydrogen or a (C₁-C₆) alkyl, phenyl, substituted phenyl, heterocyclyl, (C₁-C₆)acyl, phenacyl or substituted phenacyl group, and A' represents (C₁-C₆)alkyl;
- R₃: represents the characterising group of a natural or non-natural α amino acid in which any functional groups may be protected;
- Y: is a group of formula (ID) or (IE) 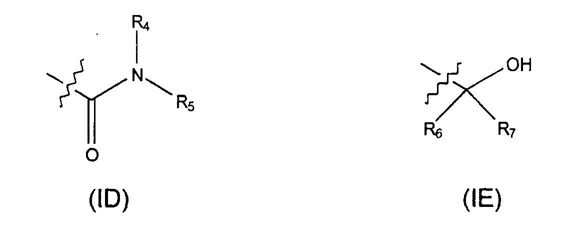
wherein:
- R₄: represents
(a) an optionally substituted cycloalkyl or cycloalkenyl ring; or
(b) a phenyl or heteroaryl ring which may be fused to a benzene or heteroaryl ring, either or both of which rings may be substituted, and in which any ring nitrogen atom may be oxidised as an N-oxide, or
(c) a group -CHR^{x}R^{y} wherein R^{x} and R^{y} each independently represents an optionally substituted phenyl or heteroaryl ring which may be linked covalently to each other by a bond or by a C₁-C₄ alkylene or C₂-C₄ alkenylene bridge;
(d) a group of formula -(Z'-O)_{w}-Z wherein Z' is straight or branched C₁-C₆ alkyl optionally interrupted by one or more non-adjacent S and/or N atoms, w is an integer >1, and no continuous linear sequence of atoms in the group R₄ is >12, or
(e) a straight or branched C₁-C₆ alkyl group, optionally interrupted by one or more non-adjacent S and/or N atoms, which is substituted by at least two substituents of formula -(Z''')ₓ-(OZ)_{q} wherein Z"' is straight or branched C₁-C₆ alkyl optionally interrupted by one or more non-adjacent S and/or N atoms, x is 0 or 1, q is 1 or 2, and no continuous linear sequence of atoms in the group R₄ is >12, or
(f) hydrogen, C₁-C₆ alkyl, C₁-C₄ perfluoroalkyl, or a group D-(C₁-C₆ alkyl)- wherein D is hydroxy, C₁-C₆ alkoxy, C₁-C₆ alkylthio, acylamino. optionally substituted phenyl or heteroaryl, NH₂, or mono- or di-(C₁-C₆ alkyl)amino or N-morpholino;
or R₃ and R₄ taken together represent a divalent chain of formula -C(R^{a})(R^{b})-A"-Alk- wherein R^{a} and R^{b} are independently hydrogen or C₁-C₆ alkyl, A" is a bond, -O-, -S-, -S-S-, -NH- or -NR^{a}- wherein R^{a} is C₁-C₆ alkyl, and Alk is C₁-C₆ alkylene; and
- R₅: is hydrogen or a C₁-C₆ alkyl group;
- R₆: is hydrogen, C₁-C₆ alkyl, phenyl(C₁-C₆ alkyl) or heterocyclyl(C₁-C₆ alkyl);
- R₇: is hydrogen or a C₁-C₆ alkyl group;
or (when R₇ is hydrogen) R₃ and R₇ taken together with the carbon atoms to which they are attached form a 2-hydroxycyclohexyl or C₆ sugar (hexose) ring;
or R₆ and R₇ taken together with the carbon atom to which they are attached form a 5 or 6-membered carbocyclic or heterocyclic ring;
and salts hydrates and solvates thereof, PROVIDED THAT when m and n are both 1, and R₁ is methoxy, and R₃ is tert-butyl, and X is -CONHOH, and Y is a group of formula (ID) wherein R₄ is methyl and R₅ is hydrogen, then Ar is not 4-chlorophenyl or 4-phenylphenyl.

As used herein the term "(C₁-C₆)alkyl" means a straight or branched chain alkyl moiety having from 1 to 6 carbon atoms, including for example, methyl, ethyl, n-propyl, isopropyl, n-butyl, isobutyl, sec-butyl, t-butyl, pentyl and hexyl.

The term "(C₂-C₆)alkenyl" means a straight or branched chain alkenyl moiety having from 2 to 6 carbon atoms having at least one double bond of either E or Z stereochemistry where applicable. This term would include, for example, vinyl, allyl, 1- and 2-butenyl and 2-methyl-2-propenyl.

The term "cycloalkyl" means a saturated alicyclic moiety having from 3-8 carbon atoms and includes, for example, cyclohexyl, cyclooctyl, cycloheptyl, cyclopentyl, cyclobutyl and cyclopropyl.

The term "cycloalkenyl" means an unsaturated alicyclic moiety having from 4-8 carbon atoms and includes, for example, cyclohexenyl, cyclooctenyl, cycloheptenyl, cyclopentenyl, and cyclobutenyl. In the case of cycloalkenyl rings of from 5-8 carbon atoms, the ring may contain more than one double bond.

The unqualified term "heterocyclyl" or "heterocyclic" as used herein means a 5-7 membered aromatic or non-aromatic heterocyclic ring containing one or more heteroatoms selected from S, N and O, and optionally fused to a carbocyclic or second heterocyclic ring. Specific examples of such groups include pyrrolyl, furyl, thienyl, imidazolyl, oxazolyl, thiazolyl, thiadiazolyl, pyrazolyl, pyridinyl, pyrrolidinyl, pyrimidinyl, morpholinyl, piperazinyl, indolyl, and benzimidazolyl. The term encompasses a 5- or 6-membered N-heterocyclic ring which (a) is attached via the N atom, (b) optionally contains N, O and/or S, SO or SO₂ as an additional ring member, (c) is substituted by oxo on one or both C atoms adjacent to the linking N atom and (d) is optionally benz-fused or optionally substituted on one or more other C atoms by C₁-C₆alkyl, or oxo and/or on any additional N atoms by C₁-C₆alkyl, phenyl or heteroaryl. Examples of the latter class of heterocyclic groups include maleimido, succinimido, phthalimido, 1,2-dimethyl-3,5-dioxo-1,2,4-triazolidin-4-yl, 3,4,4-trimethyl-2,5-dioxo-1-imidazolidinyl, 2-methyl-3,5-dioxo-1,2,4-oxadiazol-4-yl, 3-methyl-2,4,5-trioxo-1-imidazolidinyl, 2,5-dioxo-3-phenyl-1-imidazolidinyl-2-oxo-1-pyrrolidinyl, 2,5-dioxo-1-pyrrolidinyl, 2,6-dioxopiperidinylnaphthalimido (i.e. 1,3-dihydro-1,3-dioxo-2H-benz[f]iso-indol-2-yl), 1,3-dihydro-1-oxo-2H-benz[f]isoindol-2-yl, 1,3-dihydro-1,3-dioxo-2H-pyrrolo[3,4-b]quinolin-2-yl, 2,3-dihydro-1,3-dioxo-1H-benz[d,e]isoquinolin-2-yl group or saccharinyl (1,1,3-trioxo-benz[3,4-d]isothiazol-2-yl).

The term "heteroaryl" as used herein means an aromatic 5 or 6 membered monocyclic aromatic heterocyclic group. Specific examples of the latter include thienyl, furyl, pyrrolyl, imidazolyl, thiazolyl, pyrazolyl, isoxazolyl, isothiazolyl, triazolyl, thiadiazolyl, oxadiazolyl, pyridinyl, pyridazinyl, pyrimidinyl, pyrazinyl and triazinyl.

An "optionally substituted phenyl or heteroaryl group" is a phenyl or heteroaryl group which is either unsubstituted or is substituted
(i) with 1, 2, 3 or 4 substituents, each of which independently may be selected from C₁-C₆ alkyl, C₂-C₆ alkenyl, C₁-C₆ alkoxy(C₁-C₆ alkoxy), trifluoromethyl, halo, cyano (-CN), -CH₂CN, -CO₂H, -CO₂R, -CONH₂, -CONHR, -CON(R)₂, -OH, -OR, oxo-, -SH, -SR, -NHCOR, and -NHCO₂R wherein R is C₁-C₆ alkyl or benzyl; or
(ii) with 0, 1, or 2 substituents, each of which independently is selected from those listed under (i) above, and with a phenyl, phenoxy, phenylthio, heteroaryl, heteroaryloxy or heteroarylthio group which may be unsubstituted or substituted with 1, 2, 3 or 4 substituents, each of which independently is selected from those listed under (i) above.

In all contexts except in "optionally substituted phenyl or heteroaryl", the term "substituted" as applied to any moiety herein means substituted with up to four substituents, each of which independently may be (C₁-C₆)alkyl, (C₂-C₆)alkenyl, (C₂-C₆)alkynyl, (C₁-C₆)alkoxy, hydroxy, mercapto, (C₁-C₆)alkylthio, amino, halo (including fluoro, chloro, bromo and iodo), trifluoromethyl, nitro, CN, -COOH, -CONH₂, -COOR^{A},-NHCOR^{A}, -NHCO₂R^{A}, -CONHR^{A}, or -CONR^{A}R^{B} wherein R^{A} and R^{B} are each independently a (C₁-C₆)alkyl, benzyl, or heterocyclyl(C₁-C₆)alkyl- group.

The term "side chain of a natural or non-natural alpha-amino acid" means the group R in a natural or non-natural amino acid of formula NH₂-CH(R)-COOH.

Examples of side chains of natural alpha amino acids include those of alanine, arginine, asparagine, aspartic acid, cysteine, cystine, glutamic acid, histidine, 5-hydroxylysine, 4-hydroxyproline, isoleucine, leucine, lysine, methionine, phenylalanine, proline, serine, threonine, tryptophan, tyrosine, valine, α-aminoadipic acid, α-amino-n-butyric acid, 3,4-dihydroxyphenylalanine, homoserine, α-methylserine, ornithine, pipecolic acid, and thyroxine.

Natural alpha-amino acids which contain functional substituents, for example amino, carboxyl, hydroxy, mercapto, guanidyl, imidazolyl, phenolic or indolyl groups in their characteristic side chains include arginine, lysine, glutamic acid, aspartic acid, tryptophan, histidine, serine, threonine, tyrosine, and cysteine. When R₃ in the compounds of the invention is one of those side chains, the functional substituent may optionally be protected.

Examples of side chains of non-natural alpha amino acids include those referred to below in the discussion of suitable R₃ groups for use in compounds of the present invention.

The term "protected" when used in relation to an amino, hydroxy, mercapto, or carboxy group means a derivative of such a group which is substantially non-functional. Such groups are widely known, for example from the art of peptide synthesis, and are discussed in the widely used handbook by T.W. Greene and P.G.M Wuts, Protective Groups in Organic Synthesis, 2nd Edition, Wiley, New York, 1991. For example, carboxyl groups may be esterified (for example as a C₁-C₆ alkyl ester), amino groups may be converted to amides (for example as a NHCOC₁-C₆ alkyl amide) or carbamates (for example as an NHC(=O)OC₁-C₆ alkyl or NHC(=O)OCH₂Ph carbamate), hydroxyl groups may be converted to ethers (for example an OC₁-C₆ alkyl or a O(C₁-C₆ alkyl)phenyl ether) or esters (for example a OC(=O)C₁-C₆ alkyl ester) and thiol groups may be converted to thioethers (for example a tert-butyl or benzyl thioether) or thioesters (for example a SC(=O)C₁-C₆ alkyl thioester).

Salts of the compounds of the invention include physiologically acceptable acid addition salts for example hydrochlorides, hydrobromides, sulphates, methane sulphonates, p-toluenesulphonates, phosphates, acetates, citrates, succinates, lactates, tartrates, fumarates and maleates. Salts may also be formed with bases, for example sodium, potassium, magnesium, and calcium salts.

There are at least two chiral centres in the compounds according to the invention because of the presence of asymmetric carbon atoms. The presence of these asymmetric carbon atoms gives rise to a number of diastereomers with R or S stereochemistry at each chiral centre. General formula (I), and (unless specified otherwise) all other formulae in this specification are to be understood to include all such stereoisomers and mixtures (for example racemic mixtures) thereof. At present, the preferred stereochemistry is in general as follows:
C atom carrying the groups X and R₁ - S,
C atom carrying the side chain containing the alkyne group - R,
C atom carrying the groups R₃ and Y - S,
but mixtures in which the above configurations predominate are also contemplated.

Ar represents an optionally substituted phenyl or heteroaryl group. Heteroaryl Ar groups may be bonded to the rest of the molecule (I) via a ring carbon atom in Ar or via a ring nitrogen atom in Ar. Suitable heteroaryl groups include thienyl, furyl, pyrrolyl, imidazolyl, thiazolyl, pyrazolyl, isoxazolyl, isothiazolyl, triazolyl, thiadiazolyl, oxadiazolyl, pyridinyl, pyridazinyl, pyrimidinyl, pyrazinyl and triazinyl.

When the group Ar is substituted in accordance with the definition of Ar in formula (I), preferably only one substituent is present. In 6 membered Ar groups, such as phenyl and pyridyl, the substituent is preferably in the 4-position of the ring relative to the bond connecting Ar to the rest of molecule (I). In 5 membered Ar groups, such as thienyl and furanyl, the substituent is preferably in the 3- or 4-position of the ring relative to the bond connecting Ar to the rest of molecule (I).

A sole substituent in Ar may be any of those defined above in part (i) of the definition of "optionally substituted phenyl or heteroaryl". Preferred such substituents include C₁-C₆ alkyl eg methyl, ethyl, n-propyl, n-butyl, n-pentyl and n-hexyl; 2-methoxyethoxy, phenoxy, phenylthio, trifluoromethyl, halo, cyano (-CN), -CH₂CN, -OH, or -OR, wherein R is C₁-C₆ alkyl or benzyl.

Another sole substituent in Ar may be any of those defined above in part (ii) of the definition of "optionally substituted phenyl or heteroaryl". Preferred such substituents include a phenyl, phenoxy, phenylthio, heteroaryl (eg 2-, 3- or 4-pyridyl), heteroaryloxy (eg 2-, 3- or 4-pyridyloxy) or heteroarylthio (eg 2-, 3- or 4-pyridylthio) group which is either unsubstituted or substituted with one substituent selected from C₁-C₆ alkyl eg methyl, ethyl, n-propyl, n-butyl, n-pentyl and n-hexyl; C₁-C₆ alkoxy(C₁-C₆ alkoxy) eg 2-methoxyethoxy, phenoxy, phenylthio, trifluoromethyl, halo, cyano (-CN), -CH₂CN, -OH, and -OR, wherein R is C₁-C₆ alkyl or benzyl.

Again the preferred location of a single substituent in a phenyl or heteroaryl-substituted Ar group is the 4-position of phenyl or 6 membered heteroaryl groups or the 3- or 4-position of 5 membered heteroaryl groups, relative to the bond connecting the phenyl or heteroaryl goup to Ar.

Examples of particular R₁ groups are hydrogen, C₁-C₄ alkyl, cyclopentyl, hydroxy, methoxy, allyl, or a group -(CH₂)ₜ-W wherein t represents 1, 2, 3 or 4 and W represents phthalimido, 1,2-dimethyl-3,5-dioxo-1,2,4-triazolidin-4-yl, 3,4,4-trimethyl-2,5-dioxo-1-imidazolidinyl, 2-methyl-3,5-dioxo-1,2,4-oxadiazol-4-yl, 3-methyl-2,4,5-trioxo-1-imidazolidinyl, 2,5-dioxo-3-phenyl-1-imidazolidinyl-2-oxo-1-pyrrolidinyl, 2,5-dioxo-1-pyrrolidinyl, 2,6-dioxopiperidinylnaphthalimido (ie 1,3-dihydro-1,3-dioxo-2H-benz[f]isoindol-2-yl), 1,3-dihydro-1-oxo-2H-benz[f]isoindol-2-yl, 1,3-dihydro-1,3-dioxo-2H-pyrrolo[3,4-b]quinolin-2-yl, 2,3-dihydro-1,3-dioxo-1H-benz[d,e]isoquinolin-2-yl or saccharinyl.

The following classes of substituent R₃ are suitable for use in compounds of the present invention:
(C₁-C₆)alkyl, benzyl, hydroxybenzyl, benzyloxybenzyl, (C₁-C₆)alkoxybenzyl, or benzyloxy(C₁-C₆)alkyl group; and
the characterising group of a natural α-amino acid, in which any functional group may be protected, any amino group may be acylated and any carboxyl group present may be amidated; and
a group -[Alk]ₙR₂₂ where Alk is a (C₁-C₆)alkyl or (C₂-C₆)alkenyl group optionally interrupted by one or more -O-, or -S- atoms or -N(R₂₃)- groups [where R₂₃ is a hydrogen atom or a (C₁-C₆)alkyl group], n is 0 or 1, and R₂₂ is an optionally substituted cycloalkyl or cycloalkenyl group; and
a benzyl group substituted in the phenyl ring by a group of formula -OCH₂COR₂₄ where R₂₄ is hydroxyl, amino, (C₁-C₆)alkoxy, phenyl(C₁-C₆)alkoxy, (C₁-C₆)alkylamino, di((C₁-C₆)alkyl)amino, phenyl(C₁-C₆)alkylamino, the residue of an amino acid or acid halide, ester or amide derivative thereof, said residue being linked via an amide bond, said amino acid being selected from giycine, α or β alanine, valine, leucine, isoleucine, phenylalanine, tyrosine, tryptophan, serine, threonine, cysteine, methionine, asparagine, glutamine, lysine, histidine, arginine, glutamic acid, and aspartic acid; and
a heterocyclic(C₁-C₆)alkyl group, either being unsubstituted or mono- or disubstituted in the heterocyclic ring with halo, nitro, carboxy, (C₁-C₆)alkoxy, cyano, (C₁-C₆)alkanoyl, trifluoromethyl (C₁-C₆)alkyl, hydroxy, formyl, amino, (C₁-C₆)alkylamino, di-(C₁-C₆)alkylamino, mercapto, (C₁-C₆)alkylthio, hydroxy(C₁-C₆)alkyl, mercapto(C₁-C₆)alkyl or (C₁-C₆)alkylphenylmethyl;
a group -CRₐR_{b}R_{c} in which:
   each of Rₐ, R_{b} and R_{c} is independently hydrogen, (C₁-C₆)alkyl, (C₂-C₆)alkenyl, (C₂-C₆)alkynyl, phenyl(C₁-C₆)alkyl, (C₃-C₈)cycloalkyl, the foregoing being subject to the proviso that Rₐ, R_{b} and R_{c} are not all hydrogen; or
   R_{c} is hydrogen, (C₁-C₆)alkyl, (C₂-C₆)alkenyl, (C₂-C₆)alkynyl, phenyl(C₁-C₆)alkyl, or (C₃-C₈)cycloalkyl, and Rₐ and R_{b} together with the carbon atom to which they are attached form a 3 to 8 membered cycloalkyl or a 5- to 6-membered heterocyclic ring; or
   Rₐ, R_{b} and R_{c} together with the carbon atom to which they are attached form a tricyclic ring (for example adamantyl); or
   Rₐ and R_{b} are each independently (C₁-C₆)alkyl, (C₂-C₆)alkenyl, (C₂-C₆)alkynyl, phenyl(C₁-C₆)alkyl, or a group as defined for R_{c} below other than hydrogen, or Rₐ and R_{b} together with the carbon atom to which they are attached form a 3 to 8 membered cycloalkyl or a 3- to 8-membered heterocyclic ring, and R_{c} is hydrogen, -OH, -SH, halogen, -CN, -CO₂H, (C₁-C₄)perfluoroalkyl, -CH₂OH, -CO₂(C₁-C₆)alkyl, -O(C₁-C₆)alkyl, -O(C₂-C₆)alkenyl, -S(C₁-C₆)alkyl, -SO(C₁-C₆)alkyl, -SO₂(C₁-C₆) alkyl, -S(C₂-C₆)alkenyl, -SO(C₂-C₆)alkenyl, -SO₂(C₂-C₆)alkenyl or a group -Q-W wherein Q represents a bond or -O-, -S-, -SO- or -SO₂- and W represents a phenyl, phenylalkyl, (C₃-C₈)cycloalkyl, (C₃-C₈)cycloalkylalkyl, (C₄-C₈)cycloalkenyl, (C₄-C₈)cycloalkenylalkyl, heteroaryl or heteroarylalkyl group, which group W may optionally be substituted by one or more substituents independently selected from, hydroxyl, halogen, -CN, -CO₂H, -CO₂(C₁-C₆)alkyl, -CONH₂, -CONH(C₁-C₆)alkyl, -CONH(C₁-C₆alkyl)₂, -CHO, -CH₂OH, (C₁-C₄)perfluoroalkyl, -O(C₁-C₆)alkyl, -S(C₁-C₆)alkyl, -SO(C₁-C₆)alkyl, -SO₂(C₁-C₆)alkyl, -NO₂, -NH₂, -NH(C₁-C₆)alkyl, -N((C₁-C₆)alkyl)₂,-NHCO(C₁-C₆)alkyl, (C₁-C₆)alkyl (C₂-C₆)alkenyl, (C₂-C₆)alkynyl, (C₃-C₈)cycloalkyl, (C₄-C₈)cycloalkenyl, phenyl or benzyl.

Examples of particular R₃ groups suitable for inclusion in the compounds of the invention include methyl, benzyl, 4-chlorophenylmethyl, 2-thienylmethyl, iso-butyl or t-butyl, 1-benzylthio-1-methylethyl, and 1-mercapto-1-methylethyl or 3H-imidazol-4-ylmethyl. Presently preferred are compounds in which R₃ is benzyl, t-butyl 1-mercapto-1-methylethyl or 3H-imidazol-4-ylmethyl.

In compounds of the invention wherein Y is a group (ID), R₄ may for example be
cyclopropyl, cyclobutyl, cyclopentyl, cyclohexyl, cycloheptyl or cyclooctyl;
optionally substituted phenyl, naphthyl, furanyl, thienyl, pyrrolinyl, tetrahydrofuranyl, imidazolyl, oxadiazolyl, thiazolyl, thiadiazolyl, pyridinyl, pyridinyl N-oxides, piperazinyl, indolyl, benzimidazolyl, benzotriazolyl, pyrazinyl, pyridazinyl, pyrimidinyl, dithianyl, benzo[b]thienyl, isoxazolyl or quinolinyl. Examples of particular R₄ groups of this type include phenyl, 2-methoxyphenyl, 3-methoxyphenyl, 4-methoxyphenyl, 3,4-dimethoxyphenyl, 2-fluorophenyl, 3-fluorophenyl, 4-fluorophenyl, 2-chlorophenyl, 3-chlorophenyl, 4-chlorophenyl, 3,4-dichlorophenyl, 3,5-dichlorophenyl, 2-bromophenyl, 3-bromophenyl, 4-bromophenyl, 2-iodophenyl, 3-iodophenyl, 4-iodophenyl, 2-methylphenyl, 3-methylphenyl, 4-methylphenyl, 3,4-dimethyl, 2-t-butylphenyl, 3-t-butylphenyl, 4-t-butylphenyl, 4-t-butyl-2,6-dimethylphenyl, 2-nitrophenyl, 3-nitrophenyl, 4-nitrophenyl, 2-cyanophenyl, 3-cyanophenyl, 4-cyanophenyl, 2-acetylphenyl, 3-acetylphenyl, 4-acetylphenyl, 2-methylsulphonylphenyl, 3-methylsulphonylphenyl, 4-methylsulphonylphenyl, 2-trifluoromethylphenyl, 3-trifluoromethylphenyl, 4-trifluoromethylphenyl, 3,5-ditrifluoro-methylphenyl, 2-aminophenyl, 3-aminophenyl, 4-aminophenyl, 2-N,N-dimethyl-aminophenyl, 3-N,N-dimethylaminophenyl, 4-N,N-dimethylaminophenyl, 2-hydroxyphenyl, 3-hydroxyphenyl, 4-hydroxyphenyl, 2-napthyl, furan-2-yl, thien-2-yl, pyrrol-2-yl, tetrahydrofuran-2-yl, imidazol-2-yl, thiazol-2-yl, 4-ethoxycarbonyl-methylthiazol-2-yl, 4-phenylthiazol-2-yl, 4,5-dimethylthiazol-2-yl, 5-bromothiazol-2-yl, 4-*tert*-butylthiazol-2-yl, benzothiazol-2-yl, 1,2,4-oxadiazol-5-yl, 3-methyl-1,2,4-oxadiazol-5-yl , 3-phenyl-1,2,4-oxadiazol-5-yl, 1,2,4-oxadiazol-3-yl, 1,3,4-oxadiazol-2-yl, 1,2,4-thiadiazol-5-yl, 3-phenyl-1,2,4-thiadiazol-5-yl, 1,3,4-thiadiazol-2-yl, 5-methyl-1,3,4-thiadiazol-2-yl, pyridin-2-yl, pyridin-3-yl, pyridin-4-yl, N-oxides of pyridin-2-yl pyridin-3-yl and pyridin-4-yl, piperazin-1-yl, indol-2-yl, benzimidazol-2-yl, benzotriazol-2-yl, pyrazin-2-yl, 1,2-pyridazin-3-yl, 1,3-pyrimidin-5-yl, 1,3-dithian-2-yl, benzo[b]thien-2-yl, isoxazol-5-yl, quinolin-3-yl. Presently preferred are compounds in which R₄ is phenyl, 3-methoxyphenyl, pyridin-2-yl, pyridin-3-yl, and thiazol-2-yl, 4,5-dimethylthiazol-2-yl, 5-bromothiazol-2-yl, 4-ethoxycarbonylmethylthiazol-2-yl, 5-methyl-1,3,4-thiadiazol-2-yl or 4-*tert*butylthiazol-2-yl. Particularly preferred R₄ groups of this type are 3-methoxyphenyl, pyridin-2-yl, pyridin-3-yl, pyridin-4-yl, thiazol-2-yl, 4-ethoxyca rbonylmethylthiazol-2-yl, 5-methyl-1,3,4-thiadiazol-2-yl or 4-*tert*-butylthiazol-2-yl;
a group -CHR^{x}R^{y} wherein R^{x} and R^{y} independently represent optionally substituted phenyl, thienyl, furyl, pyrrolyl, imidazolyl, thiazolyl, pyrazolyl, isoxazolyl, isothiazolyl, triazolyl, thiadiazolyl, oxadiazolyl, pyridinyl, pyridazinolyl, pyrimidinyl, piperazinyl or triazinyl. Examples of particular R^{x} and R^{y} include phenyl, 4-chlorophenyl and pyridinyl. Where R^{x} and R^{y} are linked covalently, an example of a group R₄ is 9-H-fluoren-9-yl;
a polyether chain possessing at least two non-adjacent oxygen atoms, for example 2-(2-methoxyethoxymethoxy)ethyl, 1,1-dimethyl-2-(2-methoxyethoxymethoxy)ethyl, 2-(2-ethoxyethoxymethoxy)ethyl, 2-(2-(2-methoxyethoxy)ethoxy)ethyl, 2-(2-(3-methoxypropoxymethoxy)ethyl, 3-(2-methoxyethoxymethoxy)propyl, 2,2-dimethyl-3-(2-methoxyethoxymethoxy)propyl, 2-(2-methoxyethoxy)ethyl, 3-(2-methoxyethoxy)-propyl, 2-methyl-2,2-di(2-methoxyethyl)propyl, 2-methyl-2,2-di(2-methoxyethyl)butyl, and 2-methyl-2,2-di(2-methoxymethyl)propyl. A presently preferred R₄ group of this type is 2-(2-methoxyethoxy)ethyl;
methyl, ethyl, n- or iso-propyl, n-, sec- or tert-butyl, hydroxyethyl, hydroxypropyl, 2,2-dimethyl-3-hydroxypropyl, hydroxybutyl, methoxyethyl, ethoxyethyl, methoxypropyl, 2,2-dimethyl-3-methoxypropyl, 2,2-dimethyl-3-ethoxypropyl, 2-ethylthioethyl, 2-acetoxyethyl, N-acetyl-aminoethyl, 3-(2-pyrrolidone)propyl, morpholin-4-ylpropyl, optionally substituted phenylethyl, phenylpropyl, phenylbutyl, or phenylpentyl. Presently preferred R₄ groups of this type are hydrogen, methyl or morpholin-4-ylpropyl.

In compounds of the invention wherein Y is a group (ID), where R₃ and R₄ taken together represent a divalent chain of formula -C(R^{a})(R^{b})-A-Alk- wherein R^{a} and R^{b} are independently hydrogen or C₁-C₆ alkyl, A is a bond, -O-, -S-, -S-S-, -NH- or -NR^{a}- wherein R^{a} is C₁-C₆ alkyl, and Alk is C₁-C₆ alkylene, examples of such divalent chains include -C(CH₃)₂SCH₂CH₂CH₂-, and -C(CH₃)₂SSCH₂CH₂-.

In compounds of the invention wherein Y is a group (ID), examples of particular R₅ groups include hydrogen, methyl and ethyl. Presently preferred are compounds in which R₅ is methyl.

In compounds of the invention wherein Y is a group (IE), R₆ may be, for example, hydrogen, methyl, ethyl, benzyl or pyridylmethyl, and R₇ may be, for example hydrogen or methyl. R₆ and R₇ taken together with the carbon atom to which they are attached may form, for example, a cyciopentyl, cyclohexyl or morpholino ring. Presently preferred are compounds in which R₆ and R₇ are both hydrogen.

In compounds of the invention wherein Y is a group (IE), when R₇ is hydrogen, R₃ and R₆ taken together with the carbon atoms to which they are attached may form a 2-hydroxycyclohexyl or a glucose ring.

In a preferred subclass of the compounds of formula (I) of the invention
Ar is phenyl or 4-substituted phenyl, examples of the latter being 4-phenyl-phenyl, 4-phenoxy-phenyl, 4-(4'-chlorophenyl)-phenyl, 4-(4-cyanophenyl)-phenyl, 4-(4'-methoxy)-phenyl, 4-[4'-(2-methoxyethoxy)phenyl]-phenyl, 4-(pyridin-4-yl)-phenyl, 4-(pyridin-4-yloxy)-phenyl, 4-(4' -bromophenyl)-phenyl, 4-trifluoromethylphenyl, 4-fluorophenyl, 4-methoxyphenyl, 4-methoxyethoxyphenyl, 4-propylphenyl, 4-methylphenyl and 4-chlorophenyl;
n is 1;
X is -CONHOH,
R₁ is hydrogen, C₁-C₄alkyl, cyclopentyl, hydroxy, methoxy, allyl, or a group-(CH₂)ₜ-W wherein t represents 1, 2, 3 or 4 and W represents phthalimido, 1,2-dimethyl-3,5-dioxo-1,2,4-triazolidin-4-yl, 3,4,4-trimethyl-2,5-dioxo-1-imidazolidinyl, 2-methyl-3,5-dioxo-1,2,4-oxadiazol-4-yl, 3-methyl-2,4,5-trioxo-1-imidazolidinyl, 2,5-dioxo-3-phenyl-1-imidazolidinyl-2-oxo-1-pyrrolidinyl, 2,5-dioxo-1-pyrrolidinyl or 2,6-dioxo-piperidinylnaphthalimido (ie 1,3-dihydro-1,3-dioxo-2H-benz[f]isoindol-2-yl), 1,3-dihydro-1-oxo-2H-benz[f]isoindol-2-yl, 1,3-dihydro-1,3-dioxo-2H-pyrrolo[3,4-b] quinolin-2-yl, 2,3-dihydro-1,3-dioxo-1H-benz[d,e]isoquinolin-2-yl or saccharinyl;
R₃ represents t-butyl or 1-benzylthio-1-methylethyl, benzyl, methyl, or 3H-imidazol-4-ylmethyl;
Y is a group of formula (ID) wherein R₄ represents
   cyclopropyl, cyclobutyl, cyclopentyl, cyclohexyl, cycloheptyl or cyclooctyl;
   phenyl, 3-methoxyphenyl, pyridin-2-yl, pyridin-3-yl, thiazol-2-yl, 4-ethoxycarbonylmethylthiazol-2-yl, 5-methyl-1,3,4-thiadiazol-2-yl or 4-*tert*-butylthiazol-2-yl;
   a group -CHR^{x}R^{y} wherein R^{x} and R^{y} independently represent phenyl or 4-chlorophenyl or R^{x} and R^{y} are linked covalently in a 9-H-fluoren-9-yl ring;
   a polyether chain possessing at least two non-adjacent oxygen atoms, for example 2-(2-methoxyethoxy)ethyl; or
   hydrogen, methyl or 3-morpholin-4-ylpropyl;
   or R₃ and R₄ taken together represent -C(CH₃)₂SCH₂CH₂CH₂-, or-C(CH₃)₂SSCH₂CH₂-; and
   R₅ represents hydrogen or methyl;
or Y is a group of formula (IE) wherein R₆ and R₇ are both hydrogen;
and salts, hydrates and solvates thereof.

Interesting compounds of the invention include
3R-(3-biphenyl-4-yl-prop-2-ynyl)-N⁴-(2,2-dimethyl-1S-methylcarbamoyl-propyl)-2S,N¹-dihydroxy-succinamide;
N⁴-(2,2-dimethyl-1S-methylcarbamoyl-propyl)-2S,N¹-dihydroxy-3R-(3-phenyl-prop-2-ynyl)-succinamide;
N⁴-(2,2-dimethyl-1S-methylcarbamoyl-propyl)-2S,N¹-dihydroxy-3R-[3-(4-trifluoromethyl-phenyl)-prop-2-ynyl]-succinamide;
3R-[3-(4-chloro-phenyl)-prop-2-ynyl]-N⁴-(2,2-dimethyl-1S-methylcarbamoyl-propyl)-2S,N¹-dihydroxy-succinamide;
N⁴-(2,2-dimethyl-1S-methylcarbamoyl-propyl)-2S,N¹-dihydroxy-3R-[3-(4-methoxy-phenyl)-prop-2-ynyl]-succinamide,
3R-[3-(4'-chloro-biphenyl-4-yl)-prop-2-ynyl]-N⁴-(2,2-dimethyl-1S-methylcarbamoyl-propyl)-2S,N¹-dihydroxy-succinamide,
3R-[3-(4-cyanophenyl)-prop-2-ynyl]-N⁴-(2,2-dimethyl-1S-methylcarbamoyl-propyl)-2S,N¹-dihydroxy-succinamide,
3R-[3-(4-chloro-phenyl)-prop-2-ynyl]-N⁴-[2,2-dimethyl-1S-(3-morpholin-4-yl-propylcarbamoyl)-propyl]-2S,N¹-dihydroxy-succinamide,
N⁴-(2,2-dimethyl-1S-methylcarbamoyl-propyl)-2S,N¹-dihydroxy-3-{3-[4-(2-methoxy-ethoxy)-phenyl]-prop-2-ynyl}-succinamide,
3R-[3-(4-chloro-phenyl)-prop-2-ynyl]-2S,N¹-dihydroxy-N⁴-{1S-[2-(2-methoxy-ethoxy)-ethylcarbamoyl]-2,2-dimethyl-propyl}-succinamide,
3R-[3-(2-chloro-phenyl)-prop-2-ynyl]-N⁴-(2,2-dimethyl-1S-methylcarbamoyl-propyl)-2S,N¹-dihydroxy-succinamide,
3R-[3-(4-chloro-phenyl)-prop-2-ynyl]-N⁴-(1S-dimethylcarbamoyl-2,2-dimethyl-propyl)-2S,N¹-dihydroxy-succinamide,
N⁴-(1S-benzyl-2-hydroxy-ethyl)-3R-[3-(4-chloro-phenyl)-prop-2-ynyl]-2S,N¹-dihydroxy-succinamide,
3R-[3-(4-chloro-phenyl)-prop-2-ynyl]-N⁴-(2-cyclohexylmethyl-1S-hydroxymethyl-ethyl)-2S,N¹-dihydroxy-succinamide
3R-[3-(4-chloro-phenyl)-prop-2-ynyl]-2S,N¹-dihydroxy-N⁴-[1S-hydroxymethyl-2-(3H-imidazol-4-yl)-ethyl]-succinamide
N⁴-[2,2-dimethyl-1S-(pyridin-2-ylcarbamoyl)-propyl]-2S,N¹-dihydroxy-3R-(3-thiophen-2-ylprop-2-ynyl)-succinamide
N⁴-(1S-Benzyl-2R(or S)-hydroxy-propyl)-3R-[3-(4-chloro-phenyl)-prop-2-ynyl]-2,N¹-dihydroxy-succinamide
N⁴-(1S-benzyl-2R(or S)-hydroxy-propyl)-3R-[3-(4-chloro-phenyl)-prop-2-ynyl]-2,N¹-dihydroxy-succinamide
N⁴-(1-tert-butylcarbamoyl-2,2-dimethylpropyl)-3R-[3-(4-chloro-phenyl)-prop-2-ynyl]-2,N¹-dihydroxy-succinamide
3R-(3-biphenyl-4-yl-prop-2-ynyl)-N⁴-(1S(or R)-butylcarbamoyl-2,2-dimethyl-propyl)-2,N¹-dihydroxy-succinamide
3R(or S)-(3-biphenyl-4-yl-prop-2-ynyl)-N⁴-[2,2-dimethyl-1S-(pyridin-2-ylcarbamoyl)-propyl]-N¹-hydroxy-succinamide
6-(4-chlorophenyl)-3R-(2,2-dimethyl-1S-methylcarbamoyl-propylcarbamoyl)-2R(or S)-hydroxy-hex-5-ynoic acid
and salts, hydrates and solvates thereof.

Further interesting compound of the invention include:
N⁴-(1S-dimethylcarbamoyl-2,2-dimethyl-propyl)-2S,N¹-dihydroxy-3R-[3-(4-methoxy-phenyl)-prop-2-ynyl]-succinamide,
3R-(3-biphenyl-4-yl-prop-2-ynyl)-N⁴-(1S-dimethylcarbamoyl-2,2-dimethyl-propyl)-2S,N¹-dihydroxy-succinamide,
3R-[3-(4'-chloro-biphenyl-4-yl)-prop-2-ynyl]-N⁴-(1S-dimethylcarbamoyl-2,2-dimethyl-propyl)-2S,N¹-dihydroxy-succinamide,
3R-[3-(4-chloro-phenyl)-prop-2-ynyl]-2S,N¹-dihydroxy-N⁴-(1S-hydroxymethyl-2,2-dimethyl-propyl)-succinamide,
3R-[3-(4-chloro-phenyl)-prop-2-ynyl]-2S,N¹-dihydroxy-N⁴-[2-hydroxy-1-(3H-imidazol-4-ylmethyl)-ethyl]-succinamide,
3R-[3-(4'-chloro-biphenyl-4-yl)-prop-2-ynyl]-2S,N¹-dihydroxy-N⁴-(2-hydroxy-1S-methyl-ethyl)-succinamide,
N⁴-(1S-benzyl-2-hydroxy-ethyl)-3R-(3-biphenyl-4-yl-prop-2-ynyl)-2S,N¹-dihydroxy-succinamide,
3R-[3-(4-chloro-phenyl)-prop-2-ynyl]-N⁴-(1S-dimethylcarbamoyl-2,2-dimethyl-propyl)-N¹-hydroxy-succinamide,
N⁴-(2-benzylsulfanyl-1S-dimethylcarbamoyl-2-methyl-propyl)-3R-[3-(4-chloro-phenyl)-prop-2-ynyl]-2S,N¹-dihydroxy-succinamide,
and salts, hydrates and solvated thereof.

Compounds according to the present invention in which X is a hydroxamic acid group, -CONHOH may be prepared from corresponding compounds of the invention in which X is a carboxylic acid group -COOH or from the corresponding protected hydroxamic acid derivatives. That process, which forms another aspect of the invention, comprises causing an acid of general formula (II) 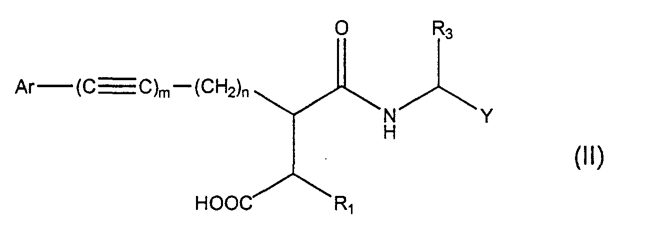 or an activated derivative thereof to react with hydroxylamine, O-protected hydroxylamine, or an N,O-diprotected hydroxylamine, or a salt thereof, Ar, m, n, R₁, R₃, and Y being as defined in general formula (I) except that any substituents in Ar, R₁, R₃, and Y which are potentially reactive with hydroxylamine, O-protected hydroxylamine, the N,O-diprotected hydroxylamine or their salts may themselves be protected from such reaction, then removing any protecting groups from the resultant hydroxamic acid moiety and from any protected substituents in Ar, R₁, R₃, and Y. In particular, when Y is a group of formula (IE), the terminal hydroxy group may optionally be protected during the foregoing reaction.

Conversion of (II) to an activated derivative such as the pentafluorophenyl, hydroxysuccinyl, or hydroxybenzotriazolyl ester may be effected by reaction with the appropriate alcohol in the presence of a dehydrating agent such as dicyclohexyl dicarbodiimide (DCC), N,N-dimethylaminopropyl-N'-ethyl carbodiimide (EDC), or 2-ethoxy-1-ethoxycarbonyl-1,2-dihydroquinoline (EEDQ).

Protecting groups as referred to above are well known per se, for example from the techniques of peptide chemistry. Amino groups are often protectable by benzyloxycarbonyl, t-butoxycarbonyl or acetyl groups, or in the form of a phthalimido group. Hydroxy groups are often protectable as readily cleavable ethers such as the t-butyl or benzyl ether, or as readily cleavable esters such as the acetate. Carboxy groups are often protectable as readily cleavable esters, such as the t-butyl or benzyl ester.

Examples of O-protected hydroxylamines for use in method (a) above include O-benzylhydroxylamine, 0-4-methoxybenzylhydroxylamine, O-trimethylsilylhydroxylamine, O-tert-butyldimethylsilylhydroxylamine and O-tert-butoxycarbonylhydroxylamine.

Examples of O,N-diprotected hydroxylamines for use in method (a) above include N,O-bis(benzyl)hydroxylamine, N,O-bis(4-methoxybenzyl)hydroxylamine, N-tert-butoxycarbonyl-O-tert-butyldimethylsilylhydroxylamine, N-tert-butoxycarbonyl-O-tetrahydropyranylhydroxylamine, and N,O-bis(tert-butoxycarbonyl)hydroxylamine.

In the special case where R₁ in compound (I) is hydroxy, a particularly useful technique may be reaction of hydroxylamine with a dioxalone of formula (IIa): 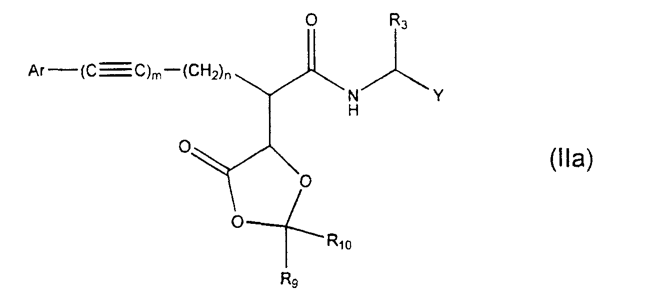 wherein the groups R₉ and R₁₀ are derived from a dioxalone forming reagent, and may be, for example, hydrogen, alkyl eg methyl, phenyl or substituted phenyl. The dioxalone ring is opened on reaction with hydroxylamine to give the required hydroxamic acid derivative of formula (I).

Compounds according to the present invention wherein X is a carboxylic acid group -COOH, ie compounds of formula (II) above, may be prepared by a process comprising: coupling an acid of formula (III) or an activated derivative thereof with an amine or aminoalcohol of formula (IV). 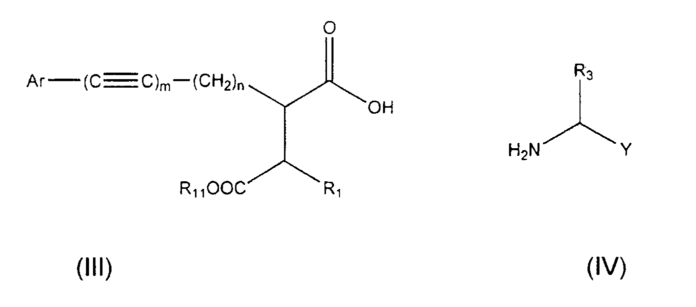 wherein Ar, m, n, R₁, R₃, and Y being as defined in general formula (I) except that any substituents in Ar, R₁, R₃, and Y which are potentially reactive in the coupling reaction may themselves be protected from such reaction, and R₁₁ represents a hydroxy protecting group, and subsequently removing the protecting group R₁₁ and any protecting groups from Ar, R₁, R₃, and Y. In particular, when Y is a group of formula (IE), the terminal hydroxy group may optionally be protected during the coupling reaction of (IV) with (III).

An alternative procedure for the preparation of compounds of formulae (II)/(IIa) wherein Y is a group of formula (ID) and R₅ is H involves a Ugi 4-component condensation reaction between (III) and ammonia, an aldehyde (R₃CHO) and an isonitrile (CNR₄), as described in WO 96/26918.

Active derivatives of acids (III) include activated esters such as the pentafluorophenyl ester, acid anhydrides and acid halides, eg chlorides. Suitable hydroxy protecting groups may be selected from those known in the art.

Amino acid amides and amino alcohols of formula (IV) are either known or are prepared by routine known synthetic methods. Compounds of formula (III) may in many cases be prepared by alkylation of a compound of formula (V) with a halide of formula (VI)

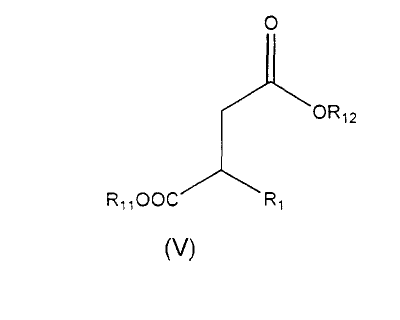

H―(C≡C)ₘ―(CH₂)ₙ―L (VI)

wherein R₁, m and n are as defined in relation to formula (I) R₁₁ and R₁₂ are carboxyl protecting groups, and L is a reactive halo group (eg bromo) capable of reacting with (V) at the C atom adjacent the -COOR₁₂ group with elimination of the elements of HL and then reacting the resultant compound of formula (VII) with a halide of formula (VIII) under palladium catalysis (the Heck reaction; see R. F. Heck, *Palladium Reagents in Organic Synthesis,* Academic Press, London 1985).

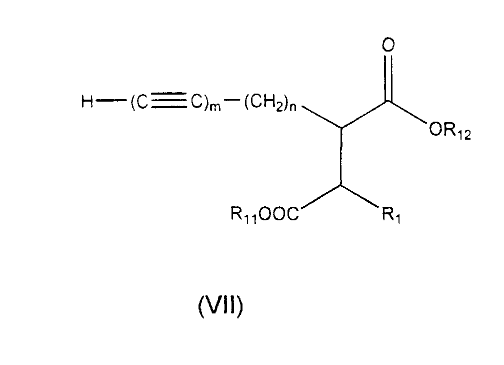

Ar―L (VIII)

again with elimination of the elements of HL, and thereafter removing protecting group R₁₂ or removing both protecting groups R₁₁ and R₁₂ and reprotecting the carboxylic acid group adjacent the group R₁. The terminal hydrogen of compound (VI) may optionally be protected during the reaction of (VI) with (V), for example as a silyl derivative such as trimethylsilyl. In the specific case where R₁ is hydroxy, the protected intermediate (VIIa) is suitable: 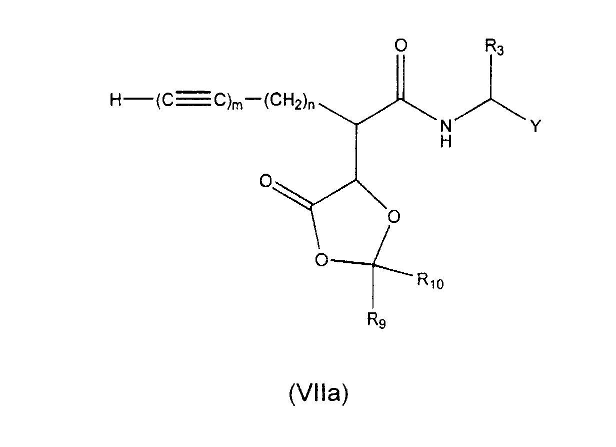 wherein R₉ and R₁₀ are as defined in relation to formula (Ila) above.

Compounds (VII) wherein R₁ is hydrogen and m is 1 may be prepared by ozonolysis of a compound (VIIb) 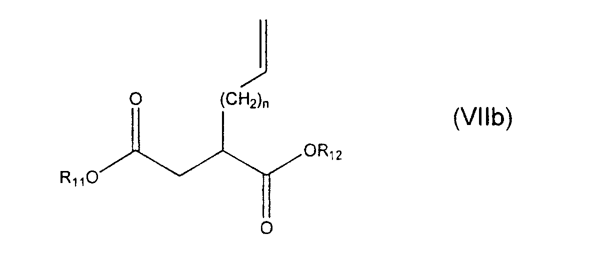 wherein R₁₁ and R₁₂ are carboxyl protecting groups and n is as defined in formula (I), followed by treatment of the resulting compound (VIIc) with a compound (X) as generally described in Synlett, 1996, 521 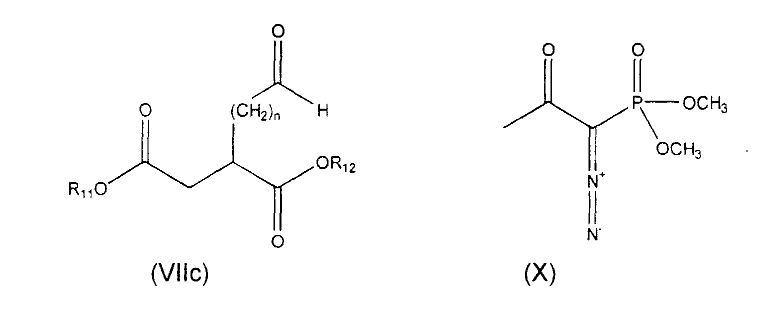 Compounds (VII) wherein R₁ is hydrogen and m is 2 may be prepared by reaction of a compounds (VII) wherein R₁ is hydrogen and m is 1 with a compound of formula (Villa)

Ar―C≡C―L (VIIa)

wherein L is a reactive halo group (eg bromo).

Yet another route to compounds (VII) where R₁ is hydrogen comprises alkylation of a compound (XI) with a compound (XII) 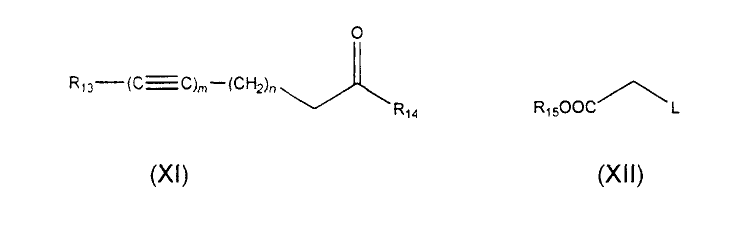 wherein R₁₃ is a protecting group such as trimethylsilyl, R₁₄ is a chiral auxiliary such as 4S-benzyloxyazolidin-2-one, R₁₅ is a carboxyl protecting group, and L is a leaving group such as a halide, to form (XIII) 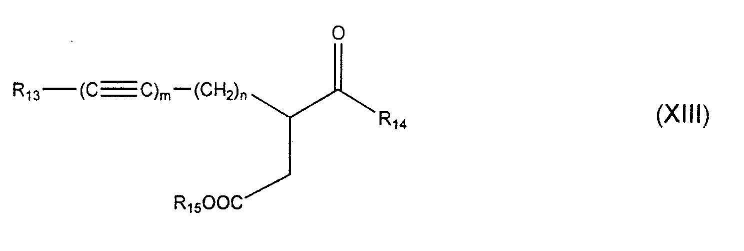 which is then deprotected and reprotected as appropriate to form the desired compound (VII).

Compounds (VII) wherein R₁ is hydrogen may be used as intermediates in the preparation of compounds wherein R₁ is one of a variety of substituents by known methods, eg alkylation (WO 94/21625), Claisen rearrangement (GB patent application No 9610985.5) or carboxylation followed by the Mannich and Michael reactions (WO 90/05719).

In some cases it may be possible to prepare componds (III) using only one alkylation step, by reacting compound (V) with a compound of formula (IX)

Ar―(C≡C)ₘ―(CH₂)ₙ―L (IX)

wherein L is a reactive halo group (eg bromo) capable of reacting with (V) at the C atom adjacent the -COOR₁₂ group with elimination of the elements of HL.

As mentioned above, compounds of formula (I) are useful in human or veterinary medicine since they are active as inhibitors of MMPs.

Accordingly in another aspect, this invention concerns:
(i) a method of management (by which is meant treatment or prophylaxis) of diseases or conditions mediated by MMPs in mammals, in particular in humans, which method comprises administering to the mammal an effective amount of a compound as defined with respect to formula (I) above, or a pharmaceutically acceptable salt thereof; and
(ii) a compound as defined with respect to formula (I) for use in human or veterinary medicine, particularly in the management (by which is meant treatment or prophylaxis) of diseases or conditions mediated by MMPs; and
(iii) the use of a compound as defined with respect to formula (I) in the preparation of an agent for the management (by which is meant treatment or prophylaxis) of diseases or conditions mediated by MMPs.

Diseases or conditions mediated by MMPs include those involving tissue breakdown such as bone resorption, inflammatory diseases, dermatological conditions and tumour invasion by secondary metastases, in particular rheumatoid arthritis, osteoarthritis, periodontitis, gingivitis, corneal ulceration and tumour invasion by secondary metastases as well as neuroinflammatory disorders, including those involving myelin degradation, for example multiple sclerosis.

In a further aspect of the invention there is provided a pharmaceutical or veterinary composition comprising a compound of formula (I) together with a pharmaceutically or veterinarily acceptable excipient or carrier.

One or more compounds of general formula (I) may be present in the composition together with one or more excipient or carrier.

The compounds with which the invention is concerned may be prepared for administration by any route consistent with their pharmacokinetic properties. The orally administrable compositions may be in the form of tablets, capsules, powders, granules, lozenges, liquid or gel preparations, such as oral, topical, or sterile parenteral solutions or suspensions. Tablets and capsules for oral administration may be in unit dose presentation form, and may contain conventional excipients such as binding agents, for example syrup, acacia, gelatin, sorbitol, tragacanth, or polyvinyl-pyrrolidone; fillers for example lactose, sugar, maize-starch, calcium phosphate, sorbitol or glycine; tabletting lubricant, for example magnesium stearate, talc, polyethylene glycol or silica; disintegrants for example potato starch, or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, syrup, methyl cellulose, glucose syrup, gelatin hydrogenated edible fats; emulsifying agents, for example lecithin, sorbitan monooleate, or acacia; non-aqueous vehicles (which may include edible oils), for example almond oil, fractionated coconut oil, oily esters such as glycerine, propylene glycol, or ethyl alcohol; preservatives, for example methyl or propyl p-hydroxybenzoate or sorbic acid, and if desired conventional flavouring or colouring agents.

The dosage unit involved in oral administration may contain from about 1 to 250mg, preferably from about 5 to 100mg of a compound of the invention. A suitable daily dose for a mammal may vary widely depending on the condition of the patient. However, a dose of a compound of general formula I of about 0.1 to 10mg/kg body weight, particularly from about .1 to 3mg/kg body weight may be appropriate.

For topical application to the skin, the drug may be made up into a cream, lotion or ointment. Cream or ointment formulations which may be used for the drug are conventional formulations well known in the art, for example as described in standard textbooks of pharmaceutics such as the British Pharmacopoeia.

For topical application to the eye, the drug may be made up into a solution or suspension in a suitable sterile aqueous or non aqueous vehicle. Additives, for instance buffers such as sodium metabisulphite os disodium edeate; preservatives including bactericidal and fungicidal agents such as phenyl mercuric acetate or nitrate, benzalkonium chloride or chlorhexidine, and thickening agents such as hypromellose may also be included.

The dosage for topical administration will of course depend on the size of the area being treated. For the eyes, each dose may typically be in the range from 10 to 100mg of the drug.

The active ingredient may also be administered parenterally in a sterile medium. Depending on the vehicle and concentration used, the drug can either be suspended or dissolved in the vehicle. Advantageously, adjuvants such as a local anaesthetic, preservative and buffering agents can be dissolved in the vehicle.

For use in the treatment of rheumatoid arthritis, the drug can be administered by the oral route or by injection intra-articularly into the affected joint.

The examples which follow serve to illustrate the preparation of compounds the invention but are not intended to limit the scope in any way. Amino acid derivatives and amino alcohols were available commercially or were prepared according to literature methods. Unless stated otherwise, starting aryl and alkynyl halides were obtained from commercial suppliers.

The following abbreviations have been used throughout:
- EDC: N-Ethyl-N'(3-dimethylaminopropyl)carbodiimide hydrochloride
- HOBt: 1-Hydroxybenzotriazole
- LDA: Lithium diisopropylamide
- THF: Tetrahydrofuran
- HPLC: High Performance Liquid Chromatography
- TFA: Trifluoroacetic acid

¹H and ¹³C NMR spectra were recorded using a Bruker AC 250E spectrometer at 250.1 and 62.9 MHz, respectively. Elemental microanalyses were performed by MEDAC Ltd, Department of Chemistry, Brunel University, Uxbridge, Middlesex, UB8 3PH.

### EXAMPLE 1

### 3R-(3-Biphenyl-4-yl-prop-2-ynyl)-N⁴-(2,2-Dimethyl-1S-methylcarbamoyl-propyl)-2S,N¹-dihydroxy-succinamide

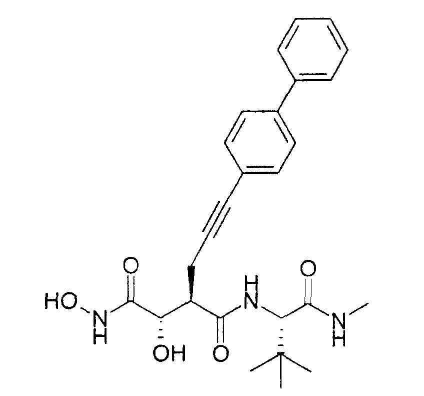

The title compound was prepared according to the route outlined in Scheme 1 and is described in detail below.

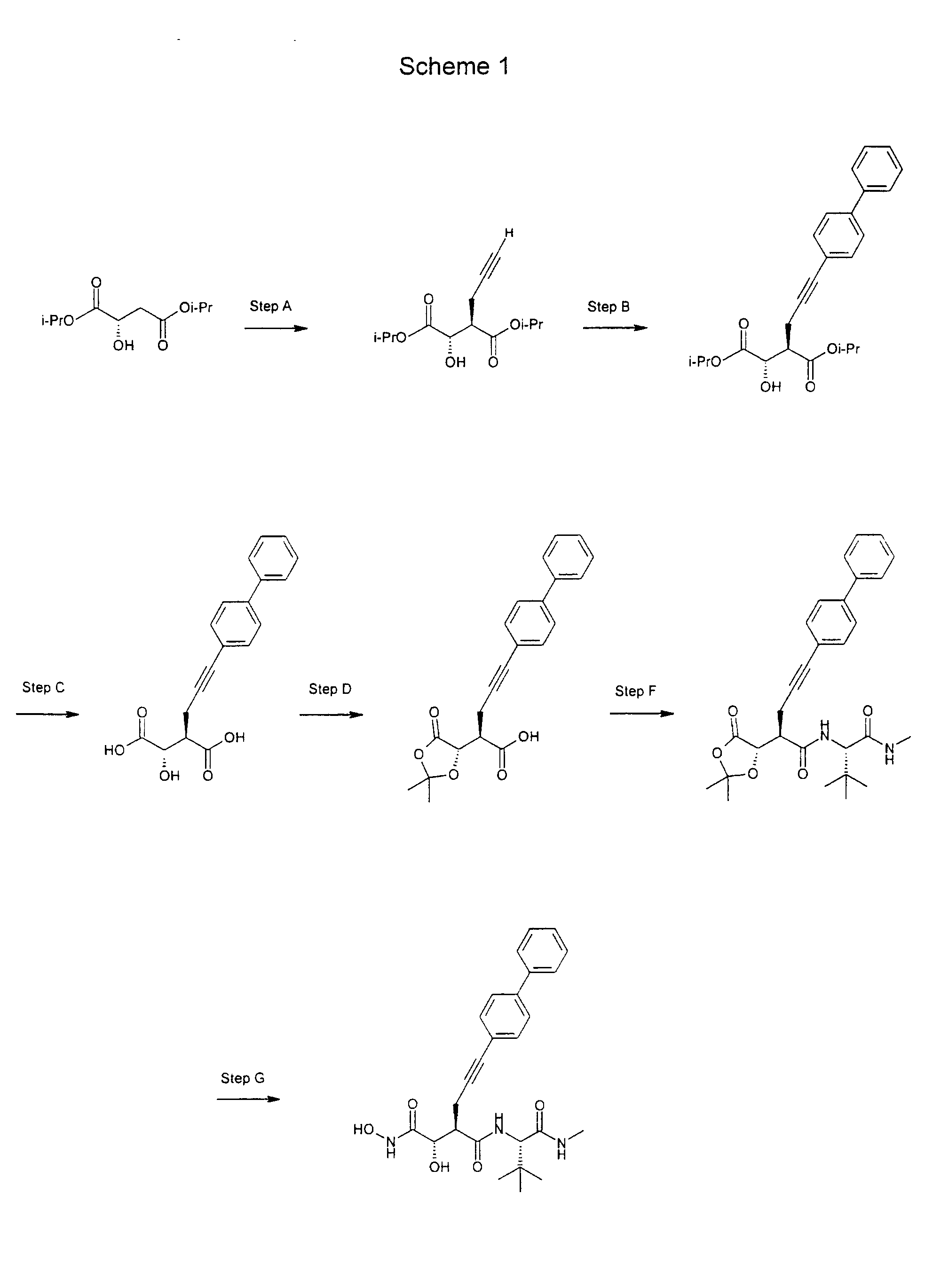

### STEP A:

### 2S-Hydroxy-3R-prop-2-ynyl-succinic acid diisopropyl ester

A solution of S-malic acid diisopropyl ester (5.5 g, 25.23 mmol) in dry THF (20 ml) was added to a solution of freshly prepared LDA [from N,N-diisopropylamine (6.9 ml, 52.5 mmol) and 2.5 M n-butyllithium (21 ml, 52.5 mmol)] in dry THF (50 ml), whilst maintaining the temperature at -5°C. The reaction mixture was stirred at -5°C for 75 minutes then cooled to -70°C. A solution of propargyl bromide (80% solution in toluene, 3.10 ml, 27.75 mmol) was added slowly, whilst maintaining the temperature at -70°C. The cooling bath was removed and the solution was stirred overnight before quenching with saturated aqueous ammonium chloride (50 ml). The aqueous layer was separated and extracted with ethyl acetate (2 x 20 ml). The organic layers were combined and washed with 1M hydrochloric acid (2 x 20 ml) and brine (20 ml) and dried over anhydrous magnesium sulphate. The solution was filtered and concentrated *in vacuo* to give a brown oil which was purified by column chromatography (silica gel, 25% ethyl acetate in hexanes) to provide the title compound as an orange oil (1.4 g, 5.46 mmol, 22%; 9:1 mixture of diastereomers by NMR). ¹H-NMR; δ (CDCl₃, major diastereoisomer), 5.12 (1H, m), 5.04 (1H, m), 4.45 (1H, dd, J = 5.8, 2.6 Hz), 3.17 (1H, d, J = 5.8 Hz), 3.08 (1H, ddd, J = 5.8, 2.5 Hz), 2.67 (1H, m), 2.05 (1H, t, J = 2.9 Hz), 1.29 (6H, d, J = 6.1 Hz) and 1.19 (6H, d, J = 6.2 Hz).

### STEP B:

### 3R-(3-Biphenyl-4-yl-prop-2-ynyl)-2S-hydroxy-succinic acid diisopropyl ester

2S-Hydroxy-3R-prop-2-ynyl-succinic acid diisopropyl ester (0.51 g, 2 mmol) and 4-bromobiphenyl (0.35 g, 1.5 mmol) were dissolved in diisopropylamine (4 ml) in a 5 ml Wheaton vial. Palladium (48 mg, 4.6 mol%) and copper (I) iodide (12 mg, 4.0 mol%) were added and the vial was sealed and heated at 130°C for 3.5 hours then stirred at room temperature overnight. The solution was filtered through celite, flushing with dichloromethane (30 ml). The filtrate was washed with 1M hydrochloric acid (2 x 20 ml) and brine (20 ml), dried (anhydrous magnesium sulphate) and filtered. The solvents were removed *in vacuo* and the residue was purified by column chromatography (silica gel, 25% ethyl acetate in hexanes) to provide the title compound as a yellow oil (0.42 g, 1.03 mmol, 68%). ¹H-NMR; δ (CDCl₃), 7.53 (9H, m), 5.16 (1H, m), 5.06 (1H, m), 4.55 (1H, d, J = 2.5 Hz), 3.2 (1H, ddd, J = 5.5, 2.5 Hz), 2.94 (2H, m), 1.32 (6H, d, J = 6.3 Hz) and 1.25 (6H, d, J = 6.2 Hz).

### STEP C:

### 3R-(3-Biphenyl-4-yl-prop-2-ynyl)-2S-hydroxy-succinic acid

A solution of 3R-(3-biphenyl-4-yl-prop-2-ynyl)-2S-hydroxy-succinic acid diisopropyl ester (0.7 g, 1.71 mmol) in 1M sodium hydroxide (6 ml, 6 mmol) was heated at reflux for 1 hour then cooled to room temperature. The solution was washed with dichloromethane (20 ml), acidified to pH 2 with 1M hydrochloric acid and extracted with ethyl acetate (3 x 10 ml). Organic extracts were combined, dried over anhydrous magnesium sulphate, filtered and evaporated to provide product as a light brown foam (0.48 g, 1.48 mmol, 86%). ¹H-NMR; δ (CDCl₃), 7.55 (9H, m), 4.76 (1H, d, J = 2.5 Hz), 4.18 (3H, br s), 3.37 (1H, m) and 3.01 (2H, m).

### STEP D:

### 5-Biphenyl-4-yl-2R-(2,2-dimethyl-5-oxo-[1,3]dioxolan-4S-yl)-pent-4-ynoic acid

A solution of 3R-(3-biphenyl-4-yl-prop-2-ynyl)-2S-hydroxy-succinic acid (0.48 g, 1.48 mmol) in ethyl acetate (5 ml) and 2,2-dimethoxypropane (5 ml) containing a catalytic amount of p-toluenesulfonic acid (10 mg) was heated at reflux for 2.5 hours. Solvents were removed *in vacuo* to provide the title compound as a brown foam (0.55 g, 1.5 mmol, quantitative). ¹H-NMR; δ (CDCl₃), 7.54 (9H, m), 4.88 (1H, d, J = 2.5 Hz), 3.33 (1H, m), 3.11 (1H, dd, J = 17.2, 5.2 Hz), 2.92 (1H, dd, J = 14.5, 4.4 Hz), 1.62 (3H, s) and 1.60 (3H, s).

### STEP E:

### 5-Biphenyl-4-yl-2R-(2,2-dimethyl-5-oxo-[1,3]dioxolan-4S-yl)-pent-4-ynoic acid (2,2-dimethyl-1S-methylcarbamoyl-propyl)-amide

To a solution of 5-biphenyl-4-yl-2R-(2,2-dimethyl-5-oxo-[1,3]dioxolan-4S-yl)-pent-4-ynoic acid (0.55 g, 1.5 mmol) in dichloromethane (20 ml) was added HOBt (0.22 g, 1.65 mmol) followed by EDC (0.36 g, 1.87 mmol). The mixture was heated at reflux for 1 hour then cooled to room temperature. L*-tert*-Leucine-N-methylamide (0.22 g, 1.5 mmol) was added and the resulting solution was heated at reflux for 4 hours. The solution was allowed to cool to room temperature and washed with 1M hydrochloric acid (20 ml), saturated sodium hydrogen carbonate (20 ml) and brine (20 ml), dried (magnesium sulphate), filtered and concentrated *in vacuo.* The product was purified by column chromatography (silica gel, 50% ethyl acetate in hexanes) to provide the title compound as a yellow solid (140 mg, 0.28 mmol, 19%). ¹H-NMR; δ (CDCl₃), 7.54 (9H, m), 6.85 (1H, d, J = 9.2 Hz), 6.45 (1H, m), 4.75 (1H, d, J = 5.2 Hz), 4.34 (1H, d, J = 9.2 Hz), 3.02 (3H, m), 2.73 (3H, d, J = 4.8 Hz), 1.63 (3H, s), 1.57 (3H, s) and 1.02 (9H, s).

### STEP F:

### 3R-(3-Biphenyl-4-yl-prop-2-ynyl)-N⁴-(2,2-Dimethyl-1S-methylcarbamoyl-propyl)-N¹, 2S-dihydroxy-succinamide

A suspension of sodium methoxide (46 mg, 0.85 mmol) and hydroxylamine hydrochloride (59 mg, 0.85 mmol) in methanol (2 ml) was stirred at room temperature for 1.5 hours then filtered into a solution of 5-biphenyl-4-yl-2R-(2,2-dimethyl-5-oxo-[1,3]dioxolan-4S-yl)-pent-4-ynoic acid (2,2-dimethyl-1S-methylcarbamoyl-propyl)-amide (140 mg, 0.28 mmol) and the resulting solution was stirred at room temperature overnight. Solvents were removed *in vacuo* and the residue was purified by column chromatography (acid-washed silica gel, 10% methanol in dichloromethane) to provide the title compound as a white solid (25 mg, 0.054 mmol, 19%). ¹H-NMR; δ (CD₃OD), 7.97 (1H, s), 7.85 (1H, d, J = 9.1 Hz), 7.32 (9H, m), 4.75 (1H, s), 4.17 (1H, dd, J = 9.1, 3.5 Hz), 3.01 (1H, m), 2.66 (2H, m), 2.50 (3H, s), 1.74 (1H, d, J = 2.8 Hz) and 0.90 (9H, s). ¹³C-NMR; δ (CD₃OD), 176.2, 175.4, 166.8, 144.4, 144.0, 135.6, 132.3, 131.1, 130.2, 126.1, 90.1, 85.8, 74.8, 64.7, 52.8, 38.0, 29.6, 28.4 and 23.0.

### EXAMPLE 2

### N⁴-(2,2-Dimethyl-1S-methylcarbamoyl-propyl)-2S,N¹-dihydroxy-3R-(3-phenyl-prop-2-ynyl)-succinamide

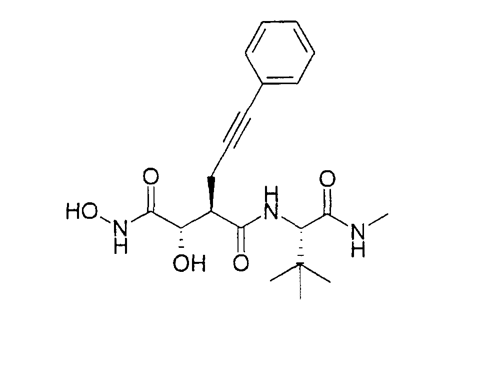

The title compound was prepared according to the route outlined in Scheme 2 and is described in detail below.

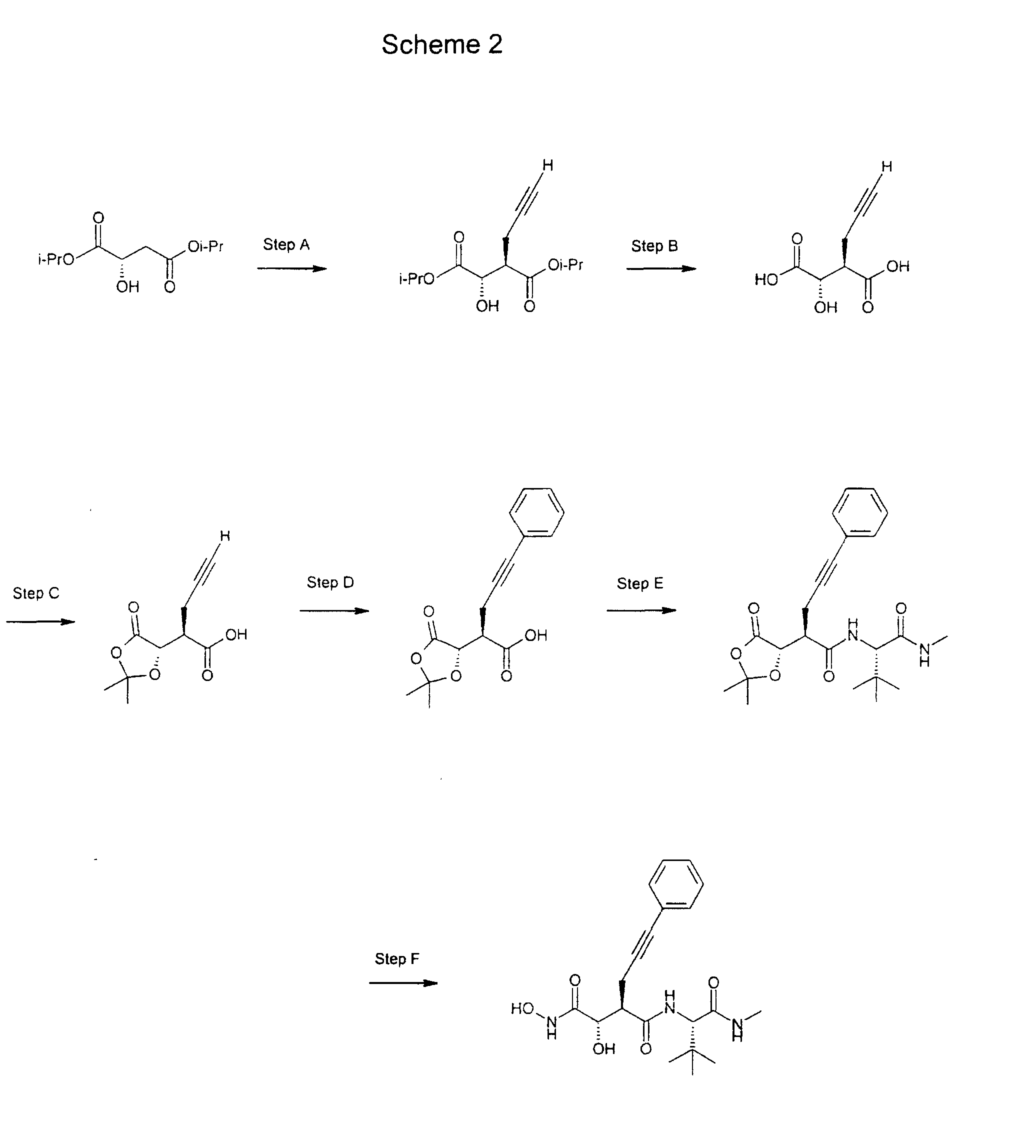

### STEP A:

### Identical to example 1, step A

### STEP B:

### 2S-Hydroxy-3R-prop-2-ynyl-succinic acid

A solution of 2S-hydroxy-3R-prop-2-ynyl-succinic acid diisopropyl ester (2.47 g, 9.56 mmol) in 1M sodium hydroxide (32 ml, 32 mmol) was heated at reflux for 1 hour then cooled to room temperature. The solution was acidified to pH 2 with 1M hydrochloric acid and extracted with ethyl acetate (5 x 10 ml). The combined organics were dried over magnesium sulphate, filtered and concentrated *in vacuo* to provide the title compound as a brown oil (0.94 g, 6.18 mmol, 64%). ¹H-NMR; δ (CD₃OD), 4.37 (1H, d, J = 3.4 Hz), 3.01 (1H, m), 2.51 (2H, m), 2.21 (1H, t, J = 2.5 Hz)

### STEP C:

### 2R-(2,2-Dimethyl-5-oxo-[1,3]dioxolan-4S-yl)-pent-4-ynoic acid

2S-Hydroxy-3R-prop-2-ynyl-succinic acid (0.94 g, 6.18 mmol) was dissolved in ethyl acetate (5 ml). Dimethoxypropane (10 ml) and p-toluenesulfonic acid (10 mg) were added and the solution heated at reflux for 2.5 hours. Solvents were removed in vacuo to provide the title compound as a dark brown gum (1.0 g, 5.2 mmol, 84%). ¹H-NMR; δ (CDCl₃), 4.80 (1H, d, J = 2.4 Hz), 3.22 (1H, m), 2.86 (1H, ddd, J = 17.2, 5.4, 2.6 Hz), 2.61 (1H, ddd, J = 13.0, 10.3, 2.6 Hz), 2.10 (1H, t, J = 2.8 Hz), 1.58 (3H, s) and 1.57 (3H, s).

### STEP D:

### 2R-(2,2-Dimethyl-5-oxo-[1,3]dioxolan-4S-yl)-5-phenyl-pent-4-ynoic acid

2R-(2,2-Dimethyl-5-oxo-[1,3]dioxolan-4S-yl)-pent-4-ynoic acid (3 g, 15.62 mmol) and iodobenzene (1.31 ml, 11.74 mmol) were dissolved in diisopropylamine (30 ml) in a 35 ml pressure tube. Dichlorobis(triphenylphosphine)palladium(II) (379 mg, 4.6 mo%) and copper (I) iodide (89 mg, 4.0 mo%) were added and the tube heated at 130°C for 3.5 hours. The solution was filtered through celite, washing with dichloromethane (100 ml) and the organic solution washed with 1M hydrochloric acid (3 x 50 ml) then extracted with saturated aqueous sodium hydrogen carbonate (4 x 30 ml). The basic extracts were combined, acidified to pH2 with 1M hydrochloric acid then extracted with dichloromethane (3 x 30 ml). Combined organics were dried (magnesium sulphate), filtered and solvents removed *in vacuo* to provide the title compound as an orange solid (2.1 g, 7.83 mmol, 67 %). ¹H-NMR; δ (CDCl₃), 7.32 (5H, m), 4.86 (1H, d, J = 3.0 Hz), 3.32 (1H, m), 3.10 (1H, dd, J = 17.13, 5.2 Hz), 2.88 (1H, dd, J = 17.3, 10.4 Hz), 1.61 (3H, s) and 1.59 (3H, s).

### STEP E:

### 2R-(2,2-Dimethyl-5-oxo-[1,3]dioxalan-4S-yl)-5-phenyl-pent-4-ynoic acid (2,2-dimethyl-1S-methylcarbamoyl-propyl)-amide

A solution of 2R-(2,2-dimethyl-5-oxo-[1,3]dioxolan-4S-yl)-5-phenyl-pent-4-ynoic acid (0.7 g, 2.61 mmol) and HOBt (0.42 g, 3.13 mmol) in dichloromethane (35 ml) was cooled to 0°C and treated with EDC (0.63 g, 3.26 mmol). The ice bath was removed and the solution stirred for 2 hours. L-*tert*-Leucine-N-methyl amide (0.45 g, 3.13.mmol) was then added and the resulting solution was stirred at room temperature overnight. The solution was washed with 1M hydrochloric acid (2 x 20 ml), saturated aqueous sodium hydrogen carbonate (2 x 20 ml) and brine (20 ml), dried over anhydrous magnesium sulphate, filtered and concentrated *in vacuo.* The product was purified by column chromatography (silica gel, 50% ethyl acetate in hexanes) to provide the title compound as a yellow foam (0.25 g, 0.63 mmol, 24%). ¹H-NMR; δ (CDCl₃), 7.29 (5H, m), 6.73 (1H, d, J = 9.0 Hz), 6.06 (1H, m), 4.74 (1H, d, J = 5.3 Hz), 4.23 (1H, d, J = 9.2 Hz), 3.01 (2H, m), 2.87 (1H, dd, J = 18.1, 10.0 Hz), 2.71 (3H, d, J = 4.8 Hz), 1.62 (3H, s), 1.57 (3H, s) and 1.00 (9H, s).

### STEP F:

### N¹-(2,2-Dimethyl-1S-methylcarbamoyl-propyl)-N⁴-hydroxy-2R-(phenyl-4-yl-prop-2-ynyl)-3S-hydroxy-succinamide.

A suspension of sodium methoxide (102 mg, 1.9 mmol) and hydroxylamine hydrochloride (131 mg, 1.9 mmol) in methanol (5 ml) was stirred at room temperature for 1.5 hours then filtered into a solution of 2R-(2,2-dimethyl-5-oxo-[1,3]dioxalan-4S-yl)-5-phenyl-pent-4-ynoic acid (2,2-dimethyl-1S-methylcarbamoyl-propyl)-amide (250 mg, 0.63 mmol) and the resulting solution was stirred at room temperature overnight. Solvents were removed *in vacuo* and the residue was purified by column chromatography (acid-washed silica gel, 10% methanol in dichloromethane) to provide the title compound as an off-white solid (77 mg, 31%). m.p. 186.5 -187°C. ¹H-NMR; δ (CD₃OD), 7.23 (5H, m), 4.19 (1H, d, J = 5.8 Hz), 4.15 (1H, s), 2.98 (1H, m), 2.63 (2H, dd, J = 7.8Hz), 2.48 (3H, s) and 0.88 (9H, s). ¹³C-NMR; δ (CD₃OD), 176.2, 175.4, 135.1, 131.7, 131.4, 127.2, 89.4, 85.9, 64.7, 52.8, 38.0, 29.6, 28.4 and 22.9.

The following additional compounds were prepared by the method of Example 2, substituting the appropriate aryl/biaryl/heteroaryl halide or 1-bromo-2-phenylacetylene for iodobenzene in Step D.

### EXAMPLE 3

### N⁴-(2,2-Dimethyl-1S-methylcarbamoyl-propyl)-2S,N¹-dihydroxy-3R-[3-(4-trifluoromethyl-phenyl)-prop-2-ynyl]-succinamide

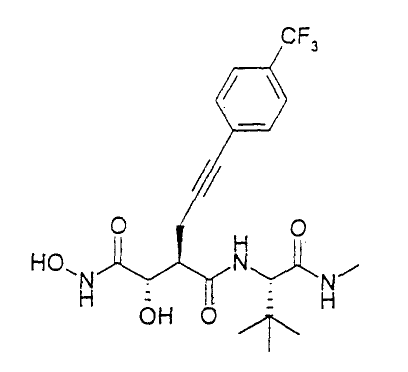

Pink solid. m.p. 186.5 - 188°C. 1H-NMR; δ (CD₃OD), 7.47 (4H, m), 4.17 (2H, m), 3.00 (1H, m), 2.66 (2H, m), 2.48 (3H, s) and 0.88 (9H, s). ¹³C-NMR; δ (CD₃OD), 176.0, 175.4, 174.1, 135.6, 133.0 (d, J_{CF} = 3.4 Hz), 127.9 (d, J_{CF} = 270.9 Hz), 92.7, 84.6, 74.6, 69.3, 64.2, 52.6, 38.0, 29.6, 28.3, 22.9 and 17.8. IR: vₘₐₓ (KBr), 3317, 2964, 2358, 1635, 1528, 1327, 1168, 1120, 1068 and 840 cm⁻¹.

### EXAMPLE 4

### N⁴-(2,2-Dimethyl-1S-methylcarbamoyl-propyl)-2S,N¹-dihydroxy-3R-[3-(4-methoxy-phenyl)-prop-2-ynyl]-succinamide

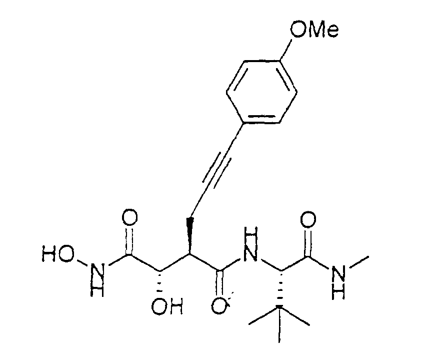

White solid. ¹H-NMR; δ ((CD₃)₂SO), 8.87 (1H, s), 7.89 (1H, m), 7.65 (1H, d, J = 9.5 Hz), 7.25 (2H, d, J = 8.7 Hz), 6.87 (2H, d, J = 8.7 Hz), 5.54 (1H, d, J = 7.6 Hz), 4.24 (1H, d, J = 9.5 Hz), 3.91 (1H, t, J = 7.7 Hz), 3.74 (3H, s), 2.93 (1H, m), 2.57 (1H, m), 2.50 (3H, d, J = 4.0 Hz), 2.38 (1H, dd, J = 5.4, 16.5 Hz) and 0.90 (9H, s). ¹³C-NMR; δ ((CD₃)₂SO), 170.8, 170.3, 168.4, 136.0, 131.4, 115.2, 112.6, 85.9, 81.3, 71.7, 58.6, 56.3, 54.0, 34.4, 27.7, 26.3 and 25.6.

### EXAMPLE 5

### 3R-[3-(4'-Chloro-biphenyl-4-yl)-prop-2-ynyl]-N⁴-(2,2-dimethyl-1S-methylcarbamoyl-propyl)-2S,N¹-dihydroxy-succinamide

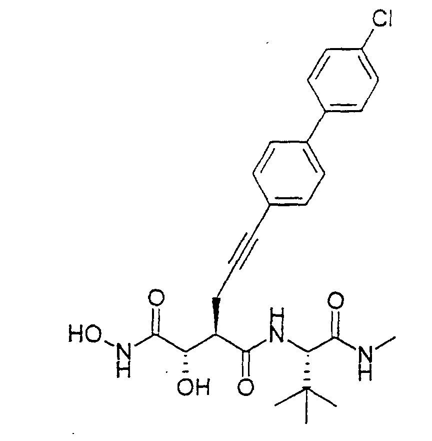

White solid. ¹H-NMR; δ ((CD₃)₂SO), 10.69 (1H, s), 8.89 (1H, s), 7.93 (1H, m), 7.70 (2H, d, J = 8.5 Hz), 7.64 (2H, d, J = 8.1 Hz), 7.51 (2H, d, J = 8.5 Hz), 7.42 (2H, d, J = 8.1 Hz), 5.58 (1H, d, J = 7.6 Hz), 4.25 (1H, d, J = 9.4 Hz), 3.93 (1H, d, J = 7.6 Hz), 2.99 (1H, m), 2.51 (5H, m) and 0.90 (9H, s). ¹³C-NMR; δ ((CD₃)₂SO), 175.8, 175.5, 173.5, 143.1, 137.7, 137.1, 134.0, 133.4, 131.6, 127.6, 127.5, 94.0, 86.3, 75.6, 64.9, 53.6, 39.5, 31.8, 30.3 and 24.1.

The starting material was prepared as follows:
A mixture of 4-chloro-iodobenzene (0.99 g, 4.15 mmol), (4-bromophenyl)boronic acid (1.0 g, 4.98 mmol), anhydrous potassium carbonate (860 mg, 6.22 mmol) and dichlorobis(triphenylphosphine) palladium (II) (87 mg, 0.12 mmol) in toluene (40 ml) was heated at 90°C for 4 hours under an argon atmosphere. The solvent was removed under reduced pressure and the residue was dissolved in dichloromethane (50 ml). The organic solution was washed successively with 1M hydrochloric acid (2x30 ml), saturated aq. sodium hydrogen carbonate (2x30 ml) and brine, dried over anhydrous magnesium sulfate and filtered. The solvent was removed under reduced pressure to leave an orange solid, which was purifed by trituration with ethyl acetate-hexane (1:6) to provide 4'-chloro-4-bromo-biphenyl (0.5 g, 45%) as a pale orange solid. ¹H-NMR; δ (CDCl₃) 7.49 (m).

### EXAMPLE 6

### 3R-[3-(4-Cyanophenyl)-prop-2-ynyl]-N⁴-(2,2-dimethyl-1S-methylcarbamoyl-propyl)-2S,N¹-dihydroxy-succinamide

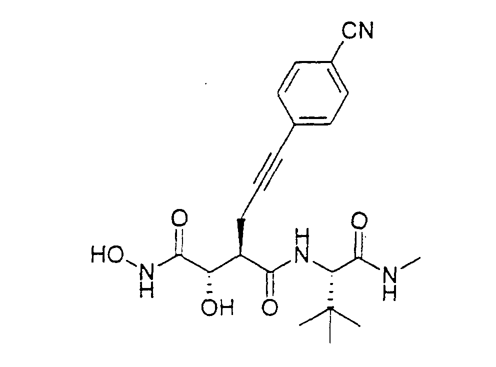

White solid. ¹H-NMR; δ ((CD₃)₂SO), 10.68 (1H, (s), 8.88 (1H, s), 7.91 (1H, d, J = 4.3 Hz), 7.81 (2H, d, J = 8.2 Hz), 7.73 (1H, d, J = 9.5 Hz), 7.50 (2H, d, J = 8.2 Hz), 5.56 (1H, br s), 4.24 (1H, d, J = 9.5 Hz), 3.91 (1H, d, J = 8.3 Hz), 2.58 (1H, dd, J = 9.7, 16.8 Hz), 2.49 (4H, m) and 0.89 (9H, s). ¹³C-NMR; δ ((CD₃)₂SO), 175.7, 175.5, 173.4, 137.3, 133.1, 132.2, 123.6, 115.4, 97.7, 85.4, 75.6, 64.9, 53.3, 39.4, 31.7, 30.2 and 24.1.

### EXAMPLE 7

### 3R-[3-(4-Chloro-phenyl)-prop-2-ynyl]-N⁴-[2,2-dimethyl-1S-(3-morpholin-4-yl-propylcarbamoyl)-propyl]-2S,N¹-dihydroxy-succinamide

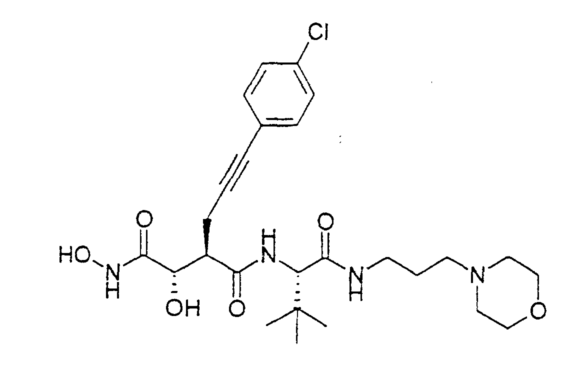

White solid. m.p. 108 - 110°C. ¹H-NMR; δ (CD₃OD), 7.24 (4H, m), 4.30 (1H, d, J = 4.4 Hz), 3.98 (1H, s), 3.80 (4H, m), 3.12 (9H, m), 2.70 (2H, d, J = 7.2 Hz), 1.85 (2H, m) and 0.92 (9H, s). ¹³C-NMR; δ (CD₃OD), 173.9, 173.5, 171.3, 135.0, 134.2, 129.6, 123.4, 88.5, 82.5, 71.5, 65.3, 63.2, 56.0, 53.3, 50.3, 36.9, 34.7, 27.2, 24.8 and 20.7.

### EXAMPLE 8

### N⁴-(2,2-Dimethyl-1S-methylcarbamoyl-propyl)-2S,N¹-dihydroxy-3-{3-[4-(2-methoxy-ethoxy)-phenyl]-prop-2-ynyl}-succinamide

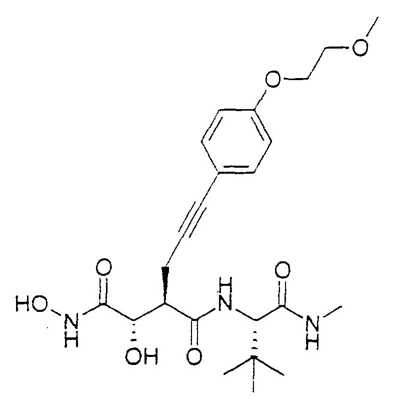

White solid. m.p. 185.4 - 185.6°C. ¹H-NMR; δ ((CD₃)₂SO), 10.65 (1H, s), 8.86 (1H, s), 7.89 (1H, m), 7.64 (1H, d, J = 9.5 Hz), 7.24 (2H, d, J = 8.6 Hz), 6.87 (2H, d, J = 8.7 Hz), 5.57 (1H, d, J = 7.6 Hz), 4.23 (1H, d, J = 10.5 Hz), 4.08 (2H, t, J = 4.3 Hz), 3.91 (1H, dd, J = 7.8 Hz), 3.63 (2H, t, J = 4.4 Hz), 3.29 (3H, s), 2.93 (1H, m), 2.40 (4H, m), 2.37 (1H, dd, J = 5.3, 16.6 Hz), and 0.89 (9H, s). ¹³C-NMR; δ ((CD₃)₂SO), 170.8, 170.4, 168.4, 158.1, 132.8, 115.1, 114.4, 85.9, 81.3, 70.5, 70.3, 67.0, 59.8, 58.1, 49,8, 48.6, 34.4, 26.6 and 25.2. IR: vₘₐₓ (KBr), 3319, 2954, 2883, 2367, 1637, 1566, 1508, 1367, 1249, 1120, 1061 and 832 cm⁻¹.

The starting material was prepared as follows:
To an ice-cooled solution of 4-bromophenoxyethanol (5 g, 23.0 mmol) in THF (30 ml) was added a suspension of sodium hydride (1.10 g, 46.1 mmol) in THF (30 ml), with stirring. After 20 minutes dimethyl sulfate (4.37 ml, 46.1 mmol) was added dropwise. The reaction mixture was stirred for 4 hours at room temperature then quenched slowly with saturated aq. ammonium chloride (50 ml). The mixture was extracted with ethyl acetate (100 ml) and the organic extract was washed successively with 1M hydrochloric acid (50 ml) and brine (50 ml), dried over anhydrous magnesium sulfate and filtered. The solvent was removed under reduced pressure to leave 4-(2-methoxy-ethoxy)-*bromobenzene* (5.5 g, ca. quant.) as a pale brown oil. ¹H-NMR; δ (CDCl₃), 7.36 (2H, d, J = 9.1 Hz), 6.80 (2H, d, J = 9.1 Hz), 4.09 (2H, t, J = 4.7 Hz), 3.74 (2H, t, J = 4.7 Hz) and 3.45 (3H, s).

### EXAMPLE 9

### 3R-[3-(4-Chloro-phenyl)-prop-2-ynyl]-2S,N¹-dihydroxy-N⁴-{1S-[2-(2-methoxy-ethoxy)-ethylcarbamoyl]-2,2-dimethyl-propyl}-succinamide

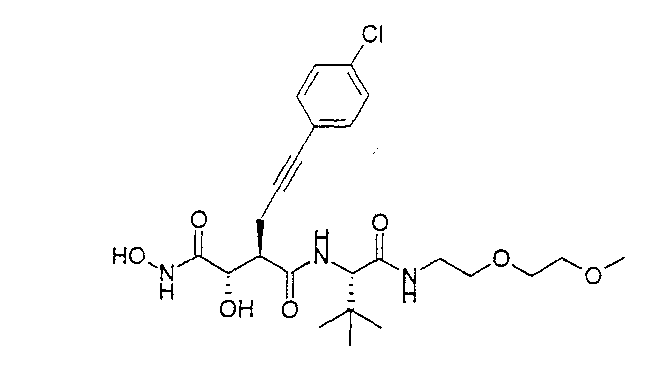

White solid. m.p. 69.2 - 70.5°C. ¹H-NMR; δ (CD₃OD), 8.25 (1H, m), 7.86 (1H, d, J = 8.7 Hz), 7.19 (4H, dd, J = 13.0, 8.7 Hz), 4.20 (1H, d, J = 5.5 Hz), 4.14 (1H, d, J = 8.8 Hz), 3.41 (8H, m), 3.25 (3H, s), 3.01 (1H, m), 2.73 (1H, dd, J = 9.5, 16.9 Hz), 2.59 (1H, dd, J = 4.6, 16.9 Hz) and 0.88 (9H, s). ¹³C-NMR; δ (CD₃OD), 176.4, 175.2, 173.3, 137.2, 136.5, 131.9, 126.1, 91.6, 84.3, 75.4, 74.6, 73.3, 72.8, 65.2, 61.5, 52.8, 42.7, 37.7, 29.7 and 21.0.

### EXAMPLE 10

### 3R-[3-(2-Chloro-phenyl)-prop-2-ynyl]-N⁴-(2,2-dimethyl-1S-methylcarbamoyl-propyl)-2S,N¹-dihydroxy-succinamide

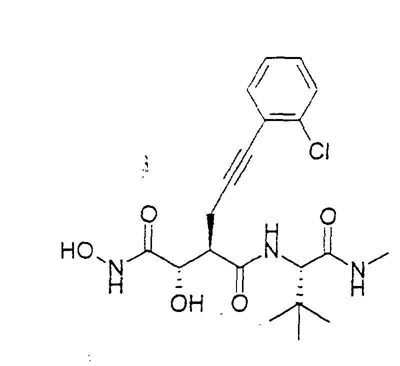

White solid. m.p. 179.3 - 180°C. ¹H-NMR; δ (CD₃OD), 8.03 (1H, m), 7.94 (1H, d, J = 3.2 Hz), 7.45 (2H, m), 7.27 (2H, m), 4.36 (1H, d, J = 5.8 Hz), 4.28 (1H, s), 3.15 (1H, m), 2.87 (1H, dd, J = 8.2, 16.7 Hz), 2.77 (1H, dd, J = 6.1, 16.7 Hz), 2.63 (3H, d, J = 8.6 Hz) and 1.01 (9H, s). ¹³C-NMR; δ (CD₃OD), 172.6, 172.0, 170.2, 135.7, 133.7, 129.3, 129.1, 126.7, 123.4, 11 0.0, 92.0, 79.2, 71.2, 61.3, 49.1, 34.6, 26.2 and 25.0.

### EXAMPLE 11

### 3R-[3-(4-Chloro-phenyl)-prop-2-ynyl]-N⁴-(1S-dimethylcarbamoyl-2,2-dimethyl-propyl)-2S,N¹-dihydroxy-succinamide

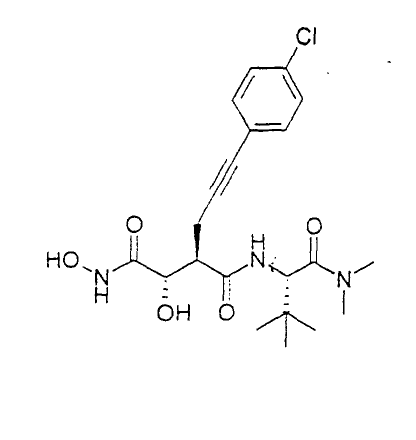

White solid. m.p. 104.5 - 105.5°C. ¹H-NMR; δ (CD₃OD), 7.35 (4H, dd, J = 11.4, 9.0 Hz), 4.96 (1H, s), 4.28 (1H, d, J = 6.1 Hz), 3.16 (3H, s), 3.10 (1H, m), 2.82 (3H, s), 2.74 (2H, t, J = 8.1 Hz) and 1.03 (9H, s). ¹³C-NMR; δ (CD₃OD), 172.8, 171.9, 170.2, 133.9, 133.2, 128.6, 122.5, 87.4, 81.3, 71.3, 65.9, 55.0, 49.2, 37.8, 35.6, 34.9 and 26.0.

### EXAMPLE 12

### N⁴-(1S-Benzyl-2-hydroxy-ethyl)-3R-[3-(4-chloro-phenyl)-prop-2-ynyl]-2S,N¹-dihydroxy-succinamide

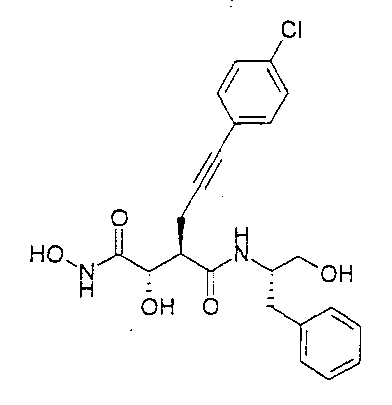

White solid. m.p. 137.2 - 137.4°C. ¹H-NMR; δ (CD₃OD), 7.16 (9H, m), 4.19 (1H, d, J = 5.4 Hz), 3.99 (1H, m), 3.39 (2H, m), 2.83 (1H, m), 2.75 (2H, t, J = 7.1 Hz) and 2.58 (2H, d, J = 7.7 Hz). ¹³C-NMR; δ (CD₃OD), 174.3, 171.8, 140.1, 135.3, 134.5, 130.9, 130.0, 129.8, 127.8, 124.0, 89.0, 82.7, 72.5, 63.9, 54.7, 50.6, 38.3 and 20.8. IR: vₘₐₓ (KBr), 3528, 3277, 3201, 2940, 1643, 1559, 1488, 1088 and 1022 cm⁻¹.

### Example 13

### 3R-[3-(4-Chloro-phenyl)-prop-2-ynyl]-N⁴-(2-cyclohexylmethyl-1S-hydroxymethyl-ethyl)-2S,N¹-dihydroxy-succinamide

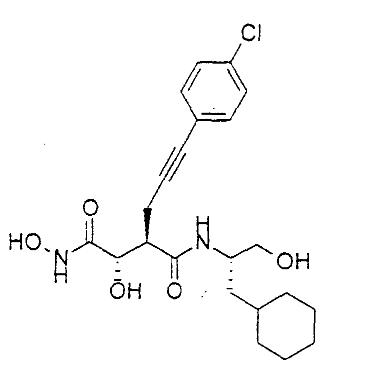

White solid. m.p. 163-163.5 °C. ¹H-NMR; δ ((CD₃)₂SO), 8.76 (1H, s), 7.68 (1H, d, J = 8.8 Hz), 7.42 (4H, s), 5.60 (1H, dd, J = 6.3 Hz), 4.51 (1H, dd, J = 4.3 Hz), 4.06 (1H, dd, J = 6.4 Hz), 3.88 (1H, m), 3.34 (3H, m), 3.26 (1H, m), 2.85 (1H, m), 2.66 (1H, m), 1.74 (1H, m) and 1.51 - 0.66 (12H, m). ¹³C-NMR; δ ((CD₃)₂SO), 171.2, 168.6, 133.4, 132.9, 128.9, 122.6, 90.4, 80.5, 70.9, 64.5, 48.8, 48.2, 34.0, 33.5, 32.2, 26.4, 25.8 and 18.3.

### EXAMPLE 14

### 3R-[3-(4-Chloro-phenyl)-prop-2-ynyl]-2S,N¹-dihydroxy-N⁴-[1S-hydroxymethyl-2-(3H-imidazol-4-yl)-ethyl]-succinamide

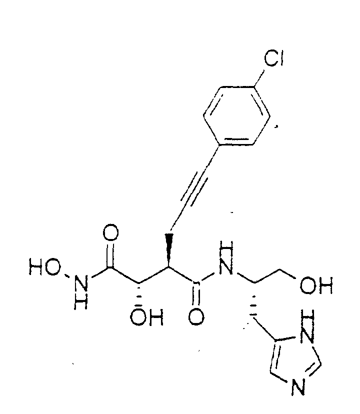

White solid. m.p. 85-87 °C. ¹H-NMR; δ (CD₃OD, mixture of diastereoisomers), 7.45 (1H, d, J = 2.6 Hz), 7.17 (4H, m), 6.78 (1H, d, J = 9.2 Hz), 4.18 (1H, d, J = 5.9 Hz), 4.03 (1H, m), 3.39 (2H, m) and 2.71 (5H, m). ¹³C-NMR; δ (CD₃OD), 174.6, 174.5, 171.8, 171.5, 135.7, 135.6, 135.4, 135.2, 134.5, 134.4, 134.3, 133.2, 130.0, 124.0, 123.9, 119.2,118.5, 89.8, 88.9, 82.7, 82.2, 72.7, 72.6, 64.4, 64.3, 52.8, 52.4, 51.5, 50.4, 28.8, 20.7 and 19.4. IR: vₘₐₓ (neat), 3220, 2236, 1647, 1543, 1486, 1088, 826 and 623 cm⁻¹.

### Example 15

### N⁴-[2,2-Dimethyl-1S-(pyridin-2-ylcarbamoyl)-propyl]-2S,N¹-dihydroxy-3R-(3-thiophen-2-ylprop-2-ynyl)-succinamide

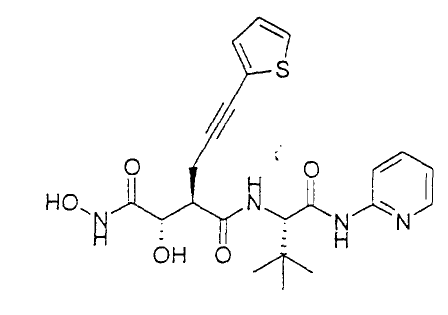

Pale yellow solid. ¹H-NMR; δ ((CD₃)₂SO), 10.52 (1H, s), 8.89 (1H, d, J = 1.4 Hz)., 8.32 (1H, dd, J = 1.0, 4.8 Hz), 8.06 (1H, d, J = 8.4 Hz), 7.87 (1H, d, J = 9.4 Hz), 7.78 (1H, dd, J = 1.8, 9.0 Hz), 7.33 (1H, dd, J=1.1, 5.2 Hz), 7.12 (1H, dd, J = 4.9, 6.5 Hz), 7.01 (1H, dd, J = 0.7, 3.4 Hz), 6.78 (1H, dd, J = 3.6, 5.1 Hz), 5.57 (1H, d, J = 7.7 Hz), 4.71 (1H, d, J = 9.4 Hz), 3.96 (1H, d, J = 7.7 Hz), 3.07 (1H, m), 2.63 (1H, dd, J = 9.4, 16.9 Hz), 2.51 (3H, m) and 1.00 (9H, s). ¹³C-NMR; δ ((CD₃)₂SO), 171.2, 170.5, 168.7, 152.0, 148.3, 138.3, 132.0, 127.4, 127.3, 123.0, 119.9, 114.3, 92.0, 75.0, 71.0, 60.5, 48.5, 35.0, 26.9 and 19.5. IR: vₘₐₓ (neat), 3293, 2965, 1651, 1538, 1470, 1435, 1301, 1210, 1080, 847, 782 and 702 cm⁻¹.

### Example 16

### N⁴-(1S-Benzyl-2R(or S)-hydroxy-propyl)-3R-[3-(4-chlore-phenyl)-prop-2-ynyl]-2,N¹-dihydroxy-succinamide

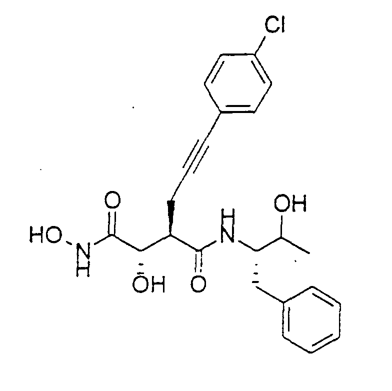

Diastereoisomer A: White solid m.p. 184.5-185 °C. ¹H-NMR; δ (CD₃OD), 7.34 (9H, m), 4.46 (1H, d, J = 5.6 Hz), 4.30 (1H, m), 4.00 (1H, m), 3.18 (1H, dd, J = 14.1, 4.6 Hz), 3.12 (1H, m), 2.92 (2H, m), 2.77 (1H, dd, J = 16.9, 5.1 Hz) amd 1.43 (3H, d, J = 6.5 Hz). ¹³C-NMR; δ (CD₃OD), 174.3, 171.4, 140.4, 135.1, 134.6, 130.6, 129.9, 129.7, 127.6, 124.2, 89.9, 82.0, 72.7, 70.4, 58.0, 50.6, 37.2, 19.9 and 18.8.
Diastereoisomer B: White solid. m.p. 175-175.5 °C. ¹H-NMR; δ (CD₃OD), 7.05 (9H, m), 4.02 (1H, d, J = 5.6 Hz), 3.80 (1H, m), 3.47 (1H, m), 2.80 (1H, dd, J = 14.0, 4.6 Hz), 2.67 (1H, dd, J = 13.6, 7.1 Hz), 2.55 (1H, dd, J = 14.0, 8.8 Hz), 2.35 (2H, d, J = 6.3 Hz) and 0.96 (3H, d, J = 7.2 Hz). ¹³C-NMR; δ (CD₃OD), 174.4, 171.7, 140.3, 135.3, 134.5, 131.0, 130.0, 129.7, 127.6, 123.9, 89.0, 82.7, 72.1, 70.3, 58.4, 50.7, 37.4, 20.9 and 20.6.

The following compounds were prepared by an Ugi 4-component condensation reaction of 2R-(2,2-dimethyl-5-oxo-[1,3]dioxolan-4S-yl)-5-(4-chlorophenyl)-pent-4-ynoic acid with ammonia, trimethylacetaldehyde and the appropriate isocyanide, followed by ring opening with hydroxylamine, as described previously (see WO 96/26918):

### Example 17

### N⁴-(1-Benzylcarbamoyl-2,2-dimethylpropyl)-3R-[3-(4-chloro-phenyl)-prop-2-ynyl]-2,N¹-dihydroxy-succinamide

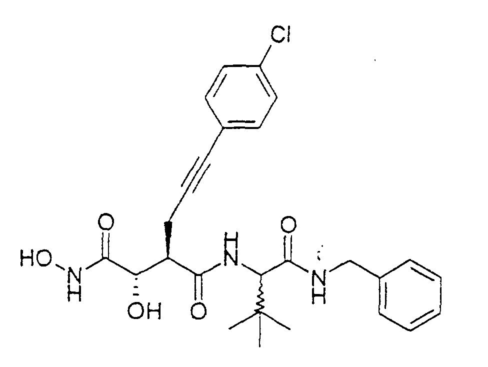

Yellow oil. ¹H-NMR; δ (CD₃OD, 1:1 mixture of diastereoisomers), 7.31 - 7.06 (9H, m), 4.37 - 4.10 (4H, m), 3.73 - 3.48 (2H, m), 3.10 - 2.89 (2H, m), 2.70 - 2.55 (1H, m), 0.86 (4.5H, s) and 0.85 (4.5H, s).

### Example 18

### N⁴-(1-tert-Butylcarbamoyl-2,2-dimethylpropyl)-3R-[3-(4-chloro-phenyl)-prop-2-ynyl)-2,N¹-dihydroxy-succinamide

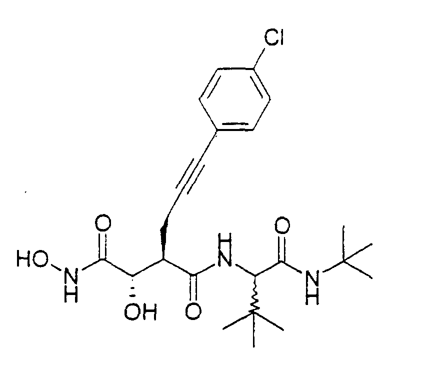

Yellow oil. ¹H-NMR; δ (CD₃OD, 1:1 mixture of diastereoisomers), 7.32 - 7.16 (4H, m), 4.22 - 4.06 (2H, m), 3.68 - 3.57 (2H, m), 2.96 (1H, m), 2.68 - 2.55 (2H, m), 1.23 (4.5H, s), 1.20 (4.5H, s), 1.15 (3H, s), 1.11 (3H, s) and 0.86 (9H, s).

### Example 19

### 3R-(3-Biphenyl-4-yl-prop-2-ynyl)-N⁴-(1S(or R)-butylcarbamoyl-2,2-dimethyl-propyl)-2,N¹-dihydroxy-succinamide

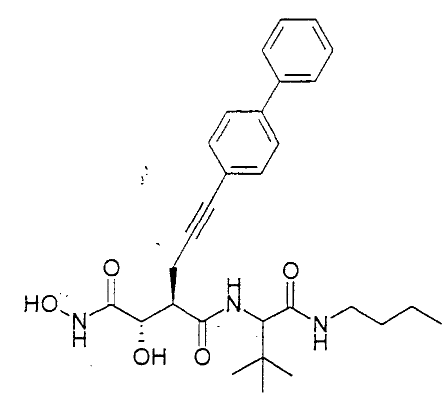

Diastereoisomers separated by preparative reverse phase HPLC. Diastereoisomer A: ¹H-NMR; δ (CD₃OD), 7.87 (1H, d, J = 8.5 Hz), 7.57 - 7.18 (11H, m), 4.22 - 4.14 (2H, m), 3.16 - 2.53 (5H, m), 1.35 - 1.10 (4H, m), 0.90 (9H, s) and 0.72 (3H, t, J = 7.1 Hz). Diastereoisomer B: ¹H-NMR; δ (CD₃OD), 7.82 (1H, d, J = 9.1 Hz), 7.55 - 7.16 (11H, m), 4.16 - 4.12 (2H, m), 3.18 - 2.96 (3H, m), 2.77 - 2.54 (2H, m), 1.48 - 1.19 (4H, m), 0.90 (9H, s) and 0.81 (3H, t, J = 7.2 Hz).

### EXAMPLE 20

### 3R(or S)-(3-Biphenyl-4-yl-prop-2-ynyl)-N⁴-[2,2-dimethyl-1S-(pyridin-2-ylcarbamoyl)-propyl]-N¹-hydroxy-succinamide

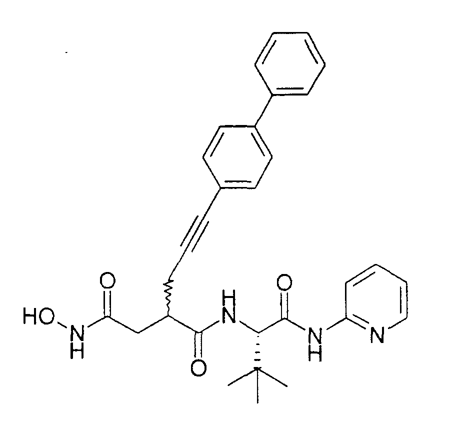

The title compound was prepared according to the route outlined in Scheme 3 and is described in detail below.

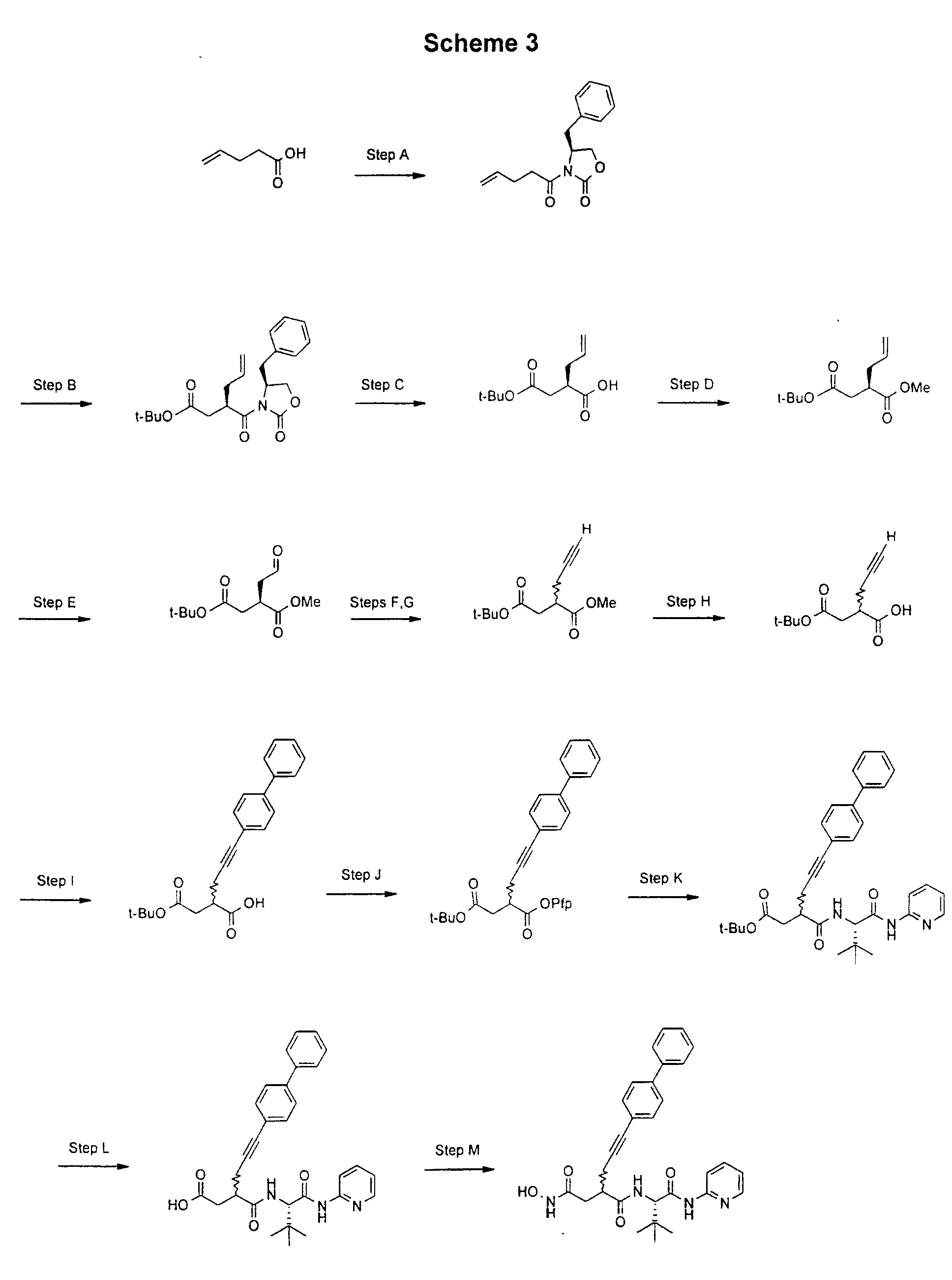

### STEP A:

### 4-Benzyl-3-pent-4-enyl-oxazolid in-2-one

To a solution of 4-pentynoic acid (100 g, 1 mol) in dichloromethane (500 ml) at 0°C, was added oxalyl chloride (140 g, 1.1 mol) and the resulting solution was stirred at 0°C for 3 hours. Dimethylformamide (0.2 ml) was then added and the solution was stirred at room temperature overnight. Solvents were then removed under reduced pressure and the residue was dissolved in dry THF (200 ml).

A solution of (S)-4-benzyl-oxazolidin-2-one (135 g, 0.76 mol) in THF (1.5 L) was cooled to -70°C and treated dropwise with a solution of n-butyl lithium in hexanes (1.6 M, 503 ml, 0.79 mol). The reaction mixture was stirred for a further 20 minutes before slow addition of the acid chloride, prepared above, while keeping the temperature below -60°C. The reaction was then allowed to warm to ambient temperature and quenched with 1M sodium carbonate . THF was removed *in vacuo* and the aqueous phase was extracted with ethyl acetate (200 ml). The organic layer was washed with 1M sodium carbonate (2 x 400 ml) and brine (200 ml), dried over anhydrous magnesium sulphate and concentrated *in vacuo* to provide 4-benzyl-3-pent-4-enyl-oxazolidin-2-one as a yellow oil (194 g, 0.75 mol, 98.4 %). ¹H-NMR was consistent with the desired product.

### STEP B:

### 3-(4-Benzyl-2-oxo-oxazolidin-3-carbonyl)hex-5-enoic acid tert-butyl ester

A solution of 4-benzyl-3-pent-4-enyl-oxazolidin-2-one (194 g, 0.748 mol) in THF (2 L) was cooled to -78°C and treated dropwise with a 1M solution of sodium hexamethyldimethylsilazide (822 ml, 0.822 mol). The reaction was then stirred for a further 20 minutes and a solution of *tert-*butyl-2-bromoacetate (153 g, 0.785 mol) in THF (400 ml) added whilst maintaining the temperature below -60°C. The resulting solution was stirred for a further 20 minutes at -70°C then allowed to warm to -50°C. Reaction was quenched with 20% aqueous ammonium chloride solution (200 ml) and allowed to warm to ambient temperature. THF was removed *in vacuo* and ethyl acetate (400 ml) was added. The organic phase was washed with water (2 x 300 ml) and brine (200 ml), dried over anhydrous magnesium sulphate, filtered and concentrated *in vacuo* to provide 3-(4-benzyl-2-oxo-oxazolidin-3-carbonyl)-hex-5-enoic acid *tert*-butyl ester as a pale yellow solid (279 g, 0.748 mol, 100%). ¹H-NMR was consistent with the desired product.

### STEP C:

### 2-Allyl-succinic acid 4-tert-butyl ester

A solution of 3-(4-benzyl-2-oxo-oxazolidin-3-carbonyl)hex-5-enoic acid *tert*-butyl ester (200 g, 0.54 mol) in THF (1.5 L) was cooled to 0°C and treated with a solution of lithium hydroxide (27 g, 0.64 mol) in water (375 ml) and 60%aqueous hydrogen peroxide (121 ml, 2.14 mol). The reaction mixture was allowed to warm to ambient temperature and stirred overnight. THF was removed *in vacuo* and the aqueous phase was washed with ethyl acetate (3 x 100 ml) then acidified with concentrated hydrochloric acid (60 ml). Product was extracted with ethyl acetate (3 x 150 ml) and the combined organic phases were washed with water (150 ml), dried over anhydrous magnesium sulphate, filtered and concentrated *in vacuo* to provide 2-allyl-succinic acid 4-*tert*-butyl ester as a yellow oil (83 g, 0.387 mol, 72%). ¹H-NMR ; δ (CDCl₃), 5.76 (2H, m), 5.06 (2H, m), 2.90 (1H, m), 2.62 (1H, dd), 2.46 (3H, m) and 1.44 (9H, s).

### STEP D:

### 2-Allyl-succinic acid 4-tert-butyl ester 1-methyl ester

A solution of 2-allyl-succinic acid 4-*tert*-butyl ester (80.3 g, 0.375 mol) in acetone (800 ml) was treated with iodomethane (25.7 ml, 0.412 mol) and potassium carbonate (129.5 g, 0.938 mol) then allowed to stir at ambient temperature for 48 hours. Ethyl acetate (300 ml) and water (300 ml) were added and the organic layer was separated, washed with brine (200 ml), dried over anhydrous magnesium sulphate, filtered and evaporated to dryness to provide 2-Allyl-succinic acid 4-*tert*-butyl ester 1-methyl ester as an orange oil (71.8 g, 0.31 mol, 84%). ¹H-NMR was consistent with the desired product.

### STEP E:

### 2-(2-Oxo-ethyl)-succinic acid 4-tert-butyl ester 1-methyl ester

A solution of 2-allyl-succinic acid 4-*tert*-butyl ester 1-methyl ester (71.8 g, 0.315 mol) in methanol (720 ml) was cooled to -78°C and a stream of ozone bubbled through the solution until a blue colour persisted (3.5 hours). The solution was then flushed with argon and treated with dimethyl sulfide (27.4 g, 0.441 mol). The reaction was allowed to warm to ambient temperature and stirred over night. Solvents were evaporated and the residue was dissolved in ethyl acetate (150 ml), washed with 1M sodium carbonate (2 x 100 ml) and brine (100 ml), dried over anhydrous magnesium sulphate, filtered and evaporated to dryness to provide crude product an a brown oil. The residue was distilled under reduced pressure, collecting the fraction at 108 °C, to provide 2-(2-oxoethyl)-succinic acid 4-*tert*-butyl ester 1-methyl ester as a colourless oil (52 g, 0.226 mol, 72%). ¹H-NMR ; δ (CDCl₃), 9.72 (1H, s), 3.71 (3H, s), 3.27 (1H, m), 2.95 (1H, dd, J = 17.5, 7.5 Hz), 2.66 (3H, m) and 1.44 (9H, s).

### STEP F:

### Dimethyl-1-diazo-2-oxopropylphosphonate

A slurry of tosyl chloride (44.4 g, 0.23 mol) in ethyl alcohol (60 ml) was added to a solution of sodium azide (50 g, 0.77 mol) in water (280 ml) and ethyl alcohol (660 ml) at -10°C and the reaction mixture was allowed to stir at -10°C for 2 hours then at ambient temperature for 1 hour. The reaction was quenched by the addition of water (2 L) and the products were extracted into dichloromethane (3 x 100 ml). The combined organic extracts were dried over anhydrous magnesium sulphate, filtered and concentrated *in vacuo* to provide tosyl azide as a colourless oil (41.8 g, 0.21 mol, 92%). ¹H-NMR ; δ (CDCl₃), 7.85 (2H, d, J = 7.5 Hz), 7.41 (2H, d, J = 7.5 Hz) and 2.49 (3H, s). 60% Sodium hydride (7.1 g, 0.18 mol) was washed with hexane then suspended in toluene (100 ml) and THF (50 ml) and cooled to 0°C. A solution of dimethyl-2-oxopropylphosphonate (28 g, 0.17 mol) in toluene (250 ml) and THF (100 ml) was added dropwise. After stirring at 0°C for 1 hour, a solution of tosyl azide (35 g, 0.18 mol) was added and the reaction was stirred at ambient temperature overnight. The reaction mixture was then filtered through Kieselghur, washing the solid precipitate with toluene (100 ml). The filtrate was concentrated *in vacuo* to provide dimethyl-1-diazo-2-oxopropylphosphonate as a colourless oil (26 g, 0.135 mol, 80%). ¹H-NMR was consistent with the desired product.

### STEP G:

### 2-Prop-2-ynyl-succinic acid 4-tert-butyl ester 1-methyl ester

To a stirred solution of 2-(2-oxo-ethyl)-succinic acid 4-*tert*-butyl ester 1-methyl ester (28.8 g, 0.125 mol) and anhydrous potassium carbonate 34.6 g, 0.25 mol) in methanol (600 ml) was added dimethyl-1-diazo-2-oxopropylphosphonate (28.8 g, 0.15 mol). Stirring was continued for 2 hours. The reaction mixture was concentrated to small volume under reduced pressure and diluted with water and ethyl acetate. The organic layer was separated, washed with water, dried over anhydrous magnesium sulphate, filtered and concentrated *in* vacuo to provide product as a yellow oil. Purification by column chromatography (silica gel, 0 - 5% methanol in dichloromethane) provided the title compound as a colourless oil (16.35 g, 72.2 mmol, 58%). ¹H-NMR ; δ (CDCl₃), 3.72 (3H, s), 3.02 (1H, m), 2.73 (1H, d, J = 7.9 Hz), 2.68 (1H, d. J = 7.9 Hz), 2.60 (2H, dd, J = 2.5, 6.0 Hz), 2.04 (1H, t, J = 2.6 Hz) and 1.45 (9H, s).

### STEP H:

### 2-Prop-2-ynyl-succinic acid 4-tert-butyl ester

2-Prop-2-ynyl-succinic acid 4-*tert*-butyl ester 1-methyl ester (14.42 g, 63.7 mmol) in methanol (120 ml) was treated with a solution of lithium hydroxide (4.01 g, 95.6 mmol) in water (25 ml) and stirred at ambient temperature for 5 hours. Solvents were removed under reduced pressure and the residue was partitioned between 2M hydrochloric acid and ethyl acetate. The organic layer was separated, washed with water, dried over anhydrous magnesium sulphate, filtered and concentrated *in vacuo* to provide product as a viscous, colourless oil (12.59 g, 59.3 mmol, 93%). ¹H-NMR ; δ (CDCl₃), 3.02 (1H, m), 2.73 (1H, d, J = 7.9 Hz), 2.68 (1H, d. J = 7.9 Hz), 2.60 (2H, dd, J = 2.5, 6.0 Hz), 2.04 (1H, t, J = 2.6 Hz) and 1.45 (9H, s).

### STEP I:

### 2-(3-Biphenyl-4-yl-prop-2-ynyl)-succinic acid 4-tert-butyl ester

2-Prop-2-ynyl-succinic acid 4-*tert*-butyl ester (5.3 g, 25 mmol) and 4-bromobiphenyl (4.85 g, 20.83 mmol) were dissolved in dichloromethane (25 ml). Dichlorobis-(triphenylphosphine)palladium(II) (670 mg, 4.6 mol%) and copper (I) iodide (158 mg, 4.0 mol%) were added and diisopropylamine (8.2 ml, 62.5 mmol) added slowly. The solution was stirred at ambient temperature for 6 hours and filtered through celite, washing with dichloromethane (100 ml). The filtrate was washed with 1M hydrochloric acid (2 x 25 ml) then brine (25 ml), dried over magnesium sulphate, filtered and solvents removed *in vacuo.* The residue was purified by column chromatography (Biotage, 50% ethyl acetate in hexanes) to provide the title compound as a pale brown solid (2.96 g, 8.15 mmol, 39%). ¹H-NMR ; δ (CDCl₃), 7.50 (9H, m), 3.10 (1H, m), 2.74 (4H, m and 1.45 (9H, s).

### STEP J:

### 2-(3-Biphenyl-4-yl-prop-2-ynyl)-succinic acid 4-tert-butyl ester 1-pentafluorophenyl ester

A solution of 2-(3-biphenyl-4-yl-prop-2-ynyl)-succinic acid 4-*tert*-butyl ester (2.96 g, 8.15 mmol) and pentafluorophenol (1.72 g, 9.37 mmol) in dichloromethane (60 ml) was cooled to 0°C and treated with EDC (1.72 g, 8.96 mmol). The resulting solution was allowed to stir at ambient temperature for 5 hours then washed with 1 M sodium carbonate (2 x 30 ml), 1 M hydrochloric acid (2 x 30 ml) and brine (30 ml), dried over anhydrous magnesium sulphate, filtered and concentrated *in vacuo* to provide product as a brown solid (4.02 g, 7.6 mmol, 93%). ¹H-NMR was consistent with the desired product.

### STEP K:

### 6-Biphenyl-4-yl-3-[2,2-dimethyl-1-(pyridin-2-ylcarbamoyl)-propylcarbamoyl]-hex-5-ynoic acid tert-butyl ester

A solution of 2-(3-biphenyl-4-yl-prop-2-ynyl)-succinic acid 4-*tert*-butyl ester 1-pentafluorophelyl ester (4.0 g, 7.6 mmol) and L-*tert*-leucine-N-2-pyridyl amide (1.43 g, 6.9 mmol) in ethyl acetate (70 ml) was heated at reflux for 2 hours. The solution was cooled to room temperature, washed with 1M hydrochloric acid (2 x 30 ml), 1M sodium carbonate (2 x 30 ml) and brine (30 ml), dried over anhydrous magnesium sulphate, filtered and concentrated *in vacuo* to provide product as a brown solid. Purification by column chromarography (Biotage, 50% ethyl acetate in hexanes) provided the title compound as a pale brown solid (1:1 mixture of diastereomers, 2.7 g, 4.89 mmol, 71%). ¹H-NMR ; δ (CDCl₃), 8.98 (1H, s), 8.34 (1H, m), 8.20 (0.5H, d, J = 8.4 Hz), 8.10 (0.5 H, d, J = 8.4 Hz), 7.64 (1H, m), 7.40 (9H, m), 7.02 (1H, m), 6.92 (0.5 h, d, J = 9.1 Hz), 6.85 (0.5H, d, J = 9.1 Hz), 4.57 (1H, m), 3.01 (1H, m), 2.72 (4H, m), 1.45 (4.5H, s), 1.37 (4.5H, s) and 1.04 (9H, s).

### STEP L:

### 6-Biphenyl-4-yl-3-[2,2-dimethyl-1-(pyridin-2-ylcarbamoyl)-propylcarbamoyl]-hex-5-ynoic acid

A solution of 6-biphenyl-4-yl-3-[2,2-dimethyl-1-(pyridin-2-ylcarbamoyl)-propylcarbamoyl]-hex-5-ynoic acid *tert*-butyl ester (2.7 g, 4.89 mmol) in dichloromethane (10 ml) and TFA (10 ml) was allowed to stand at 4°C over night. Solvents were removed *in vacuo*, and the residue was azeotroped with toluene to remove residual TFA acid. Product was purified by column chromatography (Biotage, 5% methanol in dichloromethane) to provide the title compound as a yellow solid (1.22 g, 2 mmol, 40%). ¹H-NMR was consistent with the desired product.

### STEP M:

### 2-(3-Biphenyl-4-yl-prop-2-ynyl)-N 1-[2,2-dimethyl-1-(pyridin-2-ylcarbamoyl)-propyl]-N 4-hydroxy-succinamide

A solution of 6-biphenyl-4-yl-3-[2,2-dimethyl-1-(pyridin-2-ylcarbamoyl)-propylcarbamoyl]-hex-5-ynoic acid in dimethyl formamide (20 ml) was treated with HOBT (297 mg, 2.2 mmol), EDC (460 mg, 2.4 mmol), hydroxylamine hydrochloride (208 mg, 3.0 mmol) and N-methyl morpholine (0.44 ml, 4.0 mmol) and the reaction mixture allowed to stir at ambient temperature over night. Solvents were removed under reduced pressure and the residue was washed with diethyl ether and water. Product was purified by column chromatography (acid-washed silica gel, 5-10% gradient elution, methanol in dichloromethane) to provide the title compound as a pale yellow solid (292 mg, 0.57 mmol, 28%). ¹H-NMR; δ (CD₃OD, 1:1 mixture of diastereoisomers), 8.41 (1H, m), 8.23 (1H, m), 8.09 (1H, m), 7.64 (6H, m), 7.43 (4H, m), 4.45 (1H, m), 3.03 (1H, m), 2.69 (2H, m) and 1.14 (9H, 2s)

### Example 24

### 6-(4-Chlorophenyl)-3R-(2,2-dimethyl-1S-methylcarbamoyl-propylcarbamoyl)-2R(or S)-hydroxy-hex-5-ynoic acid

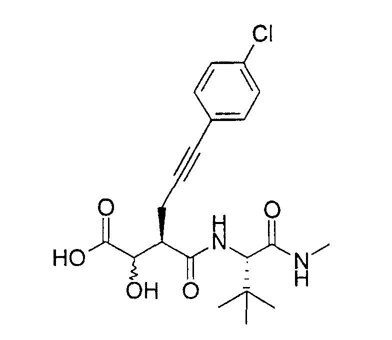

2R-(2,2-dimethyl-5-oxo-[1,3]dioxalan-4S-yl)-5-(4-chloro-phenyl)-pent-4-ynoic acid (2,2-dimethyl-1S-methylcarbamoyl-propyl)-amide (prepared by analogy with Example 2, Step E) (350 mg, 0.82 mmol) was dissolved in methanol (10ml) and water (5ml). 1M sulfuric acid was added and the mixture was stirred at room temperarure overnight. The solvents were removed under reduced pressure. The residue was dissolved in dichloromethane, washed successively with water and brine, dried over anhydrous magnesium sulphate, filtered and evaporated to dryness. The product was obtained as a white solid after chromatography (acid-washed silica, 10% methanol in dichloromethane). ¹H-NMR; δ (CD₃OD), 7.23 (2H, d, J = 8.7 Hz), 7.16 (2H, d, J = 8.7 Hz), 4.18 (1H, d, J = 4.3 Hz), 4.09 (1H, s), 2.98 (1H, m), 2.74 (2H, d, J = 7.9 Hz), 2.53 (3H, s) and 0.90 (9H, s). ¹³C-NMR; δ (CD₃OD), 182.1, 177.0, 175.7, 137.2, 136.5, 131.9, 126.1, 91.7, 84.6, 75.8, 65.1, 53.1,37.6, 29.8, 28.5 and 23.5.

## Claims

1. A compound of formula (I) 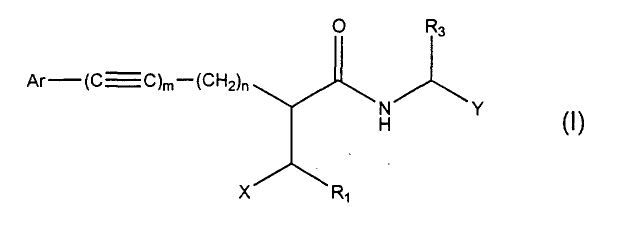 wherein
Ar represents an optionally substituted phenyl or heteroaryl group;
m is 1;
n is 0, 1, 2, 3 or 4;
X represents -COOH or -CONHOH;
R₁ represents hydrogen, (C₁-C₆)alkyl; (C₃-C₈)cycloalkyl; (C₂-C₆)alkenyl; phenyl; substituted phenyl; phenyl (C₁-C₆)alkyl; substituted phenyl(C₁-C₆)alkyl; heterocyclyl; substituted heterocyclyl; heterocyclyl(C₁-C₆)alkyl; substituted heterocyclyl(C₁-C₆)alkyl; amino; protected amino; acylamino; OH; SH; (C₁-C₆)alkoxy; (C₁-C₆)alkylamino; di-(C₁-C₆)alkylamino; (C₁-C₆)alkylthio; amino(C₁-C₆)alkyl; hydroxy(C₁-C₆)alkyl, mercapto(C₁-C₆)alkyl or carboxy(C₁-C₆)alkyl wherein the amino-, hydroxy-, mercapto- or carboxyl-group are optionally protected or the carboxyl- group amidated; or a group B'SOₚA'- wherein p is 0, 1 or 2 and B' is hydrogen or a (C₁-C₆) alkyl, phenyl, substituted phenyl, heterocyclyl, (C₁-C₆)acyl, phenacyl or substituted phenacyl group, and A' represents (C₁-C₆)alkyl;
R₃ represents the characterising group of a natural or non-natural α amino acid in which any functional groups may be protected;
Y is a group of formula (ID) or (IE) 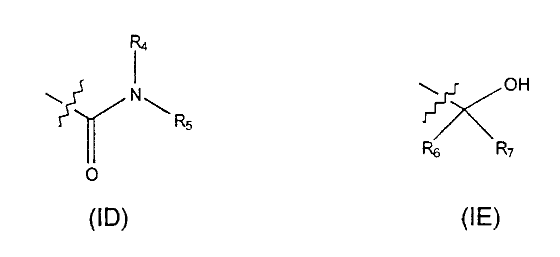
wherein:
R₄ represents
(a) an optionally substituted cycloalkyl or cycloalkenyl ring; or
(b) a phenyl or heteroaryl ring which may be fused to a benzene or heteroaryl ring, either or both of which rings may be substituted, and in which any ring nitrogen atom may be oxidised as an N-oxide, or
(c) a group -CHR^{x}R^{y} wherein R^{x} and R^{y} each independently represents an optionally substituted phenyl or heteroaryl ring which may be linked covalently to each other by a bond or by a C₁-C₄ alkylene or C₂-C₄ alkenylene bridge;
(d) a group of formula -(Z'-O)_{w}-Z wherein Z' is straight or branched C₁-C₆ alkyl optionally interrupted by one or more non-adjacent S and/or N atoms, w is an integer >1, and no continuous linear sequence of atoms in the group R₄ is >12, or
(e) a straight or branched C₁-C₆ alkyl group, optionally interrupted by one or more non-adjacent S and/or N atoms, which is substituted by at least two substituents of formula -(Z''')ₓ-(OZ)_{q} wherein Z"' is straight or branched C₁-C₆ alkyl optionally interrupted by one or more non-adjacent S and/or N atoms, x is 0 or 1, q is 1 or 2, and no continuous linear sequence of atoms in the group R₄ is >12, or
(f) hydrogen, C₁-C₆ alkyl, C₁-C₄ perfluoroalkyl, or a group D-(C₁-C₆ alkyl)- wherein D is hydroxy, C₁-C₆ alkoxy, C₁-C₆ alkylthio, acylamino. optionally substituted phenyl or heteroaryl, NH₂, or mono- or di-(C₁-C₆ alkyl)amino or N-morpholino;
or R₃ and R₄ taken together represent a divalent chain of formula -C(R^{a})(R^{b})-A"-Alk- wherein R^{a} and R^{b} are independently hydrogen or C₁-C₆ alkyl, A" is a bond, -O-, -S-,-S-S-, -NH- or -NR^{a}- wherein R^{a} is C₁-C₆ alkyl, and Alk is C₁-C₆ alkylene; and
R₅ is hydrogen or a C₁-C₆ alkyl group;
R₆ is hydrogen, C₁-C₆ alkyl, phenyl(C₁-C₆ alkyl) or heterocyclyl(C₁-C₆ alkyl);
R₇ is is hydrogen or a C₁-C₆ alkyl group;
or (when R₇ is hydrogen) R₃ and R₇ taken together with the carbon atoms to which they are attached form a 2-hydroxycyclohexyl or C₆ sugar (hexose) ring;
or R₆ and R₇ taken together with the carbon atom to which they are attached form a 5 or 6-membered carbocyclic or heterocyclic ring;
and salts hydrates and solvates thereof, PROVIDED THAT when m and n are both 1, and R₁ is methoxy, and R₃ is tert-butyl, and X is -CONHOH, and Y is a group of formula (ID) wherein R₄ is methyl and R₅ is hydrogen, then Ar is not 4-chlorophenyl or 4-phenylphenyl.

2. A compound as claimed in claim 1 wherein the stereochemistry is as follows:
C atom carrying the groups X and R₁ - S,
C atom carrying the triple unsaturated group - R,
C atom carrying the groups R₃ and Y - S.

3. A compound as claimed in claim 1 or claim 2 wherein n is 0 or 1.

4. A compound as claimed in claim 1 or claim 2 wherein Ar is a phenyl group which is substituted in the 4-position by a phenyl, phenoxy, phenylthio, 2-, 3- or 4-pyridyl, 2-, 3- or 4-pyridyloxy, 2-, 3- or 4-pyridylthio group which in turn is optionally substituted by C₁-C₆ alkyl, C₁-C₆ alkoxy(C₁-C₆ alkoxy), phenoxy, phenylthio, trifluoromethyl, halo, cyano (-CN), -CH₂CN, -OH, or -OR, wherein R is C₁-C₆ alkyl or benzyl.

5. A compound as claimed in claim 1 or claim 2 wherein Ar is a phenyl group which is optionally substituted by methyl, ethyl, n-propyl, n-butyl, n-pentyl or n-hexyl, 2-methoxyethoxy, phenoxy, phenylthio, trifluoromethyl, halo, cyano (-CN), -CH₂CN, -OH, or -OR, wherein R is C₁-C₆ alkyl or benzyl.

6. A compound as claimed in claim 1 or claim 2 wherein Ar is a phenyl group optionally substituted in the 4 position by trifluoromethyl, chloro, methoxy, cyano, or 2-methoxyethoxy.

7. A compound as claimed in claim 1 or claim 2 wherein Ar is a biphenyl group optionally substituted in the 4' position by trifluoromethyl, chloro, methoxy, cyano, or 2-methoxyethoxy.

8. A compound as claimed in any one of the preceding claims wherein R₁ is hydrogen, C₁-C₄ alkyl, cyclopentyl, hydroxy, methoxy, allyl, or a group -(CH₂)ₜ-W wherein t represents 1, 2, 3 or 4 and W represents phthalimido, 1,2-dimethyl-3,5-dioxo-1,2,4-triazolidin-4-yl, 3,4,4-trimethyl-2,5-dioxo-1-imidazolidinyl, 2-methyl-3,5-dioxo-1,2,4-oxadiazol-4-yl, 3-methyl-2,4,5-trioxo-1-imidazolidinyl, 2,5-dioxo-3-phenyl-1-imidazolidinyl-2-oxo-1-pyrrolidinyl, 2,5-dioxo-1-pyrrolidinyl, 2,6-dioxopiperidinylnaphthalimido (ie 1,3-dihydro-1,3-dioxo-2H-benz[f]isoindol-2-yl), 1,3-dihydro-1-oxo-2H-benz[f]isoindol-2-yl, 1,3-dihydro-1,3-dioxo-2H-pyrrolo[3,4-b]quinolin-2-yl, 2,3-dihydro-1,3-dioxo-1H-benz[d,e]isoquinolin-2-yl or saccharinyl.

9. A compound as claimed in any of the preceding claims wherein R₃ is methyl, benzyl, 4-chlorophenylmethyl, 2-thienylmethyl, iso-butyl or t-butyl, 1-benzylthio-1-methylethyl, 1-mercapto-1-methylethyl or 3H-imidazol-4-ylmethyl.

10. A compound as claimed in any of the preceding claims wherein Y is a group of formula (ID) and R₄ is
cyclopropyl, cyclobutyl, cyclopentyl, cyclohexyl,cycloheptyl or cyclooctyl;
phenyl, 2-methoxyphenyl, 3-methoxyphenyl, 4-methoxyphenyl, 3,4-dimethoxyphenyl, 2-fluorophenyl, 3-fluorophenyl, 4-fluorophenyl, 2-chlorophenyl, 3-chlorophenyl, 4-chlorophenyl, 3,4-dichlorophenyl, 3,5-dichlorophenyl, 2-bromophenyl, 3-bromophenyl, 4-bromophenyl, 2-iodophenyl, 3-iodophenyl, 4-iodophenyl, 2-methylphenyl, 3-methylphenyl, 4-methylphenyl, 3,4-dimethyl, 2-t-butylphenyl, 3-t-butylphenyl, 4-t-butylphenyl, 4-t-butyl-2,6-dimethylphenyl, 2-nitrophenyl, 3-nitrophenyl, 4-nitrophenyl, 2-cyanophenyl, 3-cyanophenyl, 4-cyanophenyl, 2-acetylphenyl, 3-acetylphenyl, 4-acetylphenyl, 2-methylsulphonylphenyl, 3-methylsulphonylphenyl, 4-methylsulphonylphenyl, 2-trifluoromethylphenyl, 3-trifluoromethylphenyl, 4-trifluoromethylphenyl, 3,5-ditrifluoromethylphenyl, 2-aminophenyl, 3-aminophenyl, 4-aminophenyl, 2-N,N-dimethylaminophenyl, 3-N,N-dimethylaminophenyl, 4-N,N-dimethylaminophenyl, 2-hydroxyphenyl, 3-hydroxyphenyl, 4-hydroxyphenyl, 2-napthyl, furan-2-yl, thien-2-yl, pyrrol-2-yl, tetrahydrofuran-2-yl, imidazol-2-yl, thiazol-2-yl, 4-ethoxycarbonylmethylthiazol-2-yl, 4-phenylthiazol-2-yl, 4,5-dimethylthiazol-2-yl, 5-bromothiazol-2-yl, 4-*tert*-butylthiazol-2-yl, benzothiazol-2-yl, 1,2,4-oxadiazol-5-yl, 3-methyl-1,2,4-oxadiazol-5-yl , 3-phenyl-1,2,4-oxadiazol-5-yl, 1,2,4-oxadiazol-3-yl, 1,3,4-oxadiazol-2-yl, 1,2,4-thiadiazol-5-yl, 3-phenyl-1,2,4-thiadiazol-5-yl, 1,3,4-thiadiazol-2-yl, 5-methyl-1,3,4-thiadiazol-2-yl, pyridin-2-yl, pyridin-3-yl, pyridin-4-yl, N-oxides of pyridin-2-yl pyridin-3-yl and pyridin-4-yl, piperazin-1-yl, indol-2-yl, benzimidazol-2-yl, benzotriazol-2-yl, pyrazin-2-yl, 1,2-pyridazin-3-yl, 1,3-pyrimidin-5-yl, 1,3-dithian-2-yl, benzo[b]thien-2-yl, isoxazol-5-yl, or quinolin-3-yl;
a group -CHR^{x}R^{y} wherein R^{x} and R^{y} independently represent optionally substituted phenyl, thienyl, furyl, pyrrolyl, imidazolyl, thiazolyl, pyrazolyl, isoxazolyl, isothiazolyl, triazolyl, thiadiazolyl, oxadiazolyl, pyridinyl, pyridazinolyl, pyrimidinyl, piperazinyl or triazinyl;
a polyether chain possessing at least two non-adjacent oxygen atoms, for example 2-(2-methoxyethoxymethoxy)ethyl, 1,1-dimethyl-2-(2-methoxyethoxymethoxy)ethyl, 2-(2-ethoxyethoxymethoxy)ethyl, 2-(2-(2-methoxyethoxy)ethoxy)ethyl, 2-(2-(3-methoxypropoxymethoxy)ethyl, 3-(2-methoxyethoxymethoxy)propyl, 2,2-dimethyl-3-(2-methoxyethoxymethoxy)propyl, 2-(2-methoxyethoxy)ethyl, 3-(2-methoxyethoxy)propyl, 2-methyl-2,2-di(2-methoxyethyl)propyl, 2-methyl-2,2-di(2-methoxyethyl)butyl, and 2-methyl-2,2-di(2-methoxymethyl)propyl;
methyl, ethyl, n- or iso-propyl, n-, sec- or tert-butyl, hydroxyethyl, hydroxypropyl, 2,2-dimethyl-3-hydroxypropyl, hydroxybutyl, methoxyethyl, ethoxyethyl, methoxypropyl, 2,2-dimethyl-3-methoxypropyl, 2,2-dimethyl-3-ethoxypropyl, 2-ethylthioethyl, 2-acetoxyethyl, N-acetyl-aminoethyl, 3-(2-pyrrolidone)propyl, morpholin-4-ylpropyl, optionally substituted phenylethyl, phenylpropyl, phenylbutyl, or phenylpentyl.

11. A compound as claimed in any of the preceding claims wherein Y is a group of formula (ID) and R₅ is hydrogen, methyl or ethyl.

12. A compound as claimed in any of the preceding claims wherein Y is a group of formula (ID), R₄ is hydrogen or methyl and R₅ is methyl.

13. A compound as claimed in any of claims 1 to 9 wherein Y is a group of formula (IE) and R₆ is hydrogen, methyl, ethyl, benzyl or pyridylmethyl

14. A compound as claimed in any of claims 1 to 9 wherein Y is a group of formula (IE) and R₇ is hydrogen or methyl.

15. A compound as claimed in any of claims 1 to 9 wherein Y is a group of formula (IE), and R₆ and R₇ are each hydrogen.

16. A compound as claimed in any of claims 1 to 9 wherein Y is a group of formula (IE) and R₆ and R₇ taken together with the carbon atom to which they are attached form a cyclopentyl, cyclohexyl or morpholino ring.

17. A compound as claimed in claim 1 or claim 2 wherein
Ar is phenyl or substituted phenyl;
n is 1;
X is -CONHOH,
R₁ is hydrogen, C₁-C₄alkyl, cyclopentyl, hydroxy, methoxy, allyl, or a group-(CH₂)ₜ-W wherein t represents 1, 2, 3 or 4 and W represents phthalimido, 1,2-dimethyl-3,5-dioxo-1,2,4-triazolidin-4-yl, 3,4,4-trimethyl-2,5-dioxo-1-imidazolidinyl, 2-methyl-3,5-dioxo-1,2,4-oxadiazol-4-yl, 3-methyl-2,4,5-trioxo-1-imidazolidinyl, 2,5-dioxo-3-phenyl-1-imidazolidinyl-2-oxo-1-pyrrolidinyl, 2,5-dioxo-1-pyrrolidinyl or 2,6-dioxo-piperidinylnaphthalimido (ie 1,3-dihydro-1,3-dioxo-2H-benz[f]isoindol-2-yl), 1,3-dihydro-1-oxo-2H-benz[f]isoindol-2-yl, 1,3-dihydro-1,3-dioxo-2H-pyrrolo[3,4-b] quinolin-2-yl, 2,3-dihydro-1,3-dioxo-1 H-benz[d,e]isoquinolin-2-yl or saccharinyl;
R₃ is t-butyl, 1-benzylthio-1-methylethyl, 1-mercapto-1-methylethyl, benzyl, methyl, or 3H-imidazol-4-ylmethyl;
Y is a group of formula (ID) wherein R₄ represents
cyclopropyl, cyclobutyl, cyclopentyl, cyclohexyl, cycloheptyl or cyclooctyl;
phenyl, 3-methoxyphenyl, pyridin-2-yl, pyridin-3-yl, thiazol-2-yl, 4-ethoxycarbonylmethylthiazol-2-yl, 5-methyl-1,3,4-thiadiazol-2-yl or 4-*tert*-butylthiazol-2-yl;
a group -CHR^{x}R^{y} wherein R^{x} and R^{y} independently represent phenyl or 4-chlorophenyl or R^{x} and R^{y} are linked covalently in a 9-H-fluoren-9-yl ring;
a polyether chain possessing at least two non-adjacent oxygen atoms, for example 2-(2-methoxyethoxy)ethyl; or
hydrogen, methyl or 3-morpholin-4-ylpropyl;
or R₃ and R₄ taken together represent -C(CH₃)₂SCH₂CH₂CH₂-, or -C(CH₃)₂SSCH₂CH₂-; and
R₅ represents hydrogen or methyl;
or Y is a group of formula (IE) wherein R₆ and R₇ are both hydrogen;

18. A compound as claimed in claim 17 wherein Ar is 4-phenyl-phenyl, 4-phenoxyphenyl, 4-(4'-chlorophenyl)-phenyl, 4-(4-cyanophenyl)-phenyl, 4-(4'-methoxy)-phenyl, 4-[4'-(2-methexyethoxy)phenyl]-phenyl, 4-(pyridin-4-yl)-phenyl, 4-(pyridin-4-yloxy)-phenyl, 4-(4' -bromophenyl)-phenyl, 4-trifluoromethyl-phenyl, 4-fluorophenyl, 4-methoxyphenyl, 4-methoxyethoxyphenyl, 4-propylphenyl, 4-methylphenyl or 4-chlorophenyl;

19. A compound as claimed in claim 1 selected from the group consisting of
3R-(3-biphenyl-4-yl-prop-2-ynyl)-N⁴-(2,2-dimethyl-1S-methylcarbamoyl-propyl)-2S,N¹-dihydroxy-succinamide;
N⁴-(2,2-dimethyl-1S-methylcarbamoyl-propyl)-2S,N¹-dihydroxy-3R-(3-phenyl-prop-2-ynyl)-succinamide;
N⁴-(2,2-dimethyl-1S-methylcarbamoyl-propyl)-2S,N¹-dihydroxy-3R-[3-(4-trifluoromethyl-phenyl)-prop-2-ynyl]-succinamide;
3R-[3-(4-chloro-phenyl)-prop-2-ynyl]-N⁴-(2,2-dimethyl-1S-methylcarbamoyl-propyl)-2S,N¹-dihydroxy-succinamide;
N⁴-(2,2-dimethyl-1S-methylcarbamoyl-propyl)-2S,N¹-dihydroxy-3R-[3-(4-methoxy-phenyl)-prop-2-ynyl]-succinamide,
3R-[3-(4'-chloro-biphenyl-4-yl)-prop-2-ynyl]-N⁴-(2,2-dimethyl-1S-methylcarbamoyl-propyl)-2S,N¹-dihydroxy-succinamide,
3R-[3-(4-cyanophenyl)-prop-2-ynyl]-N⁴-(2,2-dimethyl-1S-methylcarbamoyl-propyl)-2S,N¹-dihydroxy-succinamide,
3R-[3-(4-chloro-phenyl)-prop-2-ynyl]-N⁴-[2,2-dimethyl-1S-(3-morpholin-4-yl-propylcarbamoyl)-propyl]-2S,N¹-dihydroxy-succinamide,
N⁴-(2,2-dimethyl-1S-methylcarbamoyl-propyl)-2S,N¹-dihydroxy-3-{3-[4-(2-methoxy-ethoxy)-phenyl]-prop-2-ynyl}-succinamide,
3R-[3-(4-chloro-phenyl)-prop-2-ynyl]-2S,N¹-dihydroxy-N⁴-{1S-[2-(2-methoxy-ethoxy)-ethylcarbamoyl]-2,2-dimethyl-propyl}-succinamide,
3R-[3-(2-chloro-phenyl)-prop-2-ynyl]-N⁴-(2,2-dimethyl-1S-methylcarbamoyl-propyl)-2S,N¹-dihydroxy-succinamide,
3R-[3-(4-chloro-phenyl)-prop-2-ynyl]-N⁴-(1S-dimethylcarbamoyl-2,2-dimethyl-propyl)-2S,N¹-dihydroxy-succinamide,
N⁴-(1S-benzyl-2-hydroxy-ethyl)-3R-[3-(4-chloro-phenyl)-prop-2-ynyl]-2S,N¹-dihydroxy-succinamide,
3R-[3-(4-chloro-phenyl)-prop-2-ynyl]-N⁴-(2-cyclohexylmethyl-1S-hydroxymethylethyl)-2S,N¹-dihydroxy-succinamide,
3R-[3-(4-chloro-phenyl)-prop-2-ynyl]-2S,N¹-dihydroxy--N⁴-[1S-hydroxymethylethyl-2-(3H-imidazol-4-yl)-ethyl]succinamide,
N⁴-[2,2-dimethyl-1S-(pyridin-2-ylcarbamoyl)-propyl]-2S,N¹-dihydroxy-3R-(3-thiophen-2-ylprop-2-ynyl)-succinamide,
N⁴-(1S-benzyl-2R(orS)-hydroxy-propyl)-3R-[3-(4-chloro-phenyl)-prop-2-ynyl]-2,N¹-dihydroxy-succinamide,
N⁴-(1-tert-butylcarbamoyl-2,2-dimethylpropyl)-3R-[3-(4-chloro-phenyl)-prop-2-ynyl]-2,N¹-dihydroxy-succinamide,
3R-(3-biphenyl-4-yl-prop-2-ynyl)-N⁴-(1S(or R)-butylcarbamoyl-2,2-dimethylpropyl)-2,N¹-dihydroxy-succinamide,
3R(or S)-(3-biphenyl-4-yl-prop-2-ynyl)-N⁴-[2,2-dimethyl-1S-(pyridin-2-ylcarbamoyl)-propyl]-N¹-hydroxy-succinamide,
6-(4-chlorophenyl)-3R-(2,2-dimethyl-1S-methylylcarbamoyl-propylcarbamoyl)-2R(orS)-hydroxy-hex-5-ynoic acid;
and salts, hydrates and solvates thereof.

20. A compound as claimed in claim 1 selected from the group consisting of
N⁴-(1S-dimethylcarbamoyl-2,2-dimethyl-propyl)-2S,N¹-dihydroxy-3R-[3-(4-methoxy-phenyl)-prop-2-ynyl]-succinamide,
3R-(3-biphenyl-4-yl-prop-2-ynyl)-N⁴-(1S-dimethylcarbamoyl-2,2-dimethyl-propyl)-2S,N¹-dihydroxy-succinamide,
3R-[3-(4'-chloro-biphenyl-4-yl)-prop-2-ynyl]-N⁴-(1S-dimethylcarbamoyl-2,2-dimethyl-propyl)-2S,N¹-dihydroxy-succinamide,
3R-[3-(4-chloro-phenyl)-prop-2-ynyl]-2S,N¹-dihydroxy-N⁴-(1S-hydroxymethyl-2,2-dimethyl-propyl)-succinamide,
3R-[3-(4-chloro-phenyl)-prop-2-ynyl]-2S,N¹-dihydroxy-N⁴-[2-hydroxy-1-(3H-imidazol-4-ylmethyl)-ethyl]-succinamide,
3R-[3-(4'-chloro-biphenyl-4-yl)-prop-2-ynyl]-2S,N¹-dihydroxy-N⁴-(2-hydroxy-1S-methyl-ethyl)-succinamide,
N⁴-(1S-benzyl-2-hydroxy-ethyl)-3R-(3-biphenyl-4-yl-prop-2-ynyl)-2S,N¹-dihydroxy-succinamide,
3R-[3-(4-chloro-phenyl)-prop-2-ynyl]-N⁴-(1S-dimethylcarbamoyl-2,2-dimethyl-propyl)-N¹-hydroxy-succinamide,
N⁴-(2-benzylsulfanyl-1S-dimethylcarbamoyl-2-methyl-propyl)-3R-[3-(4-chloro-phenyl)-prop-2-ynyl]-2S,N¹-dihydroxy-succinamide,
and salts, hydrates and solvated thereof.

21. A pharmaceutical composition comprising a compound as claimed in any one of the preceding claims, together with a pharmaceutically or veterinarily acceptable carrier.

## Patentansprüche

1. Verbindung der Formel (I) 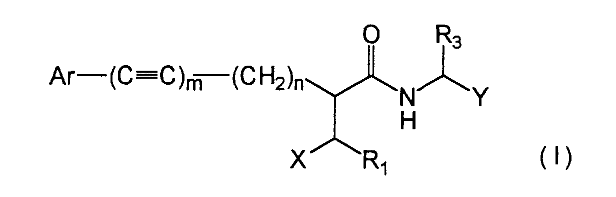 worin:
Ar eine gegebenenfalls substituierte Phenyl- oder Heteroarylgruppe darstellt;
m 1 ist;
n 0, 1, 2, 3 oder 4 ist;
X -COOH oder -CONHOH darstellt;
R₁ Wasserstoff, (C₁₋₆)-Alkyl, (C₃₋₈)-Cycloalkyl, (C₂₋₆)-Alkenyl, Phenyl, substituiertes Phenyl, Phenyl(C₁₋₆)alkyl, substituiertes Phenyl(C₁₋₆)alkyl, Heterocyclyl, substituiertes Heterocyclyl, Heterocyclyl(C₁₋₆)alkyl, substituiertes Heterocyclyl(C₁₋₆)alkyl, Amino, geschütztes Amino, Acylamino, OH, SH, (C₁₋₆)-Alkoxy, (C₁₋₆)-Alkylamino, Di(C₁₋₆)alkylamino, (C₁₋₆)-Alkylthio, Amino(C₁₋₆)alkyl, Hydroxy(C₁₋₆)alkyl, Mercapto(C₁₋₆)alkyl oder Carboxy(C₁₋₆)alkyl darstellt, worin die Amino-, Hydroxy-, Mercapto- oder Carboxylgruppe gegebenenfalls geschützt oder die Carboxylgruppe amidiert sein können;
oder eine Gruppe B'SOₚA'-, worin p 0, 1 oder 2 ist und B' Wasserstoff oder eine (C₁₋₆)-Alkyl-, Phenyl-, substituierte Phenyl-, Heterocyclyl-, (C₁₋₆)-Acyl-, Phenacyl- oder substituierte Phenacylgruppe ist, und A' (C₁₋₆)-Alkyl darstellt;
R₃ die kennzeichnende Gruppe einer natürlichen oder nicht-natürlichen α-Aminosäure darstellt, in der jede funktionelle Gruppe geschützt sein kann;
Y eine Gruppe der Formel (ID) oder (IE) ist: 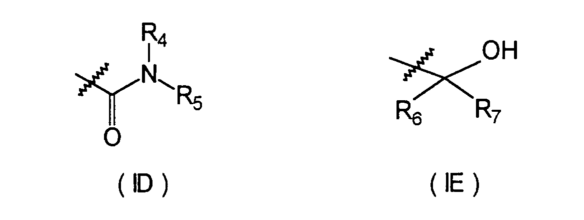
worin:
R₄ darstellt:
(a) einen gegebenenfalls substituierten Cycloalkyl- oder Cycloalkenylring, oder
(b) einen Phenyl- oder Heteroarylring, der mit einem Benzol- oder Heteroarylring kondensiert sein kann, wobei einer oder beide dieser Ringe substituiert sein können, und worin jedes Stickstoff-Ringatom zu N-Oxid oxidiert sein kann, oder
(c) eine Gruppe -CHR^{x}R^{y}, worin R^{x} und R^{y} jeweils unabhängig einen gegebenenfalls substituierten Phenyl- oder Heteroarylring darstellen, die jeweils kovalent aneinander gebunden sein können durch eine Bindung oder eine C₁₋₄-Alkylen- oder C₂₋₄-Alkenylen-Brücke,
(d) eine Gruppe der Formel -(Z'-O)_{w}-Z, worin Z' geradkettiges oder verzweigtes C₁₋₆-Alkyl ist, gegebenenfalls unterbrochen durch ein oder mehrere nicht-benachbarte S- und/oder N-Atome, w eine ganze Zahl >1 ist, und keine der linearen Atomsequenzen in der Gruppe R₄ >12 ist, oder
(e) eine geradkettige oder verzweigte C₁₋₆-Alkylgruppe, gegebenenfalls unterbrochen durch ein oder mehrere nicht-benachbarte S- und/oder N-Atome, die substituiert ist durch mindestens zwei Substituenten der Formel -(Z''')ₓ-(OZ)_{q}, worin Z''' geradkettiges oder verzweigtes C₁₋₆-Alkyl, gegebenenfalls unterbrochen durch ein oder mehrere nicht-benachbarte S- und/oder N-Atome, ist, x 0 oder 1 ist, q 1 oder 2 ist, und keine der linearen Atomsequenzen in der Gruppe R₄ >12 ist, oder
(f) Wasserstoff, C₁₋₆-Alkyl, C₁₋₄-Perfluoralkyl oder eine Gruppe D-(C₁₋₆-Alkyl)-, worin D Hydroxy, C₁₋₆-Alkoxy, C₁₋₆-Alkylthio, Acylamino, gegebenenfalls substituiertes Phenyl oder Heteroaryl, NH₂ oder Mono- oder Di-(C₁₋₆-alkyl)amino oder N-Morpholino ist;
oder R₃ und R₄ zusammen eine bivalente Kette der Formel -C(R^{a})(R^{b})-A"-Alk- darstellen, worin R^{a} und R^{b} unabhängig Wasserstoff oder C₁₋₆-Alkyl sind, A" eine Bindung, -O-, -S-, -S-S-, -NH- oder -NR^{a}- ist, worin R^{a} C₁₋₆-Alkyl ist, und Alk C₁₋₆-Alkylen ist; und
R₅ ist Wasserstoff oder eine C₁₋₆-Alkylgruppe;
R₆ ist Wasserstoff, C₁₋₆-Alkyl, Phenyl(C₁₋₆-alkyl) oder Heterocyclyl(C₁₋₆-alkyl);
R₇ ist Wasserstoff oder eine C₁₋₆-Alkylgruppe;
oder (wenn R₇ Wasserstoff ist) R₃ und R₇ bilden zusammen mit den Kohlenstoffatomen, an die sie gebunden sind, einen 2-Hydroxycyclohexyl- oder C₆-Zucker (Hexose)-Ring;
oder R₆ und R₇ bilden zusammen mit dem Kohlenstoffatom, an das sie gebunden sind, einen 5- oder 6-gliedrigen carbocyclischen oder heterocyclischen Ring;
und Salze, Hydrate und Solvate davon, unter der Voraussetzung, dass, wenn m und n beide 1 sind, und R₁ Methoxy ist, und R₃ tert-Butyl ist, und X -CONHOH ist, und Y eine Gruppe der Formel (ID) ist, worin R₄ Methyl und R₅ Wasserstoff ist, Ar nicht 4-Chlorphenyl oder 4-Phenylphenyl ist.

2. Verbindung gemäss Anspruch 1, worin die Sterochemie wie folgt ist:
C-Atom, das die Gruppen X und R₁ trägt: S,
C-Atom, das die dreifach ungesättigte Gruppe trägt: R,
C-Atom, das die Gruppen R₃ und Y trägt: S.

3. Verbindung gemäss Anspruch 1 oder 2, worin n 0 oder 1 ist.

4. Verbindung gemäss Anspruch 1 oder 2, worin Ar eine Phenylgruppe ist, die in der 4-Position durch Phenyl-, Phenoxy-, Phenylthio-, 2-, 3- oder 4-Pyridyl-, 2-, 3- oder 4-Pyridyloxy-, 2-, 3- oder 4-Pyridylthiogruppe substituiert sind, die ihrerseits gegebenfalls durch C₁₋₆-Alkyl, C₁₋₆-Alkoxy(C₁₋₆-alkoxy), Phenoxy, Phenylthio, Trifluormethyl, Halogen, Cyano (-CN), -CH₂CN, -OH oder -OR substituiert sind, worin R C₁₋₆-Alkyl oder Benzyl ist.

5. Verbindung gemäss Anspruch 1 oder 2, worin Ar eine Phenylgruppe ist, die gegebenenfalls substituiert ist durch Methyl, Ethyl, n-Propyl, n-Butyl, n-Pentyl oder n-Hexyl, 2-Methoxyethoxy, Phenoxy, Phenylthio, Trifluormethyl, Halogen, Cyano (-CN), -CH₂CN,-OH oder -OR, worin R C₁₋₆-Alkyl oder Benzyl ist.

6. Verbindung gemäss Anspruch 1 oder 2, worin Ar eine Phenylgruppe, gegebenenfalls in der 4-Position durch Trifluormethyl, Chlor, Methoxy, Cyano oder 2-Methoxyethoxy substituiert, ist.

7. Verbindung gemäss Anspruch 1 oder 2, worin Ar eine Biphenylgruppe, gegebenenfalls in der 4'-Position durch Trifluormethyl, Chlor, Methoxy, Cyano oder 2-Methoxyethoxy substituiert, ist.

8. Verbindung gemäss einem der vorhergehenden Ansprüche, worin R₁ Wasserstoff, C₁₋₄-Alkyl, Cyclopentyl, Hydroxy, Methoxy, Allyl oder eine Gruppe -(CH₂)ₜ-W ist, worin t 1, 2, 3 oder 4 darstellt und W Phthalimido, 1,2-Dimethyl-3,5-dioxo-1,2,4-triazolidin-4-yl, 3,4,4-Trimethyl-2,5-dioxo-1-imidazolidinyl, 2-Methyl-3,5-dioxo-1,2,4-oxadiazol-4-yl, 3-Methyl-2,4,5-trioxo-1-imidazolidinyl, 2,5-Dioxo-3-phenyl-1-imidazolidinyl-2-oxo-1-pyrrolidinyl, 2,5-Dioxo-1-pyrrolidinyl, 2,6-Dioxopiperidinylnaphthalimido (d.h. 1,3-Dihydro-1,3-dioxo-2H-benz[f]isoindol-2-yl), 1,3-Dihydro-1-oxo-2H-benz[f]isoindol-2-yl, 1,3-Dihydro-1,3-dioxo-2H-pyrrolo[3,4-b]chinolin-2-yl, 2,3-Dihydro-1,3-dioxo-lH-benz[d,e]isochinolin-2-yl oder Saccharinyl darstellt.

9. Verbindung gemäss einem der vorhergehenden Ansprüche, worin R₃ Methyl, Benzyl, 4-Chlorphenylmethyl, 2-Thienylmethyl, Isobutyl oder t-Butyl, 1-Benzylthio-1-methylethyl, 1-Mercapto-1-methylethyl oder 3H-Imidazol-4-ylmethyl ist.

10. Verbindung gemäss einem der vorhergehenden Ansprüche, worin Y eine Gruppe der Formel (ID) ist, und R₄ ist
Cyclopropyl, Cyclobutyl, Cyclopentyl, Cyclohexyl, Cycloheptyl oder Cyclooctyl;
Phenyl, 2-Methoxyphenyl, 3-Methoxyphenyl, 4-Methoxyphenyl, 3,4-Dimethoxyphenyl, 2-Fluorphenyl, 3-Fluorphenyl, 4-Fluorphenyl, 2-Chlorphenyl, 3-Chlorphenyl, 4-Chlorphenyl, 3,4-Dichlorphenyl, 3,5-Dichlorphenyl, 2-Bromphenyl, 3-Bromphenyl, 4-Bromphenyl, 2-Iodphenyl, 3-Iodphenyl, 4-Iodphenyl, 2-Methylphenyl, 3-Methylphenyl, 4-Methylphenyl, 3,4-Dimethyl, 2-t-Butylphenyl, 3-t-Butylphenyl, 4-t-Butylphenyl, 4-t-Butyl-2,6-dimethylphenyl, 2-Nitrophenyl, 3-Nitrophenyl, 4-Nitrophenyl, 2-Cyanophenyl, 3-Cyanophenyl, 4-Cyanophenyl, 2-Acetylphenyl, 3-Acetylphenyl, 4-Acetylphenyl, 2-Methylsulfonylphenyl, 3-Methylsulfonylphenyl, 4-Methylsulfonylphenyl, 2-Trifluormethylphenyl, 3-Trifluormethylphenyl, 4-Trifluormethylphenyl, 3,5-Ditrifluormethylphenyl, 2-Aminophenyl, 3-Aminophenyl, 4-Aminophenyl, 2-N,N-Dimethylaminophenyl, 3-N,N-Dimethylaminophenyl, 4-N,N-Dimethylaminophenyl, 2-Hydroxyphenyl, 3-Hydroxyphenyl, 4-Hydroxyphenyl, 2-Naphthyl, Furan-2-yl, Thien-2-yl, Pyrrol-2-yl, Tetrahydrofuran-2-yl, Imidazol-2-yl, Thiazol-2-yl, 4-Ethoxycarbonylmethylthiazol-2-yl, 4-Phenylthiazol-2-yl, 4,5-Dimethylthiazol-2-yl, 5-Bromthiazol-2-yl, 4-tert-Butylthiazol-2-yl, Benzothiazol-2-yl, 1,2,4-Oxadiazol-5-yl, 3-Methyl-1,2,4-oxadiazol-5-yl, 3-Phenyl-1,2,4-oxadiazol-5-yl, 1,2,4-Oxadiazol-3-yl, 1,3,4-Oxadiazol-2-yl, 1,2,4-Thiadiazol-5-yl, 3-Phenyl-1,2,4-thiadiazol-5-yl, 1,3,4-Thiadiazol-2-yl, 5-Methyl-1,3,4-thiadiazol-2-yl, Pyridin-2-yl, Pyridin-3-yl, Pyridin-4-yl, N-Oxide von Pyridin-2-yl, Pyridin-3-yl und Pyridin-4-yl, Piperazin-1-yl, Indol-2-yl, Benzimidazol-2-yl, Benzotriazol-2-yl, Pyrazin-2-yl, 1,2-Pyrazin-3-yl, 1,3-Pyrimidin-5-yl, 1,3-Dithian-2-yl, Benzo[b]thien-2-yl, Isoxazol-5-yl oder Chinolin-3-yl;
eine Gruppe -CHR^{x}R^{y}, worin R^{x} und R^{y} unabhängig gegebenenfalls substituiertes Phenyl, Thienyl, Furyl, Pyrrolyl, Imidazolyl, Thiazolyl, Pyrazolyl, Isoxazolyl, Isothiazolyl, Triazolyl, Thiadiazolyl, Oxadiazolyl, Pyridinyl, Pyridazinolyl, Pyrimidinyl, Piperazinyl oder Triazinyl darstellen;
eine Polyetherkette mit mindestens zwei nicht-benachbarten Sauerstoffatomen, z.B. 2-(2-Methoxyethoxymethoxy)ethyl, 1,1-Dimethyl-2-(2-methoxyethoxymethoxy)ethyl, 2-(2-Ethoxyethoxymethoxy)ethyl, 2-(2-(2-Methoxyethoxy)ethoxy)ethyl), 2-(2-(3-Methoxypropoxymethoxy)ethyl, 3-(2-Methoxyethoxymethoxy)propyl, 2,2-Dimethyl-3-(2-methoxyethoxymethoxy)propyl, 2-(2-Methoxyethoxy)ethyl, 3-(2-Methoxyethoxy)propyl, 2-Methyl-2,2-di(2-methoxyethyl)propyl, 2-Methyl-2,2-di(2-methoxyethyl)butyl und 2-Methyl-2,2-di(2-methoxymethyl)propyl;
Methyl, Ethyl, n- oder Isopropyl, n-, sec- oder tert-Butyl, Hydroxyethyl, Hydroxypropyl, 2,2-Dimethyl-3-hydroxypropyl, Hydroxybutyl, Methoxyethyl, Ethoxyethyl, Methoxypropyl, 2,2-Dimethyl-3-methoxypropyl, 2,2-Dimethyl-3-ethoxypropyl, 2-Ethylthioethyl, 2-Acetoxyethyl, N-Acetyl-aminoethyl, 3-(2-Pyrrolidon)propyl, Morpholin-4-ylpropyl, gegebenenfalls substituiertes Phenylethyl, Phenylpropyl, Phenylbutyl oder Phenylpentyl.

11. Verbindung gemäss einem der vorhergehenden Ansprüche, worin Y eine Gruppe der Formel (ID) ist, und R₅ Wasserstoff, Methyl oder Ethyl ist.

12. Verbindung gemäss einem der vorhergehenden Ansprüche, worin Y eine Gruppe der Formel (ID) ist, R₄ Wasserstoff oder Methyl ist und R₅ Methyl ist.

13. Verbindung gemäss einem der Ansprüche 1 bis 9, worin Y eine Gruppe der Formel (IE) ist, und R₆ Wasserstoff, Methyl, Ethyl, Benzyl oder Pyridylmethyl ist.

14. Verbindung gemäss einem der Ansprüche 1 bis 9, worin Y eine Gruppe der Formel (IE) ist, und R₇ Wasserstoff oder Methyl ist.

15. Verbindung gemäss einem der Ansprüche 1 bis 9, worin Y eine Gruppe der Formel (IE) ist, und R₆ und R₇ jeweils Wasserstoff sind.

16. Verbindung gemäss einem der Ansprüche 1 bis 9, worin Y eine Gruppe der Formel (IE) ist, und R₆ und R₇ zusammen mit dem Kohlenstoffatom, an das sie gebunden sind, einen Cyclopentyl-, Cyclohexyl- oder Morpholinoring bilden.

17. Verbindung gemäss Anspruch 1 oder 2, worin
Ar Phenyl oder substituiertes Phenyl ist;
n ist 1;
X ist -CONHOH;
R₁ ist Wasserstoff, C₁₋₄-Alkyl, Cyclopentyl, Hydroxy, Methoxy, Allyl, oder eine Gruppe -(CH₂)ₜ-W, worin t 1, 2, 3 oder 4 ist, und W Phthalimido, 1,2-Dimethyl-3,5-dioxo-1,2,4-triazolidin-4-yl, 3,4,4-Trimethyl-2,5-dioxo-1-imidazolidinyl, 2-Methyl-3,5-dioxo-1,2,4-oxadiazol-4-yl, 3-Methyl-2,4,5-trioxo-1-imidazolidinyl, 2,5-Dioxo-3-phenyl-1-imidazolidinyl-2-oxo-1-pyrrolidinyl, 2,5-Dioxo-1-pyrrolidinyl oder 2,6-Dioxo-piperidinylnaphthalimido (d.h. 1,3-Dihydro-1,3-dioxo-2H-benz[f]isoindol-2-yl), 1,3-Dihydro-1-oxo-2H-benz[f]isoindol-2-yl, 1,3-Dihydro-1,3-dioxo-2H-pyrrolo[3,4-b]chinolin-2-yl, 2,3-Dihydro-1,3-dioxo-1H-benz[d,e]isochinolin-2-yl oder Saccharinyl darstellt;
R₃ ist t-Butyl, 1-Benzylthio-1-methylethyl, 1-Mercapto-1-methylethyl, Benzyl, Methyl oder 3H-Imidazol-4-ylmethyl;
Y ist eine Gruppe der Formel (ID), worin R₄ darstellt:
Cyclopropyl, Cyclobutyl, Cyclopentyl, Cyclohexyl, Cycloheptyl oder Cyclooctyl;
Phenyl, 3-Methoxyphenyl, Pyridin-2-yl, Pyridin-3-yl, Thiazol-2-yl, 4-Ethoxycarbonylmethylthiazol-2-yl, 5-Methyl-1,3,4-thiadiazol-2-yl oder 4-tert-Butylthiazol-2-yl;
eine Gruppe -CHR^{x}R^{y}, worin R^{x} und R^{y} unabhängig Phenyl oder 4-Chlorphenyl darstellen, oder R^{x} und R^{y} sind kovalent an einen 9-H-Fluoren-9-yl-Ring gebunden;
eine Polyetherkette mit mindestens zwei nicht-benachbarten Sauerstoffatomen, z.B. 2-(2-Methoxyethoxy)ethyl; oder
Wasserstoff, Methyl oder 3-Morpholin-4-ylpropyl;
oder R₃ und R₄ stellen gemeinsam -C(CH₃)₂SCH₂CH₂CH₂- oder -C(CH₃)₂SSCH₂CH₂- dar; und
R₅ stellt Wasserstoff oder Methyl dar;
oder Y ist eine Gruppe der Formel (IE), worin R₆ und R₇ beide Wasserstoff sind.

18. Verbindung gemäss Anspruch 17, worin Ar 4-Phenyl-phenyl, 4-Phenoxy-phenyl, 4-(4'-Chlorphenyl)-phenyl, 4-(4-Cyanophenyl)-phenyl, 4-(4'-Methoxy)-phenyl, 4-[4'-(2-Methoxyethoxy)phenyl]-phenyl, 4-(Pyridin-4-yl)-phenyl, 4-(Pyridin-4-yloxy)-phenyl, 4-(4'-Bromphenyl)-phenyl, 4-Trifluormethyl-phenyl, 4-Fluorphenyl, 4-Methoxyphenyl, 4-Methoxyethoxyphenyl, 4-Propylphenyl, 4-Methylphenyl oder 4-Chlorphenyl ist.

19. Verbindung gemäss Anspruch 1, ausgewählt aus der Gruppe bestehend aus:
3R-(3-Biphenyl-4-yl-prop-2-inyl)-N⁴-(2,2-dimethyl-1S-methylcarbamoyl-propyl)-2S,N¹-dihydroxy-succinamid,
N⁴-(2,2-Dimethyl-1S-methylcarbamoyl-propyl)-2S,N¹-dihydroxy-3R-(3-phenyl-prop-2-inyl)-succinamid,
N⁴-(2,2-Dimethyl-1S-methylcarbamoyl-propyl)-2S,N¹-dihydroxy-3R-[3-(4-trifluormethyl-phenyl)-prop-2-inyl]-succinamid,
3R-[3-(4-Chlor-phenyl)-prop-2-inyl]-N⁴-(2,2-dimethyl-1S-methylcarbamoyl-propyl)-2S,N¹-dihydroxy-succinamid,
N⁴-(2,2-Dimethyl-1S-methylcarbamoyl-propyl)-2S,N¹-dihydroxy-3R-[3-(4-methoxy-phenyl)-prop-2-inyl]-succinamid,
3R-[3-(4'-Chlor-biphenyl-4-yl)-prop-2-inyl]-N⁴-(2,2-dimethyl-1S-methylcarbamoyl-propyl)-2S,N¹-dihydroxy-succinamid,
3R-[3-(4-Cyanophenyl)-prop-2-inyl]-N⁴-(2,2-dimethyl-1S-methylcarbamoyl-propyl)-2S,N¹-dihydroxy-succinamid,
3R-[3-(4-Chlorphenyl)-prop-2-inyl]-N⁴-[2,2-dimethyl-1S-(3-morpholin-4-yl-propylcarbamoyl)-propyl]-2S,N¹-dihydroxy-succinamid,
N⁴-(2,2-Dimethyl-1S-methylcarbamoyl-propyl)-2S,N¹-dihydroxy-3-{3-[4-(2-methoxy-ethoxy)-phenyl]-prop-2-inyl}-succinamid,
3R-[3-(4-Chlor-phenyl)-prop-2-inyl]-2S,N¹-dihydroxy-N⁴-{1S-[2-(2-methoxy-ethoxy)-ethylcarbamoyl]-2,2-dimethyl-propyl}-succinamid,
3R-[3-(2-Chlor-phenyl)-prop-2-inyl]-N⁴-(2,2-dimethyl-1S-methylcarbamoyl-propyl)-2S,N¹-dihydroxy-succinamid,
3R-[3-(4-Chlor-phenyl)-prop-2-inyl]-N⁴-(1S-dimethylcarbamoyl-2,2-dimethyl-propyl)-2S,N¹-dihydroxy-succinamid,
N⁴-(1S-Benzyl-2-hydroxy-ethyl)-3R-[3-(4-chlor-phenyl)-prop-2-inyl]-2S,N¹-dihydroxy-succinamid,
3R-[3-(4-Chlor-phenyl)-prop-2-inyl]-N⁴-(2-cyclohexylmethyl-1S-hydroxymethylethyl)-2S-N¹-dihydroxy-succinamid,
3R-[3-(4-Chlor-phenyl)-prop-2-inyl]-2S,N¹-dihydroxy-N⁴-[1S-hydroxymethylethyl-2-(3H-imidazol-4-yl)-ethyl]-succinamid,
N⁴-[2,2-Dimethyl-1S-(pyridin-2-ylcarbamoyl)-propyl]-2S,N¹-dihydroxy-3R-(3-thiophen-2-ylprop-2-inyl)-succinamid,
N⁴-(1S-Benzyl-2R(oder S)-hydroxy-propyl)-3R-[3-(4-chlor-phenyl)-prop-2-inyl]-2,N¹-dihydroxy-succinamid,
N⁴-(1-tert-Butylcarbamoyl-2,2-dimethylpropyl)-3R-[3-(4-chlor-phenyl)-prop-2-inyl]-2,N¹-dihydroxy-succinamid,
3R-(3-Biphenyl-4-yl-prop-2-inyl)-N⁴-(1S(oder R)-butylcarbamoyl-2,2-dimethylpropyl)-2,N¹-dihydroxy-succinamid,
3R(oder S)-(3-Biphenyl-4-yl-prop-2-inyl)-N⁴-[2,2-dimethyl-1S-(pyridin-2-ylcarbamoyl)-propyl]-N¹-hydroxy-succinamid,
6-(4-Chlorphenyl)-3R-(2,2-dimethyl-1S-methylcarbamoyl-propylcarbamoyl)-2R(oder S)-hydroxy-hex-5-insäure,
und Salze, Hydrate und Solvate davon.

20. Verbindung gemäss Anspruch 1, ausgewählt aus der Gruppe bestehend aus:
N⁴-(1S-Dimethylcarbamoyl-2,2-dimethyl-propyl)-2S,N¹-dihydroxy-3R-[3-(4-methoxy-phenyl)-prop-2-inyl]-succinamid,
3R-(3-Biphenyl-4-yl-prop-2-inyl)-N⁴-(1S-dimethylcarbamoyl-2,2-dimethyl-propyl)-2S,N¹-dihydroxy-succinamid,
3R-[3-(4'-Chlor-biphenyl-4-yl)-prop-2-inyl]-N⁴-(1S-dimethylcarbamoyl-2,2-dimethyl-propyl)-2S,N¹-dihydroxy-succinamid,
3R-[3-(4-Chlor-phenyl)-prop-2-inyl]-2S,N¹-dihydroxy-N⁴-(1S-hydroxymethyl-2,2-dimethyl-propyl)-succinamid,
3R-[3-(4-Chlor-phenyl)-prop-2-inyl]-2S,N¹-dihydroxy-N⁴-[2-hydroxy-1-(3H-imidazol-4-ylmethyl)-ethyl]-succinamid,
3R-[3-(4'-Chlor-biphenyl-4-yl)-prop-2-inyl]-2S,N¹-dihydroxy-N⁴-(2-hydroxy-1S-methyl-ethyl)-succinamid, N⁴-(1S-Benzyl-2-hydroxy-ethyl)-3R-(3-biphenyl-4-yl-prop-2-inyl)-2S,N¹-dihydroxy-succinamid,
3R-[3-(4-Chlor-phenyl)-prop-2-inyl]-N⁴-(1S-dimethylcarbamoyl-2,2-dimethyl-propyl)-N¹-hydroxy-succinamid,
N⁴-(2-Benzylsulfanyl-1S-dimethylcarbamoyl-2-methyl-propyl)-3R-[3-(4-chlor-phenyl)-prop-2-inyl]-2S,N¹-dihydroxy-succinamid,
und Salze, Hydrate und Solvate davon.

21. Pharmazeutische Zusammensetzung, umfassend eine Verbindung gemäss einem der vorhergehenden Ansprüche, zusammen mit einem pharmazeutisch oder veterinär annehmbaren Träger.

## Revendications

1. Composé de formule (I) : 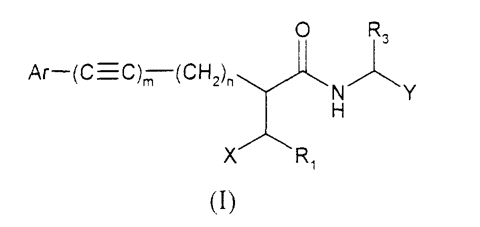 dans laquelle
Ar représente un groupe hétéroaryle ou phényle éventuellement substitué;
m vaut 1;
n vaut 0, 1, 2, 3 ou 4;
X représente -COOH ou -CONHOH;
R₁ représente l'hydrogène ou un radical alkyle en C₁ à C₆; cycloalkyle en C₃ à C₈; alcényle en C₂ à C₆; phényle; phényle substitué; phényl(alkyle en C₁ à C₆); phényl(alkyle en C₁ à C₆) substitué; hétérocyclyle; hétérocyclyle substitué; hétérocyclyl(alkyle en C₁ à C₆); hétérocyclyl(alkyle en C₁ à C₆) substitué; amino; amino protégé; acylamino; OH; SH; alcoxy en C₁ à C₆; alkylamino en C₁ à C₆; di(alkylamino en C₁ à C₆); alkylthio en C₁ à C₆; aminoalkyle en C₁ à C₆; hydroxyalkyle en C₁ à C₆; mercaptoalkyle en C₁ à C₆ ou carboxy(alkyle en C₁ à C₆), où les groupes amino, hydroxy, mercapto ou carboxy sont éventuellement protégés ou le groupe carboxy est amidé; ou un groupe B'SOₚA'- où p vaut 0, 1 ou 2 et B' est l'hydrogène ou un groupe alkyle en C₁ à C₆, phényle, phényle substitué, hétérocyclyle, acyle en C₁ à C₆, phénacyle ou phénacyle substitué, et A' représente un radical alkyle en C₁ à C₆;
R₃ représente le groupe caractérisant d'un acide α-aminé naturel ou non naturel dans lequel n'importe quels groupes fonctionnels peuvent être protégés;
Y est un groupe de formule (ID) ou (IE) 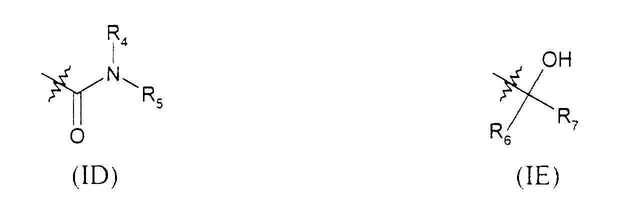
où :
R₄ représente
(a) un cycle cycloalcényle ou cycloalkyle éventuellement substitué; ou
(b) un cycle phényle ou hétéroaryle qui peut être condensé à un cycle benzène ou hétéroaryle, l'un ou les deux des cycles pouvant être substitués, et où n'importe quel atome d'azote du cycle peut être oxydé sous la forme d'un N-oxyde, ou
(c) un groupe -CHR^{x}R^{y} où R^{x} et R^{y} représentent chacun indépendamment des cycles hétéroaryle ou phényle éventuellement substitués qui peuvent être liés de façon covalente l'un à l'autre par une liaison ou par un pont alkylène en C₁ à C₄ ou alcénylène en C₂ à C₄;
(d) un groupe de formule -(Z'-O)_{w}-Z où Z' est un radical alkyle en C₁ à C₆ linéaire ou ramifié éventuellement interrompu par un ou plusieurs atomes de S et/ou de N non adjacents, w est un entier supérieur à 1, et aucune séquence linéaire continue d'atomes dans le groupe R₄ ne dépasse 12, ou bien
(e) un groupe alkyle en C₁ à C₆ linéaire ou ramifié, éventuellement interrompu par un ou plusieurs atomes de S et/ou de N non adjacents, qui est substitué par au moins deux substituants de formule -(Z''')ₓ-(OZ)_{q} où Z"' est un radical alkyle en C₁ à C₆ linéaire ou ramifié éventuellement interrompu par un ou plusieurs atomes de S et/ou de N non adjacents, x vaut 0 ou 1, q vaut 1 ou 2, et aucune séquence linéaire continue d'atomes dans le groupe R₄ ne dépasse 12, ou bien
(f) l'hydrogène ou un radical alkyle en C₁ à C₆, perfluoroalkyle en C₁ à C₄, ou un groupe D-(alkyl en C₁ à C₆)- où D est un radical hydroxy, alcoxy en C₁ à C₆, alkylthio en C₁ à C₆, acylamino, hétéroaryle ou phényle éventuellement substitué, NH₂, ou mono- ou di-(alkyl en C₁ à C₆)amino ou N-morpholino;
ou bien R₃ et R₄ pris ensemble représentent une chaîne divalente de formule -C(R^{a})(R^{b})-A"-Alk- où R^{a} et R^{b} sont indépendamment l'hydrogène ou un radical alkyle en C₁ à C₆, A" est une liaison, -O-. -S-. -S-S-. -NH- ou -NR^{a}- où R^{a} est un radical alkyle en C₁ à C₆, et Alk est un radical alkylène en C₁ à C₆: et
R₅ est l'hydrogène ou un groupe alkyle en C₁ à C₆:
R₆ est l'hydrogène ou un radical alkyle en C₁ à C₆, phényl(alkyle en C₁ à C₆) ou hétérocyclyl(alkyle en C₁ à C₆);
R₇ est l'hydrogène ou un groupe alkyle en C₁ à C₆;
ou bien (quand R₇ est l'hydrogène), R₃ et R₇ pris ensemble avec les atomes de carbone auxquels ils sont rattachés forment un cycle 2-hydroxycyclohexyle ou sucre en C₆ (hexose);
ou bien R₆ et R₇ pris ensemble avec l'atome de carbone auquel ils sont rattachés forment un cycle carbocyclique ou hétérocyclique à 5 ou 6 chaînons;
et les sels, hydrates et solvates d'un tel composé, dans la mesure où, lorsque m et n valent tous deux 1, et R₁ est le radical méthoxy, et R₃ est le radical tert-butyle, et X est -CONHOH, et Y est un groupe de formule (ID) où R₄ est le radical méthyle et R₅ est l'hydrogène, alors Ar n'est pas le radical 4-chlorophényle ou 4-phénylphényle.

2. Composé selon la revendication 1, dans lequel la stéréochimie est la suivante :
atome de C portant les groupes X et R₁ : -S,
atome de C portant le groupe à triple insaturation : -R.
atome de C portant les groupes R₃ et Y : -S.

3. Composé selon la revendication 1 ou 2, dans lequel n vaut 0 ou 1.

4. Composé selon la revendication 1 ou 2, dans lequel Ar est un groupe phényle qui est substitué en position 4 par un groupe phényle. phénoxy. phénylthio, 2-, 3- ou 4-pyridyle, 2-, 3- ou 4-pyridyloxy, 2-, 3- ou 4-pyridylthio, lequel est éventuellement substitué par un groupe alkyle en C₁ à C₆, alcoxy(en C₁ à C₆)(alcoxy en C₁ à C₆), phénoxy. phénylthio. trifluorométhyle, halogéno, cyano -(CN), -CH₂CN, -OH, ou -OR, où R est un radical alkyle en C₁ à C₆ ou benzyle.

5. Composé selon la revendication 1 ou 2, dans lequel Ar est un groupe phényle qui est éventuellement substitué par un radical méthyle. éthyle, n-propyle, n-butyle, n-pentyle ou n-hexyle, 2-méthoxyéthoxy, phénoxy, phénylthio, trifluorométhyle, halogéno, cyano (-CN), -CH₂CN, -OH, ou -OR, où R est un radical alkyle en C₁ à C₆ ou benzyle.

6. Composé selon la revendication 1 ou 2, dans lequel Ar est un groupe phényle éventuellement substitué en position 4 par un radical trifluorométhyle, chloro, méthoxy, cyano ou 2-méthoxyéthoxy.

7. Composé selon la revendication 1 ou 2, dans lequel Ar est un groupe biphényle éventuellement substitué en position 4' par un radical trifluorométhyle, chloro, méthoxy, cyano ou 2-méthoxyéthoxy.

8. Composé selon l'une quelconque des revendications précédentes, dans lequel R₁ est l'hydrogène ou un radical alkyle en C₁ à C₄, cyclopentyle, hydroxy, méthoxy, allyle, ou un groupe -(CH₂)ₜ-W où t représente 1, 2, 3 ou 4 et W représente un radical phtalimido, 1,2-diméthyl-3,5-dioxo-1,2,4-triazolidin-4-yle, 3,4,4-triméthyl-2.5-dioxo-1-imidazolidinyle, 2-méthyl-3,5-dioxo-1,2,4-oxadiazol-4-yle, 3-méthyl-2,4,5-trioxo-1-imidazolidinyle. 2,5-dioxo-3-phényl-1-imidazolidinyl-2-oxo-1-pyrrolidinyle, 2,5-dioxo-1-pyrrolidinyle, 2,6-dioxopipéridinylnaphtalimido (c'est-à-dire 1,3-dihydro-1,3-dioxo-2H-benz[f]isoindol-2-yle), 1,3-dihydro-1-oxo-2H-benz[f]isoindol-2-yle, 1,3-dihydro-1,3-dioxo-2H-pyrrolo[3,4-b]quinoléin-2-yle, 2,3-dihydro-1,3-dioxo-1H-benz[d.e]isoquinoléin-2-yle ou saccharinyle.

9. Composé selon l'une quelconque des revendications précédentes, dans lequel R₃ est un radical méthyle, benzyle, 4-chlorophénylméthyle, 2-thiénylméthyle, isobutyle ou t-butyle, 1-benzylthio-1-méthyléthyle, 1-mercapto-1-méthyléthyle ou 3H-imidazol-4-ylméthyle.

10. Composé selon l'une quelconque des revendications précédentes. dans lequel Y est un groupe de formule (ID) et R₄ est
un radical cyclopropyle, cyclobutyle, cyclopentyle, cyclohexyle, cycloheptyle ou cyclooctyle;
un radical phényle, 2-méthoxyphényle, 3-méthoxyphényle, 4-méthoxyphényle, 3,4-diméthoxyphényle, 2-fluorophényle, 3-fluorophényle, 4-fluorophényle, 2-chlorophényle, 3-chlorophényle, 4-chlorophényle, 3,4-dichlorophényle, 3,5-dichlorophényle, 2-bromophényle, 3-bromophényle, 4-bromophényle, 2-iodophényle, 3-iodophényle, 4-iodophényle, 2-méthylphényle, 3-méthylphényle, 4-méthylphényle, 3,4-diméthyle, 2-t-butylphényle, 3-t-butylphényle, 4-t-butylphényle, 4-t-butyl-2,6-diméthylphényle, 2-nitrophényle, 3-nitrophényle, 4-nitrophényle, 2-cyanophényle, 3-cyanophényle, 4-cyanophényle, 2-acétylphényle, 3-acétylphényle, 4-acétylphényle, 2-méthylsulfonylphényle, 3-méthylsulfonylphényle, 4-méthylsulfonylphényle, 2-trifluorométhylphényle, 3-trifluorométhylphényle, 4-trifluorométhylphényle, 3,5-ditrifluorométhylphényle, 2-aminophényle, 3-aminophényle, 4-aminophényle, 2-N,N-diméthylaminophényle, 3-N,N-diméthylaminophényle, 4-N,N-diméthylaminophényle, 2-hydroxyphényle, 3-hydroxyphényle, 4-hydroxyphényle, 2-naphtyle, furan-2-yle, thién-2-yle, pyrrol-2-yle, tétrahydrofuran-2-yle, imidazol-2-yle, thiazol-2-yle, 4-éthoxycarbonylméthylthiazol-2-yle, 4-phénylthiazol-2-yle, 4,5-diméthylthiazol-2-yle, 5-bromothiazol-2-yle, 4-tert-butylthiazol-2-yle, benzothiazol-2-yle, 1,2,4-oxadiazol-5-yle, 3-méthyl-1,2,4-oxadiazol-5-yle, 3-phényl-1,2,4-oxadiazol-5-yle, 1,2,4-oxadiazol-3-yle, 1,3,4-oxadiazol-2-yle, 1,2,4-thiadiazol-5-yle, 3-phényl-1,2,4-thiadiazol-5-yle, 1,3,4-thiadiazol-2-yle, 5-méthyl-1,3,4-thiadiazol-2-yle, pyridin-2-yle, pyridin-3-yle, pyridin-4-yle, N-oxydes de pyridin-2-yle, de pyridin-3-yle et de pyridin-4-yle, pipérazin-1-yle, indol-2-yle, benzimidazol-2-yle, benzotriazol-2-yle, pyrazin-2-yle, 1,2-pyridazin-3-yle, 1,3-pyrimidin-5-yle, 1,3-dithian-2-yle, benzo[b]thién-2-yle, isoxazol-5-yle, ou quinoléin-3-yle; un groupe -CHR^{x}R^{y} où R^{x} et R^{y} représentent indépendamment un radical phényle, thiényle, furyle, pyrrolyle, imidazolyle, thiazolyle, pyrazolyle, isoxazolyle, isothiazolyle, triazolyle, thiadiazolyle, oxadiazolyle, pyridinyle, pyridazinolyle, pyrimidinyle, pipérazinyle ou triazinyle, éventuellement substitué;
une chaîne polyéther possédant au moins deux atomes d'oxygène non adjacents, par exemple 2-(2-méthoxyéthoxyméthoxy)éthyle, 1,1-diméthyl-2-(2-méthoxyéthoxyméthoxy)éthyle, 2-(2-éthoxyéthoxyméthoxy)éthyle, 2-(2-(2-méthoxyéthoxy)éthoxy)éthyle, 2-(2-(3-méthoxypropoxyméthoxy)éthyle, 3-(2-méthoxyéthoxyméthoxy)propyle, 2,2-diméthyl-3-(2-méthoxyéthoxyméthoxy)propyle, 2-(2-méthoxyéthoxy)éthyle, 3-(2-méthoxyéthoxy)propyle, 2-méthyl-2,2-di(2-méthoxyéthyl)propyle, 2-méthyl-2,2-di(2-méthoxyéthyl)butyle, et 2-méthyl-2,2-di(2-méthoxyméthyl)propyle;
un radical méthyle, éthyle, n- ou isopropyle, n-, sec- ou tert-butyle. hydroxyéthyle, hydroxypropyle, 2,2-diméthyl-3-hydroxypropyle, hydroxybutyle, méthoxyéthyle, éthoxyéthyle, méthoxypropyle, 2,2-diméthyl-3-méthoxypropyle, 2,2-diméthyl-3-éthoxypropyle, 2-éthylthioéthyle, 2-acétoxyéthyle, N-acétylaminoéthyle, 3-(2-pyrrolidone)propyle, morpholin-4-ylpropyle, ou un groupe phényléthyle phénylpropyle, phénylbutyle, ou phénylpentyle, éventuellement substitué.

11. Composé selon l'une quelconque des revendications précédentes, dans lequel Y est un groupe de formule (ID) et R₅ est l'hydrogène ou un radical méthyle ou éthyle.

12. Composé selon l'une quelconque des revendications précédentes, dans lequel Y est un groupe de formule (ID), R₄ est l'hydrogène ou le radical méthyle, et R₅ est le radical méthyle.

13. Composé selon l'une quelconque des revendications 1 à 9, dans lequel Y est un groupe de formule (IE) et R₆ est l'hydrogène ou un radical méthyle, éthyle, benzyle ou pyridylméthyle.

14. Composé selon l'une quelconque des revendications 1 à 9, dans lequel Y est un groupe de formule (IE) et R₇ est l'hydrogène ou le radical méthyle.

15. Composé selon l'une quelconque des revendications 1 à 9, dans lequel Y est un groupe de formule (IE), et R₆ et R₇ sont chacun l'hydrogène.

16. Composé selon l'une quelconque des revendications 1 à 9, dans lequel Y est un groupe de formule (IE) et R₆ et R₇ pris ensemble avec l'atome de carbone auquel ils sont rattachés forment un cycle cyclopentyle, cyclohexyle ou morpholino.

17. Composé selon la revendication 1 ou 2, dans lequel
Ar est un radical phényle ou phényle substitué;
n vaut 1;
X est -CONHOH:
R₁ est l'hydrogène ou un radical alkyle en C₁ à C₄, cyclopentyle, hydroxy, méthoxy, allyle, ou un groupe -(CH₂)ₜ-W où t représente 1, 2, 3 ou 4 et W représente un radical phtalimido, 1,2-diméthyl-3.5-dioxo-1,2,4-triazolidin-4-yle, 3,4,4-triméthyl-2,5-dioxo-1-imidazolidinyle, 2-méthyl-3,5-dioxo-1,2,4-oxadiazol-4-yle, 3-méthyl-2,4,5-trioxo-1-imidazolidinyle, 2,5-dioxo-3-phényl-1-imidazolidinyl-2-oxo-1-pyrrolidinyle, 2,5-dioxo-1-pyrrolidinyle ou 2,6-dioxopipéridinylnaphtalimido (c'est-à-dire 1,3-dihydro-1,3-dioxo-2H-benz[f]isoindol-2-yle), 1,3-dihydro-1-oxo-2H-benz[f]isoindol-2-yle, 1,3-dihydro-1,3-dioxo-2H-pyrrolo[3.4-b]quinoléin-2-yle, 2,3-dihydro-1,3-dioxo-1H-benz[d,e]isoquinoléin-2-yle ou saccharinyle;
R₃ est un radical t-butyle, 1-benzylthio-1-méthyléthyle, 1-mercapto-1-méthyléthyle, benzyle, méthyle. ou 3H-imidazol-4-ylméthyle;
Y est un groupe de formule (ID) où R₄ représente
un radical cyclopropyle, cyclobutyle, cyclopentyle, cyclohexyle, cycloheptyle ou cyclooctyle;
un radical phényle, 3-méthoxyphényle, pyridin-2-yle, pyridin-3-yle, thiazol-2-yle, 4-éthoxycarbonylméthylthiazol-2-yle, 5-méthyl-1,3.4-thiadiazol-2-yle ou 4-tert-butylthiazol-2-yle;
un groupe -CHR^{x}R^{y} où R^{x} et R^{y} représentent indépendamment un radical phényle ou 4-chlorophényle ou bien R^{x} et R^{y} sont liés de façon covalente dans un cycle 9-H-fluorén-9-yle:
une chaîne polyéther possédant au moins deux atomes d'oxygène non adjacents, par exemple 2-(2-méthoxyéthoxy)éthyle; ou
l'hydrogène ou un radical méthyle ou 3-morpholin-4-ylpropyle;
ou bien R₃ et R₄ pris ensemble représentent -C(CH₃)₂SCH₂CH₂CH₂- ou -C(CH₃)₂SSCH₂CH₂-; et
R₅ représente l'hydrogène ou le radical méthyle;
ou bien Y est un groupe de formule (IE) où R₆ et R₇ sont tous deux l'hydrogène.

18. Composé selon la revendication 17, dans lequel Ar est un radical 4-phénylphényle, 4-phénoxyphényle, 4-(4'-chlorophényl)phényle, 4-(4-cyanophényl)phényle, 4-(4'-méthoxy)phényle, 4-[4'-(2-méthoxyéthoxy)phényl]phényle, 4-(pyridin-4-yl)phényle, 4-(pyridin-4-yloxy)phényle, 4-(4'-bromophényl)phényle, 4-trifluorométhylphényle, 4-fluorophényle, 4-méthoxyphényle, 4-méthoxyéthoxyphényle, 4-propylphényle, 4-méthylphényle ou 4-chlorophényle.

19. Composé selon la revendication 1, choisi dans l'ensemble constitué par les suivants :
3R-(3-biphényl-4-ylprop-2-ynyl)-N⁴-(2,2-diméthyl-1S-méthylcarbamoylpropyl)-2S,N¹-dihydroxysuccinamide; N⁴-(2,2-diméthyl-1S-méthylcarbamoylpropyl)-2S,N¹-dihydroxy-3R-(3-phénylprop-2-ynyl)succinamide;
N⁴-(2,2-diméthyl-1S-méthylcarbamoylpropyl)-2S,N¹-dihydroxy-3R-[3-(4-trifluorométhylphényl)prop-2-ynyl]succinamide;
3R-[3-(4-chlorophényl)prop-2-ynyl]-N⁴-(2,2-diméthyl-1S-méthylcarbamoylpropyl)-2S,N¹-dihydroxysuccinamide;
N⁴-(2,2-diméthyl-1S-méthylcarbamoylpropyl)-2S,N¹-dihydroxy-3R-[3-(4-méthoxyphényl)prop-2-ynyl]succinamide;
3R-[3-(4'-chlorobiphényl-4-yl)prop-2-ynyl]-N⁴-(2,2-diméthyl-1S-méthylcarbamoylpropyl)-2S,N¹-dihydroxysuccinamide;
3R-[3-(4-cyanophényl)prop-2-ynyl]-N⁴-(2,2-diméthyl-1S-méthylcarbamoylpropyl)-2S,N¹-dihydroxysuccinamide;
3R-[3-(4-chlorophényl)prop-2-ynyl]-N⁴-(2,2-diméthyl-1S-(3-morpholin-4-ylpropylcarbamoyl)propyl]-2S,N¹-dihydroxysuccinamide;
N⁴-(2,2-diméthyl-1S-méthylcarbamoylpropyl)-2S,N¹-dihydroxy-3-{3-[4-(2-méthoxyéthoxy)phényl]prop-2-ynyl}succinamide;
3R-[3-(4-chlorophényl)prop-2-ynyl]-2S,N¹-dihydroxy-N⁴-{1S-[2-(2-méthoxyéthoxy)éthylcarbamoyl]-2,2-diméthylpropyl}succinamide;
3R-[3-(2-chlorophényl)prop-2-ynyl]-N⁴-(2,2-diméthyl-1S-méthylcarbamoylpropyl)-2S,N¹-dihydroxysuccinamide;
3R-[3-(4-chlorophényl)prop-2-ynyl]-N⁴-(1S-diméthylcarbamoyl-2,2-diméthylpropyl)-2S,N¹-dihydroxysuccinamide;
N⁴-(1S-benzyl-2-hydroxyéthyl)-3R-[3-(4-chlorophényl)prop-2-ynyl]-2S,N¹-dihydroxysuccinamide;
3R-[3-(4-chlorophényl)prop-2-ynyl]-N⁴-(2-cyclohexylméthyl-1S-hydroxyméthyléthyl)-2S-N¹-dihydroxysuccinamide;
3R-[3-(4-chlorophényl)prop-2-ynyl]-2S,N¹-dihydroxy-N⁴-[1S-hydroxyméthyléthyl-2-(3H-imidazol-4-yl)éthyl]succinamide;
N⁴-[2,2-diméthyl-1S-(pyridin-2-ylcarbamoyl)propyl]-2S,N¹-dihydroxy-3R-(3-thiophén-2-ylprop-2-ynyl)succinamide;
N⁴-(1S-benzyl-2R(ou S)-hydroxypropyl)-3R-[3-(4-chlorophényl)prop-2-ynyl]-2,N¹-dihydroxysuccinamide;
N⁴-(1-tert-butylcarbamoyl-2,2-diméthylpropyl)-3R-[3-(4-chlorophényl)prop-2-ynyl]-2,N¹-dihydroxysuccinamide;
3R-(3-biphényl-4-ylprop-2-ynyl)-N⁴-(1S(ou R)-butylcarbamoyl-2.2-diméthylpropyl)-2,N¹-dihydroxysuccinamide;
3R(ou S)-(3-biphényl-4-ylprop-2-ynyl)-N⁴-[2,2-diméthyl-1S-(pyridin-2-ylcarbamoyl)propyl]-N¹-hydroxysuccinamide;
acide 6-(4-chlorophényl)-3R-(2,2-diméthyl-1S-méthylcarbamoylpropylcarbamoyl)-2R(ou S)-hydroxy-hex-5-ynoïque;
ainsi que leurs sels, hydrates et solvates.

20. Composé selon la revendication 1, choisi dans l'ensemble constitué par les suivants :
N⁴-(1S-diméthylcarbamoyl-2,2-diméthylpropyl)-2S,N¹-dihydroxy-3R-[3-(4-méthoxyphényl)prop-2-ynyl]succinamide:
3R-(3-biphényl-4-ylprop-2-ynyl)-N⁴-(1S-diméthylcarbamoyl-2,2-diméthylpropyl)-2S,N¹-dihydroxysuccinamide;
3R-[3-(4'-chlorobiphényl-4-yl)prop-2-ynyl]-N⁴-(1S-diméthylcarbamoyl-2,2-diméthylpropyl)-2S,N¹-dihydroxysuccinamide;
3R-[3-(4-chlorophényl)prop-2-ynyl]-2S,N¹-dihydroxy-N⁴-(1S-hydroxyméthyl-2,2-diméthylpropyl)succinamide;
3R-[3-(4-chlorophényl)prop-2-ynyl]-2S,N¹-dihydroxy-N⁴-[2-hydroxy-1-(3H-imidazol-4-ylméthyl)éthyl]succinamide;
3R-[3-(4'-chlorobiphényl-4-yl)prop-2-ynyl]-2S,N¹-dihydroxy-N⁴-(2-hydroxy-1S-méthyléthyl)succinamide;
N⁴-(1S-benzyl-2-hydroxyéthyl)-3R-(3-biphényl-4-ylprop-2-ynyl)-2S,N¹-dihydroxysuccinamide;
3R-[3-(4-chlorophényl)prop-2-ynyl]-N⁴-(1S-diméthylcarbamoyl-2,2-diméthylpropyl)-N¹-hydroxysuccinamide;
N⁴-(2-benzylsulfanyl-1S-diméthylcarbamoyl-2-méthylpropyl)-3R-[3-(4-chlorophényl)prop-2-ynyl]-2S,N¹-dihydroxysuccinamide; ainsi que leurs sels, hydrates et solvates.

21. Composition pharmaceutique comprenant un composé selon l'une quelconque des revendications précédentes, conjointement avec un véhicule acceptable du point de vue pharmaceutique ou vétérinaire.
